# EUROPEAN PATENT APPLICATION

(11) **EP 4 112 049 A1**
(43) Date of publication of application: **04.01.2023**
(21) Application number: 21761835.4
(22) Date of filing: 24.02.2021
(51) Int. Cl.: A61K 31/33, A61P 11/00, A61P 31/12

(54) **ANTI-CORONAVIRUS APPLICATION OF POLY ADP RIBOSE POLYMERASE INHIBITOR**

(30) Priority: 24.02.2020 CN 202010111755
(71) Applicant: Fukang (Shanghai) Health Technology Co., Ltd., Shanghai 201318 (CN)
(72) Inventor: SHEN, Xiaokun, Shanghai 201318 (CN); XIAO, Liang, Shanghai 201318 (CN); LI, Zeng, Shanghai 201318 (CN)
(74) Representative: Potter Clarkson
(86) International application number: PCT/CN2021/077610
(87) International publication number: WO 2021/169984

(57) **Abstract**

Provided is application of a poly ADP ribose polymerase inhibitor or a pharmaceutically acceptable salt thereof in the preparation of antiviral agents or in the preparation of medicines for the treatment of diseases caused by viruses, wherein the viruses are β-coronavirus viruses. Also provided is application of a compound represented by formula III and/or a compound represented by formula IV or a pharmaceutically acceptable salt thereof in the preparation of antiviral agents or in the preparation of medicines for treatment of diseases caused by viruses, the viruses being HIV, HPV, EBV, IFV and/or coronaviruses. The poly ADP ribose polymerase inhibitor comprises a compound represented by formula III and/or a compound represented by formula IV or a pharmaceutically acceptable salt thereof, and can achieve a lower effective concentration for inhibiting viruses and a higher antiviral activity while ensuring low toxicity and high safety when being used in human bodies, so that in the application to the clinical treatment of diseases caused by coronaviruses, the present inhibitor can effectively inhibiting the viruses.

## Description

This application claims the priority of the Chinese patent application 2020101117554 which has the application date of February 24, 2020. The above Chinese patent application is incorporated into the present application by reference in their entirety.

### Technical Field

The present invention belongs to the field of biomedical technology and in particular relates to the application of a medication with a poly ADP ribose polymerase inhibitor as a main component and a pharmaceutically acceptable salt thereof, and a pharmaceutical composition and a kit containing the same in anti-coronavirus-induced diseases.

### Background Art

Coronavirus (CoV) is a class of enveloped single-stranded positive-stranded RNA viruses that can infect humans and a variety of animals. It has respiratory, gastrointestinal and nervous system tropisms, and causes serious illnesses in livestock and companion animals (such as pigs, cows, chickens, dogs, cats) and can lead to illnesses ranging from common cold to severe acute respiratory syndrome in humans. In the ninth report of the International Committee on Taxonomy of Viruses, coronaviruses are divided into four groups, i.e., α, β, γ and δ according to their evolutionary characteristics. Among them, the hosts of α and β groups are mainly mammals, and γ and δ groups are mainly found in birds and fowls.

As of February 2020, there are currently seven known coronaviruses that can infect humans, including human coronaviruses 229E (HCoV-229E), NL63 (HCoV-NL63), HKU1 (HCoV-HKU1), OC43 (HCoV-OC43) and middle east respiratory syndrome coronavirus (MERS-CoV) that cause common cold, a symptom of upper respiratory tract infection, and severe acute respiratory syndrome (SARS) related coronaviruses: coronavirus SARS-CoV (severe acute respiratory syndrome coronavirus) and SARS-CoV-2 (severe acute respiratory syndrome coronavirus 2). Coronaviruses are responsible for 15%-30% of human respiratory tract infections each year, and can cause more severe diseases in newborns, the elderly and other susceptible populations, who have even higher incidences of lower respiratory tract infections. Among them, the case fatality rates are as high as 10% and 36%, respectively, for the highly lethal coronaviruses SARS-CoV and MERS-CoV. From 2019 to the beginning of 2020, the novel coronavirus SARS-CoV-2 that mainly broke out in Hubei and other places in China, caused more than 70,000 people to be infected and could lead to severe COVID-19 pneumonia (novel coronavirus pneumonia), with the severe rate ranging from 15 to 30%, the fatality rate of about 2%, again drawing much attention of people to coronavirus. However, there is currently no approved specific drug for treatment of the infections caused by coronavirus. Even for patients with severe acute respiratory tract infections caused by SARS-CoV, SARS-CoV-2 and MERS-CoV infections, the clinical treatment is mainly symptomatic treatment to reduce the complications of the patients. Therefore, there is an urgent need to develop effective drugs to treat infections caused by coronaviruses.

Small molecule compounds are a hotspot in the research of antiviral drug candidates. Exploring new uses of existing drugs has become an important approach to drug research and development. Candidate drugs have great application prospects since there is already data of their pharmacological efficacy testing, functional targets and clinical safety that is helpful for further toxicological evaluation, pharmacokinetic evaluation and formulation development, and can greatly reduce the research and development risk, shorten the research time and cut the costs for research and development.

At present, the clinical first-line treatment for infections caused by coronaviruses mostly includes broad-spectrum antiviral drugs, such as anti-HIV drug lopinavir/ritonavir (Aluvia), Arbidol, anti-Ebola virus drug Remdesivir, etc. Arbidol is a broad-spectrum antiviral drug that mainly treats upper respiratory tract infections caused by influenza A and B viruses. In recent years, many studies have proven that it has a certain inhibitory activity against SARS-CoV and MERS-CoV coronaviruses. On February 4, 2020, the latest research results of *in vitro* cell experiments performed by Li Lanjuan team have shown that Arbidol could effectively inhibit coronaviruses, and at a concentration of 10-30 micromoles, it was up to 60 times more effective than that in the untreated control group, and significantly inhibit the pathogenic effect of viruses on cells. However, from the current data, it is difficult to achieve the concentration of 10-30 micromoles in current clinical treatment. In the *Diagnosis and Treatment Protocol for Novel Coronavirus Pneumonia (Trial Version 6)*, the recommended dose for Arbidol is 200 mg per time, 3 times a day. However, as described in the package insert of "Arbidol Tablets (Manuosu)", after oral administration of single doses of Arbidol at 200 mg, 400 mg and 600 mg, the peak concentrations are 614.1 ± 342.5 ng/mL, 904.2 ± 355.6 ng/mL and 975.1 ± 661.0 ng/mL, only equivalent to the level of 1-2 micromoles and far lower than the inhibitory concentration *in vitro,* which is speculated that it can only have limited antiviral activity *in vivo.* Meanwhile, it is stated in the package insert that adverse events occur in about 6.2% of subjects taking Arbidol, mainly manifested as nausea, diarrhea, dizziness and increased serum transaminases. Also, it should be cautious that in the human bioequivalence test of domestic Arbidol preparations, some healthy subjects have developed bradycardia (some of them having a heart rate of less than 60 beats/minute in some healthy subjects and a heart rate decrease of 2-24 beats/minute at 3 hours after taking the drug). The relationship between this event and the drug remains unknown. In addition, the drug should be used with caution or as directed by a doctor for pregnant and lactating women, and those with severe renal insufficiency. The drug has unclear significance in patients with nodal disease or insufficiency, so it is recommended that this product should be used with caution for this population. It is speculated that, based on the above data, the currently available drugs are not the best choice for treatment of coronaviruses due to their low antiviral activity, insufficient clinical evidence and safety concerns, even though they are included in the recommended treatments.

In addition to Arbidol, other antiviral drugs are also undergoing *in vitro* screening and clinical trials for anti-novel coronavirus effects. However, there is evidence that not all antiviral drugs are effective against the novel coronaviruses. Among them, Aluvia, an anti-AIDS drug, is not effective in treatment of pneumonia caused by novel coronavirus infections, and has toxic and side effects, so it is no longer recommended for continued use. Remdesivir, clinical research of which is ongoing, has also relatively serious risks of liver toxicity. Therefore, according to the existing evidence and experience, the treatment of coronaviruses has its uniqueness, and it is still necessary to develop proprietary drugs that have innovative mechanisms of action, and good efficacy and safety.

Poly(ADP-ribose) polymerases (PARPs) play an important role in DNA damage repair and apoptosis, and are also involved in a series of cellular processes including infections and immune regulation. This protein family consists of 17 members, including PARP1, PARP2 and the like. PARP inhibitors were originally developed in the industry to block DNA damage repair in highly mutated cancer cells, thereby producing anti-cancer effects through "toxic damage" accumulation and "synergistic lethal" effect. At present, there are 4 PARP inhibitors that have been marketed in the world, namely, Olaparib, Niraparib, Rucaparib and Talazoparib, respectively. At the same time, several PARP inhibitors have been at the clinical study stage at home and abroad, including fluzoparib, mefuparib, simmiparib, IMP4297, BGB-290, ABT-888, etc.

It is reported in literatures that PARP may be closely related to invasion, integration and replication of viruses and the formation of capsid proteins. In 2001, Hyo Chol Ha et al. reported that PARP-1 enzyme played a key role in the replication, integration and transcription of HIV-1 during the replication of HIV virus, and Proc Natl Acad Sci U S A. 2001; 98(6): 3364-8 mentioned that PARP inhibitors helped inhibit HIV infection. It was also reported that PARP-1 could inhibit the nuclear localization of PARP-1 through participating in the process of stimulating gene transcription and expression by inflammatory pathway NF-KB, thereby inhibiting the NFKB transcription pathway and achieving the effect of inhibiting virus replication. Finally, PARP-1 can induce apoptosis of immune cells including T cells, so inhibition of PARP-1 may block the integration of HIV in host cells, thereby achieving the effect of treating AIDS. Similar findings have also been demonstrated in HPV, EBV, HSV and the like. In 2010, Tempera et al. confirmed that PARP1 inhibitors had inhibitory effects on EBV (J Virol. 2010; 84(10): 4988-97). In 2012, Grady et al. confirmed that PARP1 inhibitors had inhibitory effects on HSV (J Virol. 2012; 86(15): 8259-68). In 2017, Matthew E. Grunewald et al. proposed that the capsid protein of coronavirus was modified by ADP ribosylation and speculated that some subtypes of PARP were involved in the formation of coronavirus capsid protein (Virology. 2018, 517: 62-68), but they have not further clarified which PARP subtype selectivity is extremely important, nor do they suggest whether PARP inhibitors can inhibit viral replication or capsid protein formation. On the other hand, in 2016, Chad. V. Kuny et al. published an article discussing the role of PARP enzymes in virus-host interactions. Among them, PARP13 can degrade the reverse-transcribed RNA of viruses to block viral replication, and PARPs 10, 12, 13, 14, etc., can inhibit viral replication through the production of interferons (PLoS pathogens, 2016, 12(3): el005453). In 2019, Matthew E. Grunewald mentioned in an article that PARP enzymes had both antiviral and immunomodulatory functions, confirming that the presence of PARP12 and PARP14 could inhibit viral invasion and replication (PLoS pathogens, 2019, 15(5): e1007756). It can be seen that some subtypes of PARP enzymes can inhibit viral infections. At present, the functions of PARP are not conclusive at home and abroad, and the inhibitory effect of PARP inhibitors on various viruses remains unclear.

In summary, it is urgent to look for a drug with a lower effective concentration and higher antiviral activity when inhibiting the viruses while ensuring high safety and fewer side effects, so that it can effectively inhibit the viruses when being used in the clinical treatment of diseases caused by coronaviruses.

### Summary of the Invention

The technical problems to be solved by the present invention are to overcome the problems in the prior art, such as too high effective concentration and insufficient antiviral activity of drugs for treatment of diseases caused by coronavirus, resulting in limited antiviral activity *in vivo* when being clinically used, so the present invention provides a poly ADP ribose polymerase (PARP) inhibitor or a pharmaceutically acceptable salt thereof, and a pharmaceutical composition and a kit comprising the same, for use against viruses e.g., coronaviruses, wherein the antiviral treatment can be either the PARP inhibitor of the present invention as an antiviral agent, or the PARP inhibitor for treatment of diseases caused by viruses. The poly ADP ribose polymerase inhibitor of the present invention or a pharmaceutically acceptable salt thereof, as well as a pharmaceutical composition and a kit containing the same, can achieve a lower effective concentration for inhibiting viruses and a higher antiviral activity while ensuring low toxicity and high safety when being used in humans, so that when used in the clinical treatment of diseases caused by coronaviruses, the inhibitor of the present invention can effectively inhibit the viruses.

Through extensive experiments, it was unexpectedly found that a poly ADP ribose polymerase inhibitor or a pharmaceutically acceptable salt thereof of the present invention can significantly inhibit the replication of coronavirus *in vitro.* The present invention proves for the first time that the poly ADP ribose polymerase inhibitor or the pharmaceutically acceptable salt thereof can inhibit the infection of host cells by a coronavirus and the replication of the coronavirus and can be used for treatment of diseases caused by the coronavirus infection, and is of great significance for prevention, control and treatment of infections with coronaviruses, especially coronavirus SARS-CoV-2.

To solve the above-mentioned technical problems, the first aspect of the present invention provides application of a poly ADP ribose polymerase inhibitor or a pharmaceutically acceptable salt thereof in the preparation of antiviral agents or in the preparation of medicines for the treatment of diseases caused by viruses, wherein the viruses are β-coronavirus viruses.

In the present invention, the SARS-related coronaviruses are the coronaviruses causing severe acute respiratory syndrome (SARS). The diseases caused by SARS-related coronaviruses are symptoms or diseases of viral infections, and their early stage is mainly reflected in respiratory diseases, and the clinical manifestations include fever, fatigue, dry cough, cough, rapid respiratory rate or acute respiratory distress syndrome, shortness of breath, etc., and symptoms such as nasal congestion, runny nose and sore throat in a small number of patients. The imaging manifestations include different degrees of changes in the lungs, such as multiple spot-like and ground-glass-like shadows. The viruses are mainly transmitted by close-range droplets or contact with patients' respiratory secretions. There may also be gastrointestinal symptoms such as diarrhea, acute gastroenteritis in infants and neonates, and in rare cases, neurological syndromes. At a later stage, many complications may occur, including metabolic acidosis and coagulation dysfunctions difficult to remedy, respiratory failure, fulminant myocarditis to multiple organ failures such as heart failure, liver and kidney failure, and septic shock. Critically ill patients often develop dyspnea and/or hypoxemia one week after onset, and in severe cases, rapidly progress to acute respiratory distress syndrome, septic shock, metabolic acidosis and coagulation dysfunctions difficult to remedy, and multiple organ failures. It is expected that all of these diseases will be treated with the PARP inhibitor of the present invention. In addition, some virus carriers do not have the above symptoms, and may also receive the PARP inhibitor of the present invention to inhibit viral replication and spread, so as to prevent the virus carriers from getting sick or spreading the viruses to generate new patient cases. In an embodiment, the viruses of genus β-coronavirus are viruses that cause acute respiratory syndrome, such as SARS-related coronaviruses; preferably, the SARS-related coronaviruses are SARS-CoV (severe acute respiratory syndrome coronavirus) and/or SARS-CoV-2 (severe acute respiratory syndrome coronavirus-2).

In an embodiment, the poly ADP ribose polymerase inhibitor is an inhibitor against PARP 1 and/or PARP 2.

In an embodiment, the poly ADP ribose polymerase inhibitor is Substance **A**, a pharmaceutically acceptable salt thereof, a solvate thereof, or a solvate of a pharmaceutically acceptable salt thereof;

The Substance A is selected from the group consisting of tarazoparib, fluzoparib, simmiparib, IMP4297, BGB-290, ABT-888, one or more of PARP inhibitors as described in CN1342161A, PARP inhibitors as described in CN1788000A, PARP inhibitors as described in CN103242273A, PARP inhibitors as described in CN101415686A, and PARP inhibitors as described in CN101578279A. They are only examples herein, and do not exclude the possibility that Substance A can be selected from other compounds not listed here.

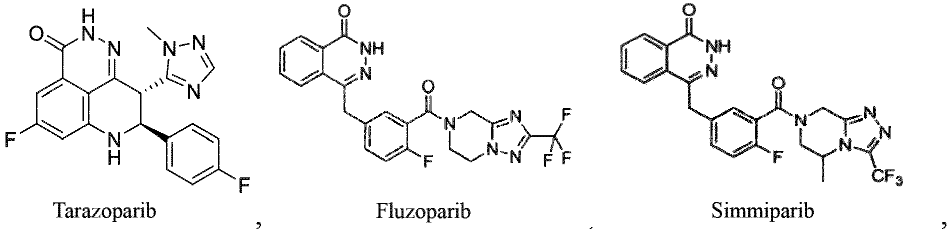

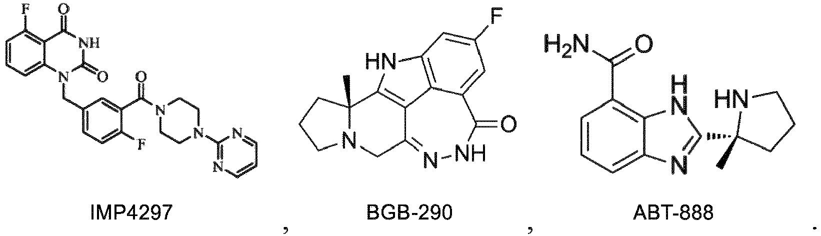

In the present invention, the "one or more selected from ..." includes the situation where the listed compounds are used in combination. In some embodiments of the present invention, there will be better therapeutic effects when two or more compounds are used in combination.

In an embodiment, the PARP inhibitor as described in CN1342161A is the compound shown in formula I;

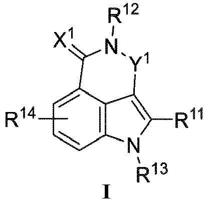

In the formula, R¹¹ is H, halogen, cyano, C₁-C₄ alkyl, C₂-C₄ alkenyl, C₂-C₄ alkynyl, C₃-C₄ cycloalkyl, 3-6-membered heterocycloalkyl "containing 1-2 heteroatoms selected from one or more of O, N and S", C₆-C₁₀ aryl, 5-10-membered heteroaryl "containing 1-2 heteroatoms selected from one or more of O, N and S", C₁-C₄ alkyl substituted by one or more R¹¹⁻¹, C₂-C₄ alkenyl substituted by one or more R¹¹⁻¹, C₂-C₄ alkynyl substituted by one or more R¹¹⁻¹, C₃-C₄ cycloalkyl substituted by one or more R¹¹⁻¹, 3-6-membered heterocycloalkyl "containing 1-2 heteroatoms selected from one or more of O, N and S", C₆-C₁₀ aryl substituted by one or more R¹¹⁻¹ (the "C₆-C₁₀ aryl" is, for example 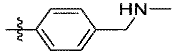 ), 5-10-membered heteroaryl "containing 1-2 heteroatoms selected from one or more of O, N and S", substituted by one or more R¹¹⁻¹, -C(=O)-R¹¹⁻², -C(=O)-O-R¹¹⁻³ or -C(=O)-NR11-4R¹¹⁻⁵;
R¹¹⁻¹ is independently halogen, hydroxyl, carboxyl, nitro, amino, C₁-C₄ alkyl or C₁-C₄ alkyl substituted by one or more NR¹¹⁻¹⁻¹R¹¹⁻¹⁻² (the "C₁-C₄ alkyl" are for example, methyl);
R¹¹⁻¹⁻¹ and R¹¹⁻¹⁻² are independently hydrogen or C₁-C₄ alkyl (e.g., methyl);
R¹¹⁻² , R¹¹⁻³, R¹¹⁻⁴ and R¹¹⁻⁵ are H, C₁-C₄ alkyl, C₂-C₄ alkenyl, C₂-C₄ alkynyl, C₃-C₄ cycloalkyl, 3-6-membered heterocycloalkyl "containing 1-2 heteroatoms selected from one or more of O, N and S", C₆-C₁₀ aryl, 5-10-membered heteroaryl "containing 1-2 heteroatoms selected from one or more of O, N and S", C₁-C₄ alkyl substituted by one or more R¹¹⁻²⁻¹, C₂-C₄ alkenyl substituted by one or more R¹¹⁻²⁻¹, C₂-C₄ alkynyl substituted by one or more R¹¹⁻²⁻¹, C₃-C₄ cycloalkyl substituted by one or more R¹¹⁻²⁻¹, 3-6-membered heterocycloalkyl "containing 1-2 heteroatoms selected from one or more of O, N and S", substituted by one or more R¹¹⁻²⁻¹, C₆-C₁₀ aryl substituted by one or more R¹¹⁻²⁻¹ or 5-10-membered heteroaryl "containing 1-2 heteroatoms selected from one or more of O, N and S", substituted by one or more R¹¹⁻²⁻¹;
R¹¹⁻²⁻¹ is independently halogen, hydroxyl, carboxyl, nitro, amino or C₁-C₄ alkyl;
Y¹ is -(CR^{Y1-}1R^{Y1-2})(CR^{Y1-3}R^{Y1-4})ₙ- or -N=C(R^{Y1-5})-;
n is 0 or 1;
R^{Y1-1}, R^{Y1-2} and R^{Y1-5} are H, C₁-C₄ alkyl, C₂-C₄ alkenyl, C₂-C₄ alkynyl, C₃-C₄ cycloalkyl, 3-6-membered heterocycloalkyl "containing 1-2 heteroatoms selected from one or more of O, N and S", C₆-C₁₀ aryl, 5-10-membered heteroaryl "containing 1-2 heteroatoms selected from one or more of O, N and S", C₁-C₄ alkyl substituted by one or more R^{Y1-1-1}, C₂-C₄ alkenyl substituted by one or more R^{Y1-1-1}, C₂-C₄ alkynyl substituted by one or more R^{Y1-1-1}, C₃-C₄ cycloalkyl substituted by one or more R^{Y1-1-1}, 3-6-membered heterocycloalkyl "containing 1-2 heteroatoms selected from one or more of O, N and S", substituted by one or more R^{Y1-1-1}, C₆-C₁₀ aryl substituted by one or more R^{Y1-1-1} or 5-10-membered heteroaryl "containing 1-2 heteroatoms selected from one or more of O, N and S", substituted by one or more R^{Y1-1-1};
R^{Y1-1-1} is independently halogen, hydroxyl, nitro, amino, C₁-C₄ alkyl or C₁-C₄ alkoxy;
R^{Y1-3} and R^{Y1-4} are H, C₁-C₄ alkyl, C₂-C₄ alkenyl, C₂-C₄ alkynyl, C₃-C₄ cycloalkyl, 3-6-membered heterocycloalkyl "containing 1-2 heteroatoms selected from one or more of O, N and S", C₆-C₁₀ aryl, 5-10-membered heteroaryl "containing 1-2 heteroatoms selected from one or more of O, N and S", C₁-C₄ alkyl substituted by one or more R^{Y1-3-1}, C₂-C₄ alkenyl substituted by one or more R^{Y1-3-1}, C₂-C₄ alkynyl substituted by one or more R^{Y1-3-1}, C₃-C₄ cycloalkyl substituted by one or more R^{Y1-3-1}, 3-6-membered heterocycloalkyl "containing 1-2 heteroatoms selected from one or more of O, N and S", substituted by one or more R^{Y1-3-1}, C₆-C₁₀ aryl substituted by one or more R^{Y1-3-1} or 5-10-membered heteroaryl "containing 1-2 heteroatoms selected from one or more of O, N and S", substituted by one or more R^{Y1-3-1};
R^{Y1-3-1} is independently halogen, hydroxyl, nitro, amino, C₁-C₄ alkyl or C₁-C₄ alkoxy;
R¹² is H or C₁-C₄ alkyl;
X¹ is O or S;
R¹⁴ is H, halogen (for example, fluorine, chlorine or bromine) or C₁-C₄ alkyl;
R¹³ is H or C₁-C₄ alkyl.

In an embodiment, the PARP inhibitor as described in CN1788000A is the compound shown in formula II;

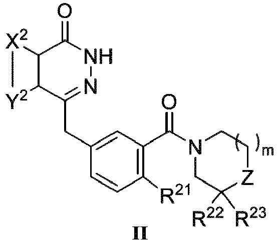

In the formula, X², Y² and the carbon atoms connected thereto together form a C₆-C₁₀ aryl (for example, phenyl), or a C₆-C₁₀ aryl substituted by one or more R^{X2-1};
R^{X2-1} is independently halogen, nitro, hydroxyl, sulfydryl, amino, C₁-C₇alkyl, C₆-C₂₀ aryl, 3-20-membered heterocyclyl "containing 1-6 heteroatoms selected from one or more of O, N and S", -OR^{X2-1-1} or -SR^{X2-1-2};
R^{X2-1-1} and R^{X2-1-2} are independently C₁-C₇ alkyl, C₆-C₂₀ aryl, or 3-20-membered heterocyclyl "containing 1-6 heteroatoms selected from one or more of O, N and S";
R²¹ is H or halogen (for example, fluorine, chlorine or bromine);
Z is -NR^{Z-1}- or -CR^{Z-2}R^{Z-3}-;
When Z is -NR^{Z-1}-, m will be 1 or 2; when Z is -CR^{Z-2}R^{Z-3}-, m will be 1;
R^{Z-1} and R^{Z-2} are independently C₁-C₂₀ alkyl, C₆-C₂₀ aryl, 3-20-membered heterocyclyl "containing 1-6 heteroatoms selected from one or more of O, N and S", -C(=O)NR^{Z-1-1}R^{Z-1-2}, -C(=O)R^{Z-1-3} (for example, 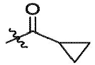 ), -C(=O)OR^{Z-1-4}, -C(=S)NR^{Z-1-5}R^{Z-1-6}, -S(=O)₂R^{Z-1-7}, C₁-C₂₀ alkyl substituted by one or more R^{Z-1-8}, C₆-C₂₀ aryl substituted by one or more R^{Z-1-9}, or 3-20-membered heterocyclyl "containing 1-6 heteroatoms selected from one or more of O, N and S", substituted by one or more R^{Z-1-10};
R^{Z-1-1}, R^{Z-1-2}, R^{Z-1-3}, R^{Z-1-4}, R^{Z-1-5}, R^{Z-1-6} and R^{Z-1-7} are independently hydrogen, C₁-C₇ alkyl, C₆-C₂₀ aryl, or 3-20-membered heterocyclyl "containing 1-6 heteroatoms selected from one or more of O, N and S";
Or, R^{Z-1-1}, R^{Z-1-2} and the carbon atoms connected thereto together form 4-8-membered heterocyclyl "containing 1-2 heteroatoms selected from one or more of O, N and S";
Or, R^{Z-1-5}, R^{Z-1-6} and the carbon atoms connected thereto together form 4-8-membered heterocyclyl "containing 1-2 heteroatoms selected from one or more of O, N and S";
R^{Z-1-8}, R^{Z-1-9} and R^{Z-1-10} are independently C₁-C₇ alkyl, C₆-C₂₀ aryl, 3-20-membered heterocyclyl "containing 1-6 heteroatoms selected from one or more of O, N and S", halogen, hydroxyl, nitro, cyano, carboxyl, sulfydryl, carbamido, -C(=O)NR^{Z-1-8-1}R^{Z-1-8-2}, -C(=O)R^{Z-1-8-3}, -C(=O)OR^{Z-1-8-4}, -C(=S)NR^{Z-1-8-5}R^{Z-1-8-6}, -S(=O)₂R^{Z-1-8-7}, -OR^{Z-1-8-8}, -SR^{Z-1-8-9}, -S(=O)₂NR^{Z-1-8-10}R^{Z-1-8-11}, -OC(=O)R^{Z1-8-12}, -S(=O)NR^{Z-1-8-13}R^{Z-1-8-14} or -C(=O)-NH-C(=O)R^{Z-1-8-15};
R^{Z-1-8-1}, R^{Z-1-8-2}, R^{Z-1-8-3}, R^{Z-1-8-4}, R^{Z-1-8-5}, R^{Z-1-8-6}, R^{Z-1-8-7}, R^{Z-1-8-8}, R^{Z-1-8-9}, R^{Z-1-8-10}, R^{Z-1-8-11}, R^{Z-1-8-12}, R^{Z-1-8-13}, R^{Z-1-8-14} and R^{Z-1-8-15} are independently hydrogen, C₁-C₇ alkyl, C₆-C₂₀ aryl, or 3-20-membered heterocyclyl "containing 1-6 heteroatoms selected from one or more of O, N and S";
Or, R^{Z-1-8-1}, R^{Z-1-8-2} and the carbon atoms connected thereto together form 4-8-membered heterocyclyl "containing 1-2 heteroatoms selected from one or more of O, N and S";
Or, R^{Z-1-8-5}, R^{Z-1-8-6} and the carbon atoms connected thereto together form 4-8-membered heterocyclyl "containing 1-2 heteroatoms selected from one or more of O, N and S";
Or, R^{Z-1-8-10}, R^{Z-1-8-11} and the carbon atoms connected thereto together form 4-8-membered heterocyclyl "containing 1-2 heteroatoms selected from one or more of O, N and S";
Or, R^{Z-1-8-13}, R^{Z-1-8-14} and the carbon atoms connected thereto together form 4-8-membered heterocyclyl "containing 1-2 heteroatoms selected from one or more of O, N and S";
R^{Z-3} is H, hydroxyl or amino;
Or, R^{Z-2}, R^{Z-3} and the carbon atoms connected thereto together form C₃-C₇ spirocycloalkyl, or 3-7-membered heterospirocycloalkyl "containing 1-3 heteroatoms selected from one or more of O, N and S";
Both R²² and R²³ are hydrogens, or, when Z is -CR^{Z-2}R^{Z-3-}, R²², R²³, R^{Z-2}, R^{Z-3} and the carbon atoms connected thereto together form a C₆-C₁₀ aryl (for example, phenyl), or a C₆-C₁₀ aryl substituted by one or more R²²⁻¹;
R²²⁻¹ is independently C₁-C₇ alkyl, C₆-C₂₀ aryl, 3-20-membered heterocyclyl "containing 1-6 heteroatoms selected from one or more of O, N and S", hydroxyl, sulfydryl, amino, -OR²²⁻¹⁻¹, -SR²²⁻¹⁻² or -O-(CH₂)ₚ-O-; p is 1, 2 or 3;
R²²⁻¹⁻¹ and R²²⁻¹⁻² are independently C₁-C₇ alkyl, C₆-C₂₀ aryl, or 3-20-membered heterocyclyl "containing 1-6 heteroatoms selected from one or more of O, N and S".

In an embodiment, the PARP inhibitor as described in CN103242273A is the compound shown in formula **III;** 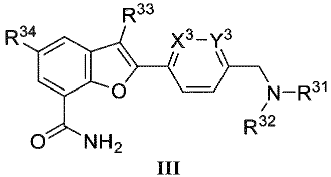
R³¹ and R³² are independently hydrogen, C₁-C₄ alkyl (for example, methyl), C₃-C₄ cycloalkyl or 5- or 6-membered heterocycloalkyl "containing 1-3 heteroatoms selected from one or more of O and N";
Or, R³¹, R³² and the N atoms connected thereto together form 5-6-membered heterocycloalkyl "containing 1-3 heteroatoms selected from one or more of O, N and S", or 5-6-membered heterocycloalkyl "containing 1-3 heteroatoms selected from one or more of O, N and S" substituted by one or more R³¹⁻¹;
R³¹⁻¹ is C₁-C₄ alkyl (for example, methyl) on N;
X³ is CH, CF or N;
Y³ is CH, CF or N;
R³³ is H or Cl;
R³⁴ is H or F.

In an embodiment, the PARP inhibitor as described in CN101415686A is the compound shown in formula **IV;** 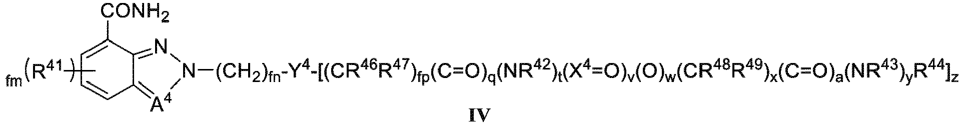
fm is 0, 1, 2 or 3;
R⁴¹ is independently hydroxy, halogen (e.g., fluorine), cyano, C₁₋₆ alkyl, halogenated C₁₋₆ alkyl, C₁₋₆ alkoxy, or halogenated C₁₋₆ alkoxy;
A⁴ is CH or N;
fn is 0, 1, 2, 3, 4, 5 or 6;
Y⁴ is a single bond, C₃₋₅ cycloalkyl, a 4-membered saturated heterocycle containing one N atom, a 5-, 6- or 7-membered saturated or partially saturated heterocycle containing 1, 2 or 3 heteroatoms independently selected from N, O and S, a 5-membered unsaturated heterocycle containing 1, 2, 3 or 4 heteroatoms independently selected from O, N and S but not more than one being O or S, a 6-membered unsaturated heterocycle containing 1, 2 or 3 nitrogen atoms, "a 6-13-membered saturated, partially saturated or unsaturated hydrocarbon ring" (for example, C₆-C₁₀ aryl, or for example, phenyl), or, "a 8-13-membered unsaturated or partially saturated heterocycle containing 1, 2, 3 or 4 heteroatoms independently selected from N, O and S";
z is 1, 2 or 3
fp is 0, 1, 2, 3, 4, 5 or 6;
R⁴⁶ and R⁴⁷ are independently hydrogen or C₁₋₆ alkyl;
q is 0 or 1;
t is 0 or 1;
R⁴² is hydrogen, C₁₋₆ alkyl or C₃₋₁₀ cycloalkyl;
v is 0 or 1;
X⁴ is C or S=O;
w is 0 or 1;
x is 0, 1, 2, 3, 4, 5 or 6;
R⁴⁸ and R⁴⁹ are independently hydrogen, C₁₋₆ alkyl, hydroxy, halo C₁₋₆ alkyl, hydroxyl C₁₋₆ alkyl, amino, C₁₋₆ alkylamino or di(C₁₋₆ alkyl)amino;
a is 0 or 1;
y is 0 or 1;
R⁴³ is hydrogen or C₁₋₆ alkyl;
R⁴⁴ is hydrogen, hydroxy, cyano, halogen (e.g., fluorine, chlorine or bromine), C₁₋₆ alkyl, C₂₋₁₀ alkenyl, halo C₁₋₆ alkyl, hydroxy C₁₋₆ alkyl, C₁₋₆ alkylcarbonyl, C₁₋₆ alkoxy, halo C₁₋₆ alkoxy, C₁₋₆ alkoxycarbonyl, carboxyl, nitro, R⁴⁴⁻¹, or, R⁴⁴⁻³ substituted by one or more -(CH₂)_{b}R⁴⁴⁻²;
R⁴⁴⁻¹ and R⁴⁴⁻³ are independently C₆₋₁₀ aryl, C₆₋₁₀ aryloxy, C₆₋₁₀ arylcarbonyl, C₃₋₁₀ cycloalkyl, 4-membered saturated heterocycle containing one N atom, "5- or 6-membered saturated or partially saturated heterocycle containing 1, 2 or 3 atoms independently selected from N, O and S" (for example, 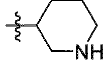 "5-membered heteroaromatic ring containing 1, 2, 3 or 4 heteroatoms independently selected from O, N and S but not more than one being O or S", "6-membered heteroaromatic ring containing 1, 2 or 3 nitrogen atoms", or "7- to 15-membered unsaturated, partially saturated or saturated heterocycle containing 1, 2, 3 or 4 heteroatoms independently selected from N, O and S";
b is independently 0, 1, 2, 3, 4, 5 or 6;
R⁴⁴⁻² is independently hydroxy, oxo, cyano, halogen, C₁₋₆ alkyl, C₂₋₁₀ alkenyl, halo C₁₋₆ alkyl, C₁₋₆ alkylcarbonyl, C₁₋₆ alkoxy, halo C₁₋₆ alkoxy, hydroxy C₁₋₆ alkyl, C₁₋₆ alkoxycarbonyl, carboxyl, -NR^{a}R^{b}, -C(=O)NR^{a}R^{b}, S(=O)_{fr}R^{e}, R⁴⁴⁻²⁻¹, or R⁴⁴⁻²⁻³ substituted by one or more R⁴⁴⁻²⁻²;
R^{a} and R^{b} are independently hydrogen, C₁₋₆ alkyl, C₁₋₆ alkylcarbonyl, halo C₁₋₆ alkyl, hydroxyl C₁₋₆ alkyl, S(O)ₛᵣR^{c} or S(O)ₜᵣN(R^{d})₂;
sr and tr are independently 0, 1 or 2;
R^{c} is C₁₋₆ alkyl, R^{c-1} or R^{c-3} substituted by one or more R^{c-2};
R^{c-1} and R^{c-3} are independently C₆₋₁₀ aryl, "5-membered heteroaromatic ring containing 1, 2, 3 or 4 heteroatoms independently selected from N, O and S, not more than one being O or S", "6-membered heteroaromatic ring containing 1, 2 or 3 nitrogen atoms", or "7- to 10-membered unsaturated or partially saturated heterocycle containing 1, 2, 3 or 4 heteroatoms independently selected from N, O and S";
R^{c-2} is independently hydroxy, cyano, halogen, C₁₋₆ alkyl, C₂₋₁₀ alkenyl or halo C₁₋₆ alkyl;
R^{d} is independently hydrogen or C₁₋₆ alkyl;
Or, R^{a}, R^{b} and the N atoms connected thereto together form R^{a-1}, or R^{a-3} substituted by one or more R^{a-2};
R^{a-1} and R^{a-3} are independently "4-membered saturated heterocycle containing one N atom", or "5-, 6- or 7-membered saturated or partially saturated heterocycle containing 1, 2 or 3 N atoms and 0 or 1 O atom";
R^{a-2} is independently hydroxy, cyano, halogen, C₁₋₆ alkyl, C₁₋₆ alkoxy, C₂₋₁₀ alkenyl or halo C₁₋₆ alkyl;
fr is 0, 1 or 2;
R^{e} is C₁₋₆ alkyl, R^{e-1} or R^{e-3} substituted by one or more R^{e-2};
R^{e-1} and R^{e-3} are independently C₆₋₁₀ aryl, "5-membered heteroaromatic ring containing 1, 2, 3 or 4 heteroatoms independently selected from N, O and S, no more than one thereof being O or S", "6-membered heteroaromatic ring containing 1, 2 or 3 nitrogen atoms", or "7- to 10-membered unsaturated or partially saturated heterocycle containing 1, 2, 3 or 4 heteroatoms independently selected from N, O and S";
R^{e-2} is independently hydroxy, cyano, halogen, C₁₋₆ alkyl, C₂₋₁₀ alkenyl or halo C₁₋₆ alkyl;
R⁴⁴⁻²⁻¹ and R⁴⁴⁻²⁻³ are independently C₆₋₁₀ aryl, C₆₋₁₀ aryl C₁₋₆ alkyl, "4-membered saturated heterocycle containing one N atom", "5-, 6- or 7-membered saturated or partially saturated heterocycle containing 1, 2 or 3 atoms independently selected from N, O and S", "5-membered heteroaromatic ring containing 1, 2, 3 or 4 heteroatoms independently selected from O, N and S, no more than one thereof being O or S", "6-membered heterocycle containing 1, 2 or 3 nitrogen atoms", or "7- to 10-membered unsaturated or partially saturated heterocycle containing 1, 2, 3 or 4 heteroatoms independently selected from N, O and S";
R⁴⁴⁻²⁻² is independently hydroxy, cyano, halogen, C₁₋₆ alkyl, C₁₋₆ alkoxy, C₂₋₁₀ alkenyl, halo C₁₋₆ alkyl, amino, C₁₋₆ alkylamino and di(C₁₋₆ alkyl)amino.

In an embodiment, the compound shown in formula I has the following definitions:
In the formula, R¹¹ is C₆-C₁₀ aryl substituted by one or more R¹¹⁻¹ (the "C₆-C₁₀aryl" is for example, phenyl; the "C₆-C₁₀ aryl substituted by one or more R¹¹⁻¹" is for example 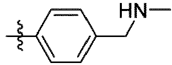 );
R¹¹⁻¹ is independently halogen, hydroxyl, carboxyl, nitro, amino, C₁-C₄ alkyl or C₁-C₄ alkyl substituted by one or more NR¹¹⁻¹⁻¹R¹¹⁻¹⁻² (the "C₁-C₄ alkyl" are for example, methyl);
R¹¹⁻¹⁻¹ and R¹¹⁻¹⁻² are independently hydrogen or C₁-C₄ alkyl (e.g., methyl);
Y¹ is -(CR^{Y1-1}R^{Y1-2})(CR^{Y1-3}R^{Y1-4})ₙ-;
n is 0 or 1;
R^{Y1-1} and R^{Y1-2} are independently H or C₁-C₄ alkyl;
R^{Y1-3} and R^{Y1-4} are independently H or C₁-C₄ alkyl;
R¹² is H or C₁-C₄ alkyl;
X¹ is O or S;
R¹⁴ is H or halogen (for example, fluorine, chlorine or bromine);
R¹³ is H or C₁-C₄ alkyl.

In an embodiment, the compound shown in formula **II** has the following definitions:
X², Y² and the carbon atoms connected thereto together form a C₆-C₁₀ aryl (for example, phenyl), or a C₆-C₁₀ aryl substituted by one or more R^{X2-1};
R^{X2-1} is independently halogen, nitro, hydroxyl, sulfydryl, amino, C₁-C₇ alkyl, C₆-C₂₀ aryl, 3-20-membered heterocyclyl "containing 1-6 heteroatoms selected from one or more of O, N and S", -OR^{X2-1-1} or -SR^{X2-1-2};
R^{X2-1-1} and R^{X2-1-2} are independently C₁-C₇ alkyl, C₆-C₂₀ aryl, or 3-20-membered heterocyclyl "containing 1-6 heteroatoms selected from one or more of O, N and S";
R²¹ is H or halogen (for example, fluorine, chlorine or bromine);
Z is -NR^{Z-1-}; m is 1 or 2;
R^{Z-1} is independently -C(=O)NR^{Z-1-1}R^{Z-1-2}, -C(=O)R^{Z-1-3} (for example, 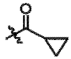 ), -C(=O)OR^{Z-1-4}, -C(=S)NR^{Z-1-5}R^{Z-1-6} or -S(=O)₂R^{Z-1-7};
R^{Z-1-1}, R^{Z-1-2}, R^{Z-1-3}, R^{Z-1-4}, R^{Z-1-5}, R^{Z-1-6} and R^{Z-1-7} are independently hydrogen, C₁-C₇ alkyl, C₆-C₂₀ aryl, or 3-20-membered heterocyclyl "containing 1-6 heteroatoms selected from one or more of O, N and S";
Or, R^{Z-1-1}, R^{Z-1-2} and the carbon atoms connected thereto together form 4-8-membered heterocyclyl "containing 1-2 heteroatoms selected from one or more of O, N and S";
Or, R^{Z-1-5}, R^{Z-1-6} and the carbon atoms connected thereto together form 4-8-membered heterocyclyl "containing 1-2 heteroatoms selected from one or more of O, N and S";
Both R²² and R²³ are hydrogen.

In an embodiment, the compound shown in formula **III** has the following definitions:
R³¹ is hydrogen, methyl, ethyl, isopropyl, cyclopropyl, piperidin-4-yl or *(R)*-tetrahydrofuran-3-yl;
R³² is hydrogen, methyl, ethyl, isopropyl, cyclopropyl, piperidin-4-yl or *(R)*-tetrahydrofuran-3-yl;
Or, R³¹, R³² and the N atom connected thereto together form unsubstituted or substituted morpholinyl, piperazinyl, homopiperazinyl, thiomorpholinyl, piperidinyl or tetrahydropyrrolyl, wherein the substitution means that N is substituted by methyl;
X³ is CH, CF or N;
Y³ is CH, CF or N;
R³³ is H;
R³⁴ is F.

In an embodiment, the compound shown in formula **III** has the following definitions:
R³¹ is hydrogen or methyl;
R³² is methyl, isopropyl, cyclopropyl, piperidin-4-yl or *(R)*-tetrahydrofuran-3-yl;
Or, R³¹, R³² and the N atom connected thereto together form unsubstituted or substituted morpholinyl, piperazinyl, homopiperazinyl, thiomorpholinyl, piperidinyl or tetrahydropyrrolyl, wherein the substitution means that N is substituted by methyl;
X³ is CH, CF or N;
Y³ is CH, CF or N;
R³³ is H;
R³⁴ is F.

In an embodiment, the compound shown in formula **IV** has the following definitions:
fm is 0, 1, 2 or 3;
R⁴¹ is independently hydroxy, halogen, cyano, C₁₋₆ alkyl, halogenated C₁₋₆ alkyl, C₁₋₆ alkoxy, or halogenated C₁₋₆ alkoxy;
A⁴ is CH or N;
fn is 0;
Y⁴ is a 5-, 6- or 7-membered saturated or partially saturated heterocycle containing 1, 2 or 3 heteroatoms independently selected from N, O and S, a 5-membered unsaturated heterocycle containing 1, 2, 3 or 4 heteroatoms independently selected from O, N and S but not more than one being O or S, a 6-membered unsaturated heterocycle containing 1, 2 or 3 nitrogen atoms, "a 6-13-membered saturated, partially saturated or unsaturated hydrocarbon ring" (for example, C₆-C₁₀ aryl, or for example, phenyl), or, "a 8-13 membered unsaturated or partially saturated heterocycle containing 1, 2, 3 or 4 heteroatoms independently selected from N, O and S";
z is 1 or 2;
fp is 0;
q is 0;
t is 0;
v is 0;
w is 0;
x is 0;
a is 0;
y is 0;
R⁴⁴ is halogen or R⁴⁴⁻¹;
R⁴⁴⁻¹ is C₃₋₁₀ cycloalkyl, 4-membered saturated heterocycle containing one N atom, "5- or 6-membered saturated or partially saturated heterocycle containing 1, 2 or 3 atoms independently selected from N, O and S" (for example, 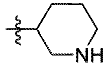 ), or, "7-15-membered unsaturated, partially saturated or saturated heterocycle containing 1, 2, 3 or 4 heteroatoms independently selected from N, O and S".

In an embodiment, the compound shown in formula **IV** has the following definitions: 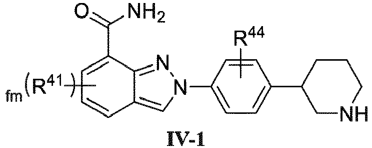
fm is 0 or 1;
R⁴¹ is halogen (e.g., fluorine);
R⁴⁴ is hydrogen or halogen (e.g., fluorine);

In an embodiment, the compound shown in formula **I** is any of the following compounds:

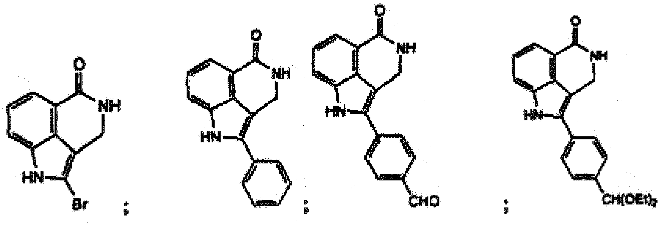

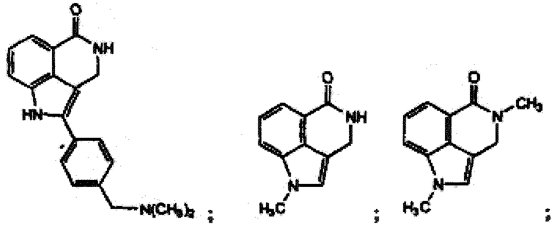

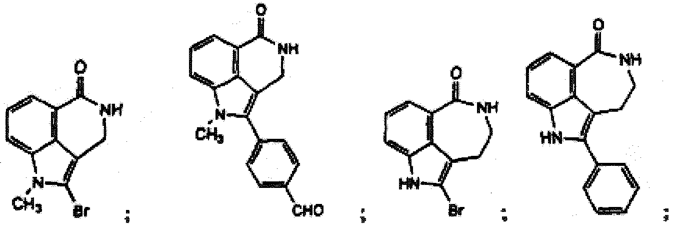

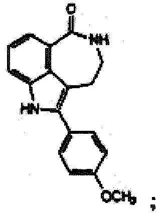

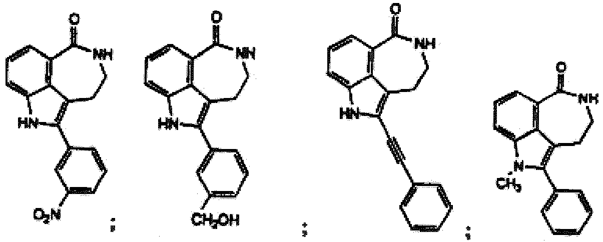

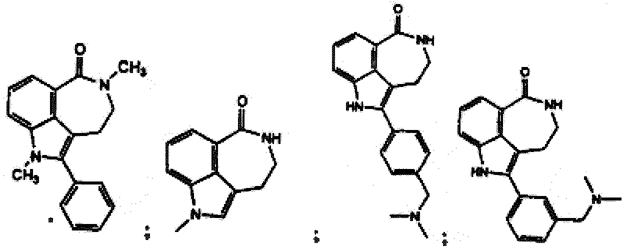

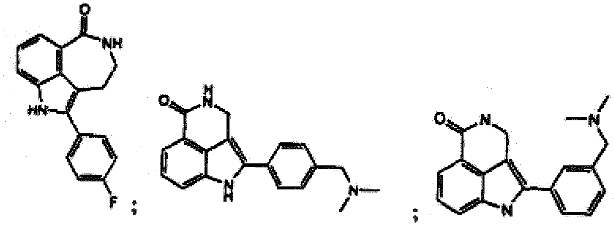

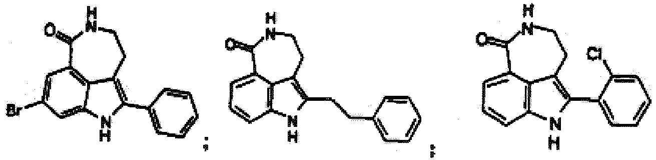

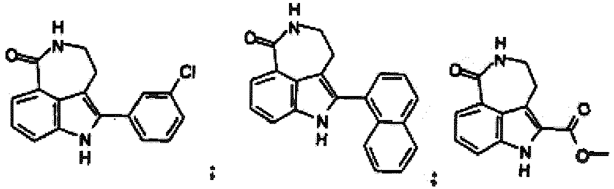

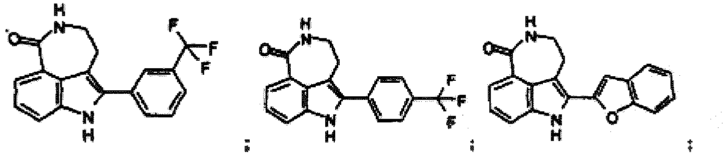

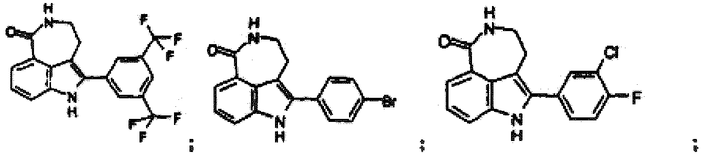

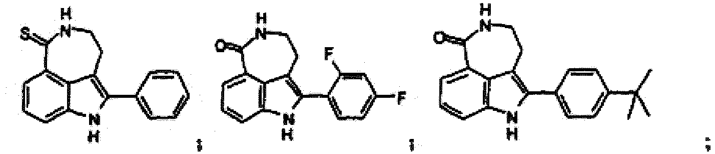

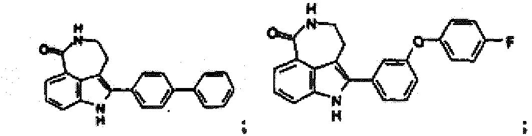

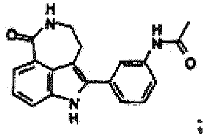

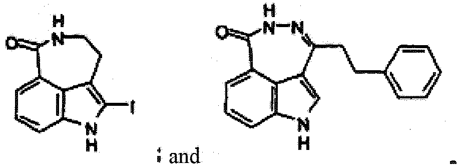

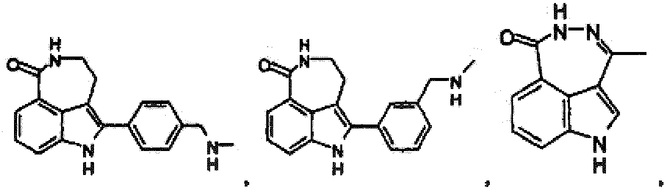

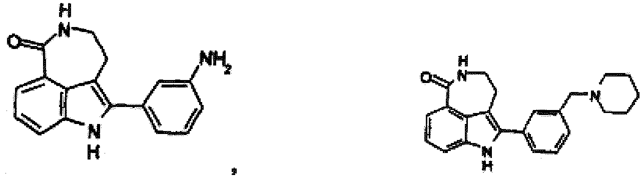

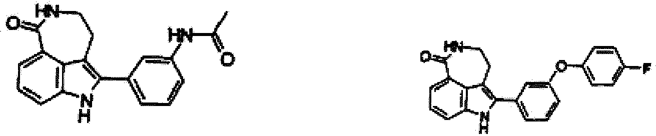

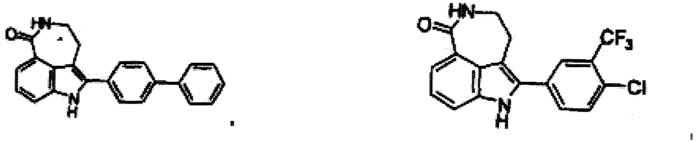

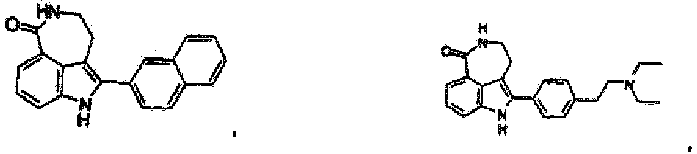

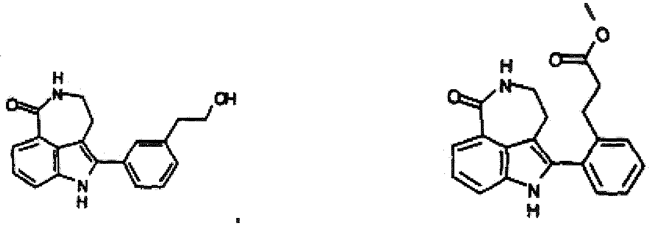

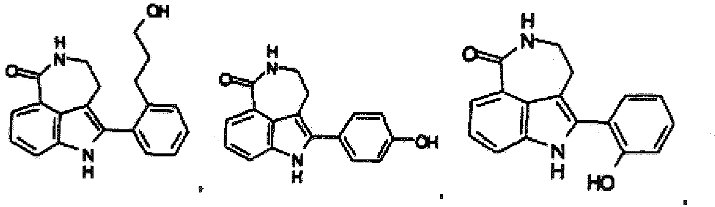

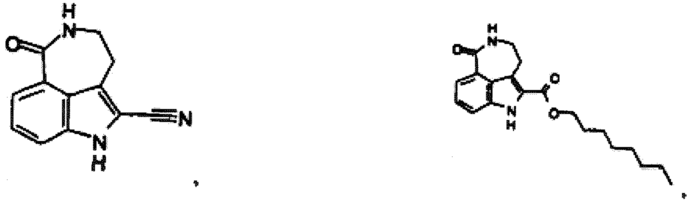

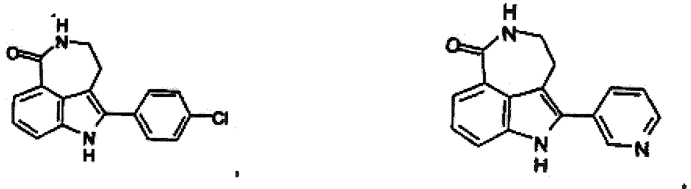

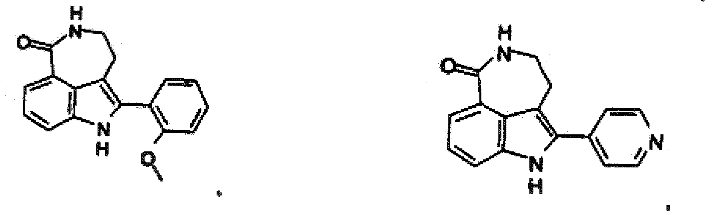

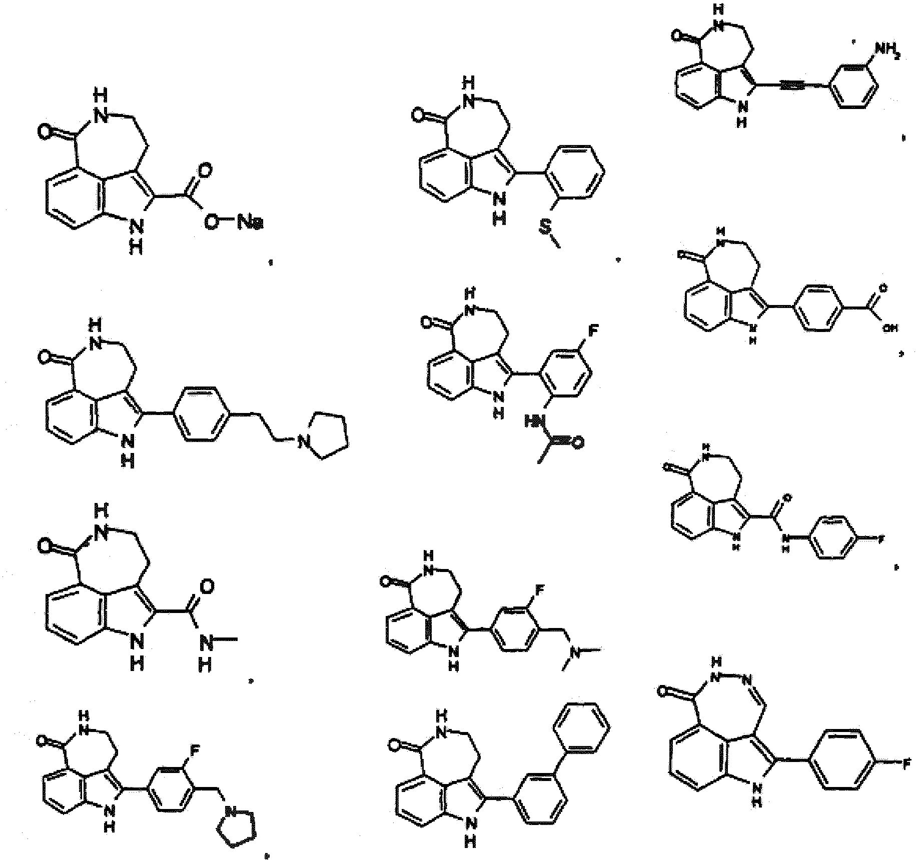

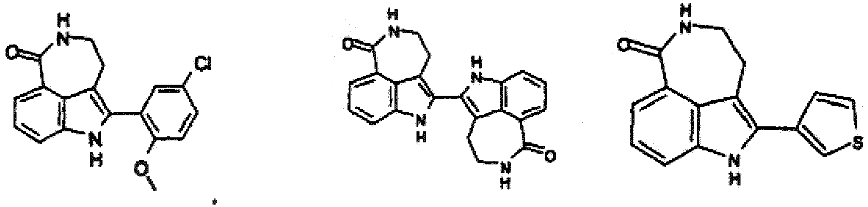

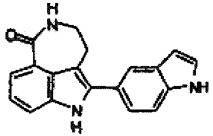

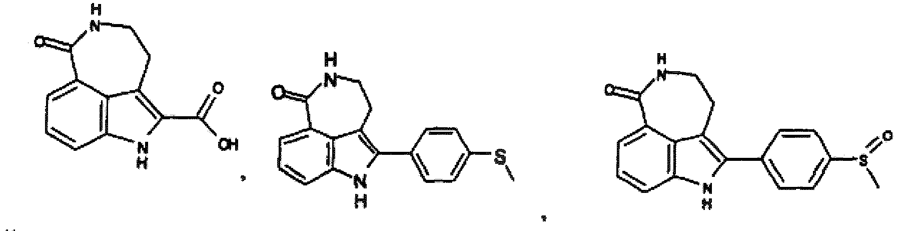

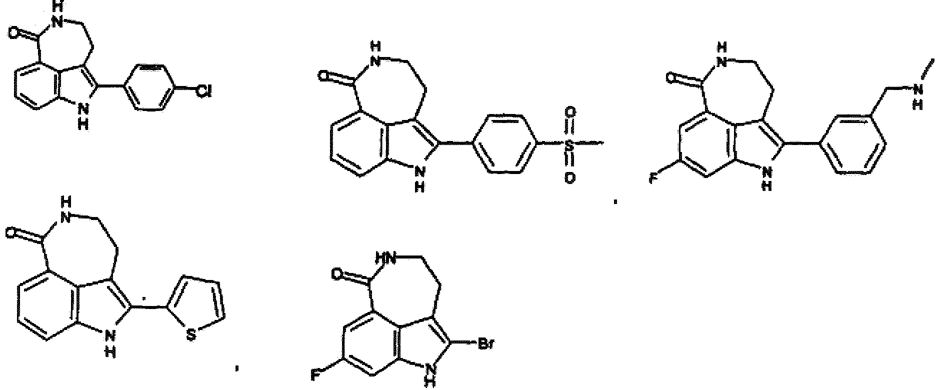

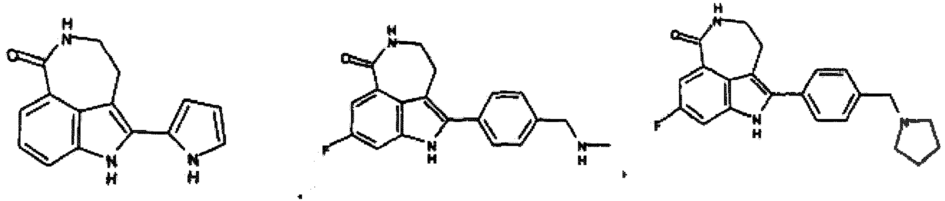

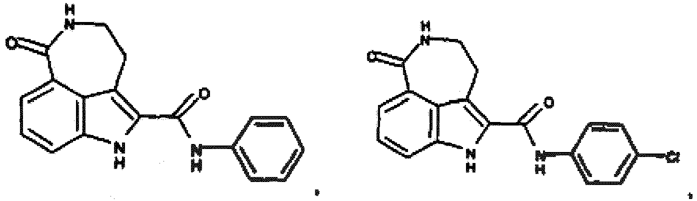

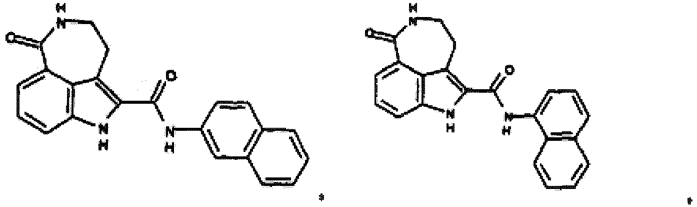

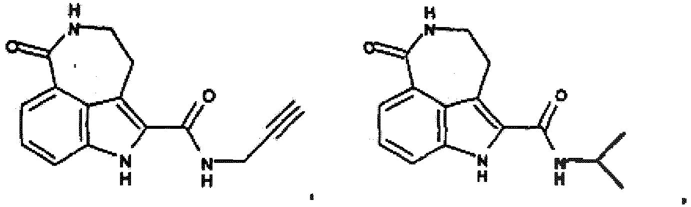

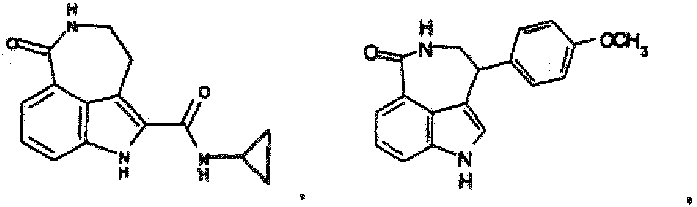

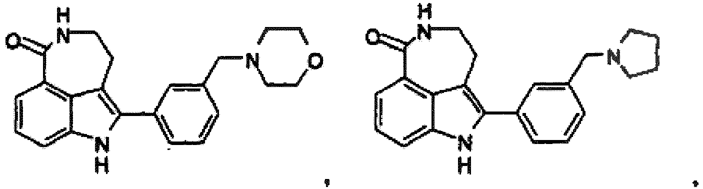

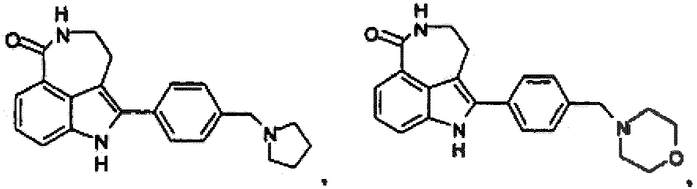

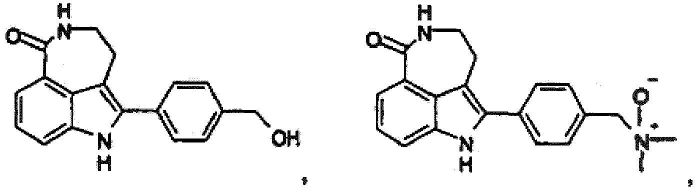

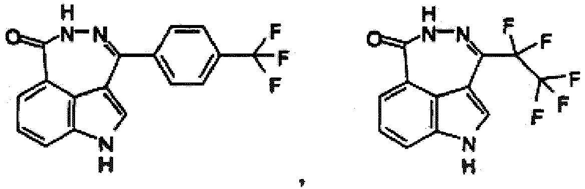

In an embodiment, the compound shown in formula **II** is any of the following compounds: 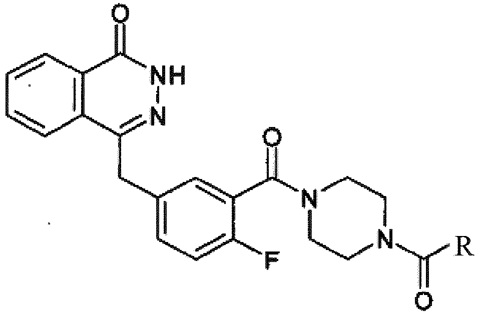 In the formula, R is selected from
**a)** 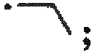 **b)** 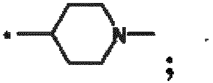 **c)** 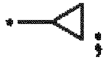 **d)** 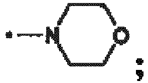 **e)** 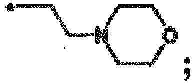 **f)** 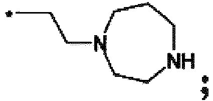 **g)** 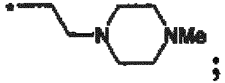 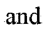 **h)**

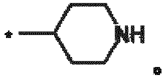

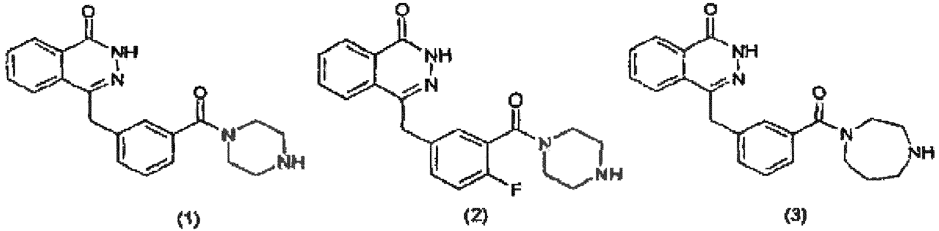

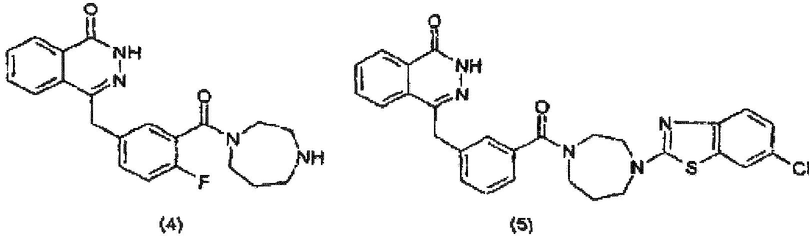

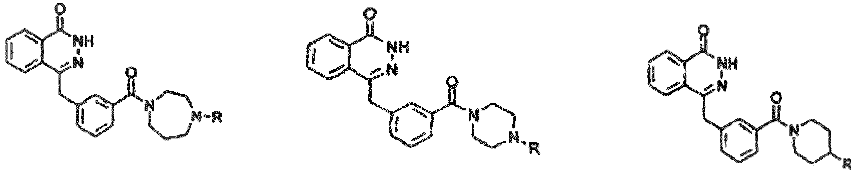

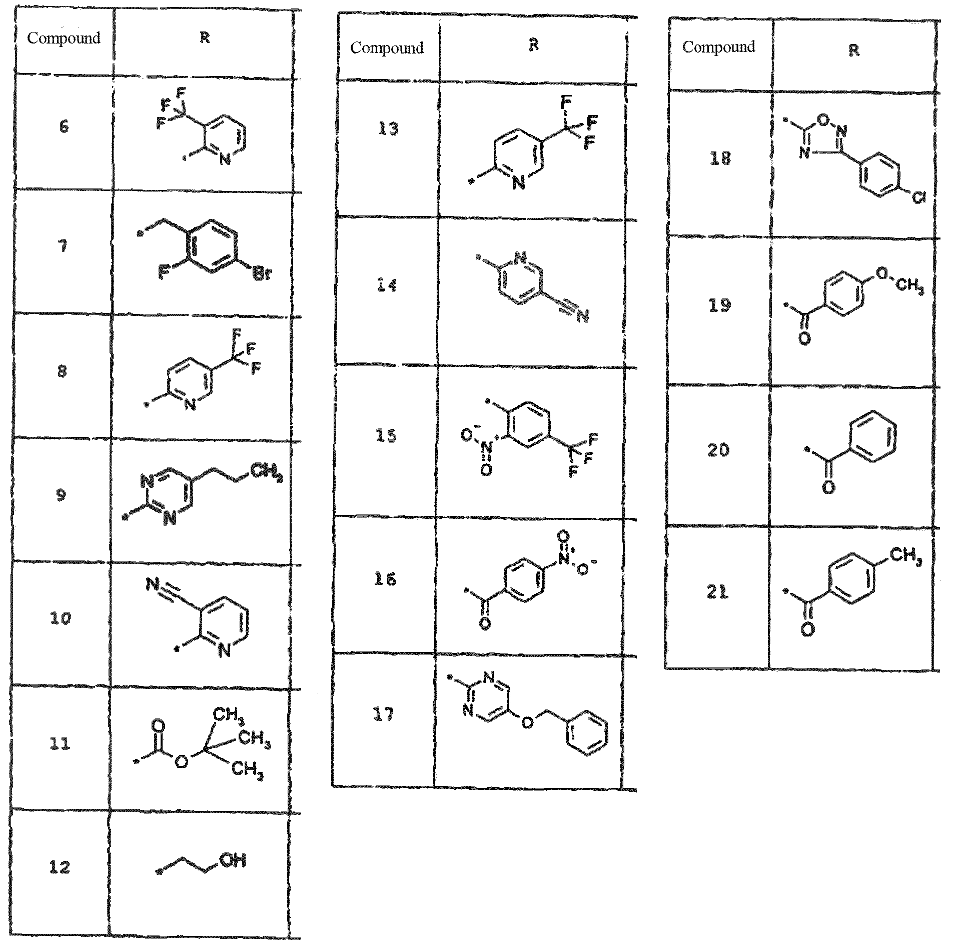

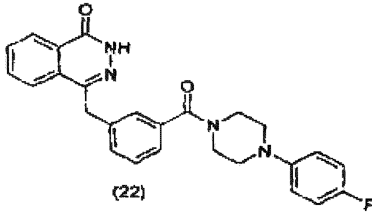

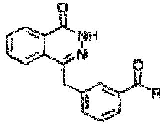

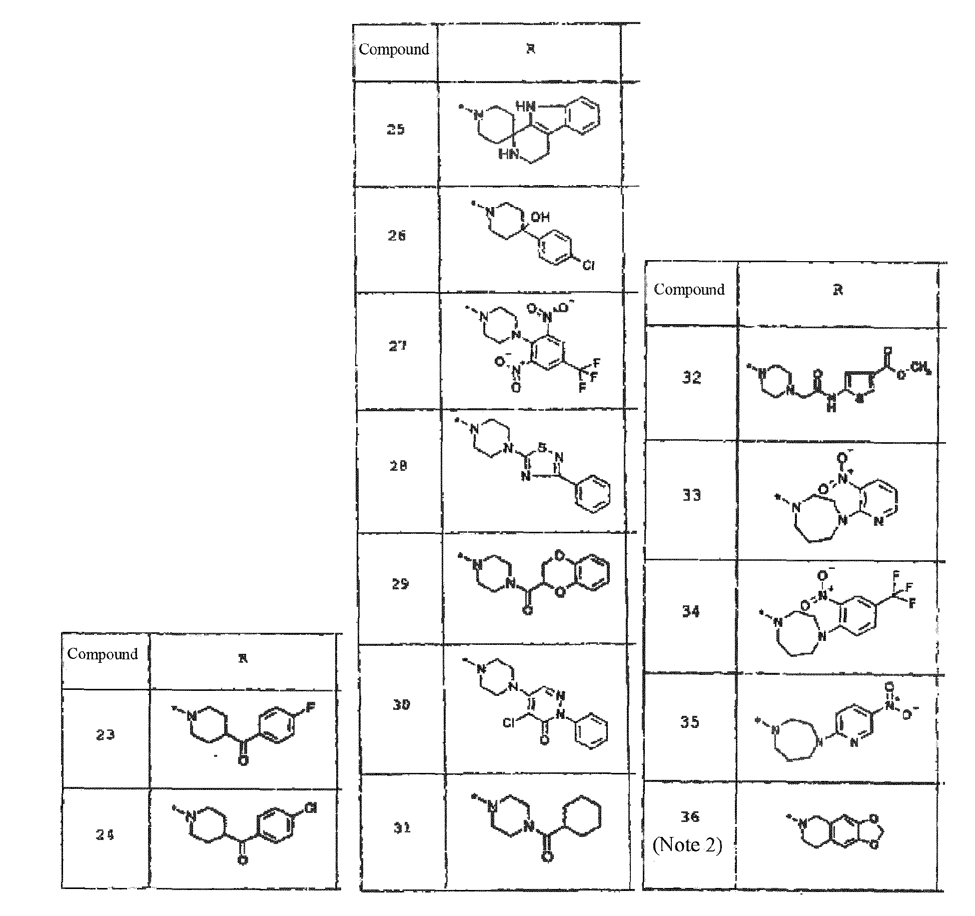

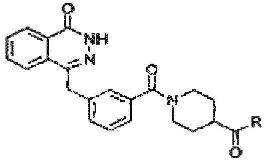

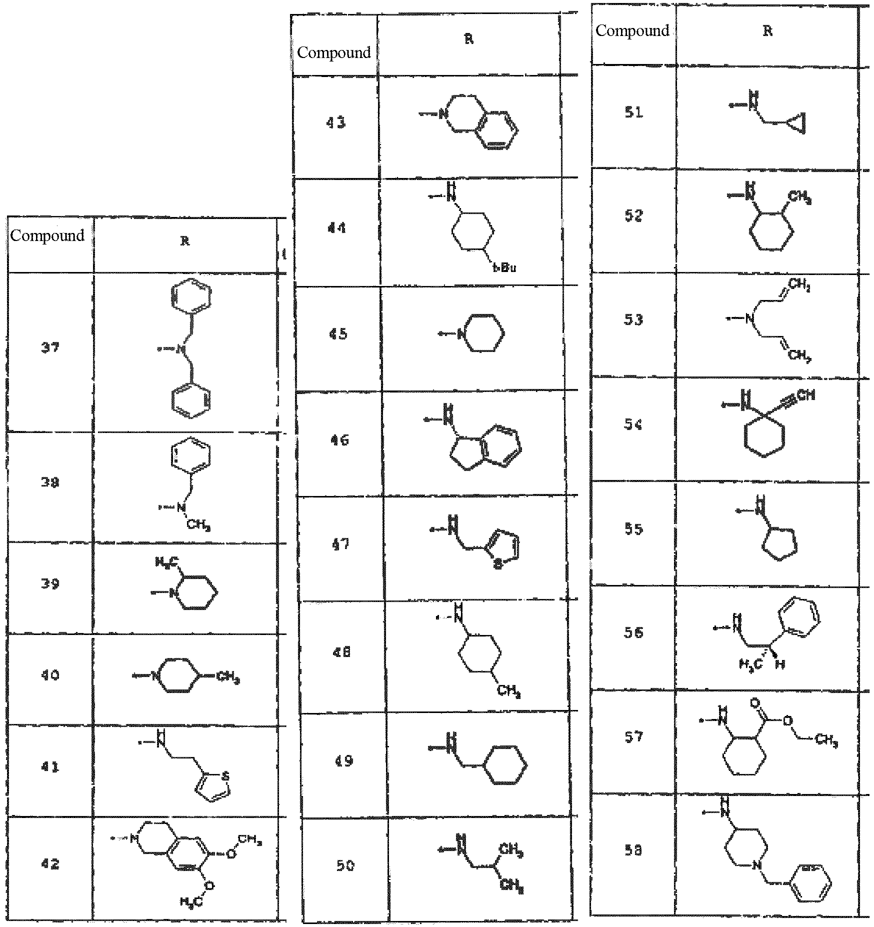

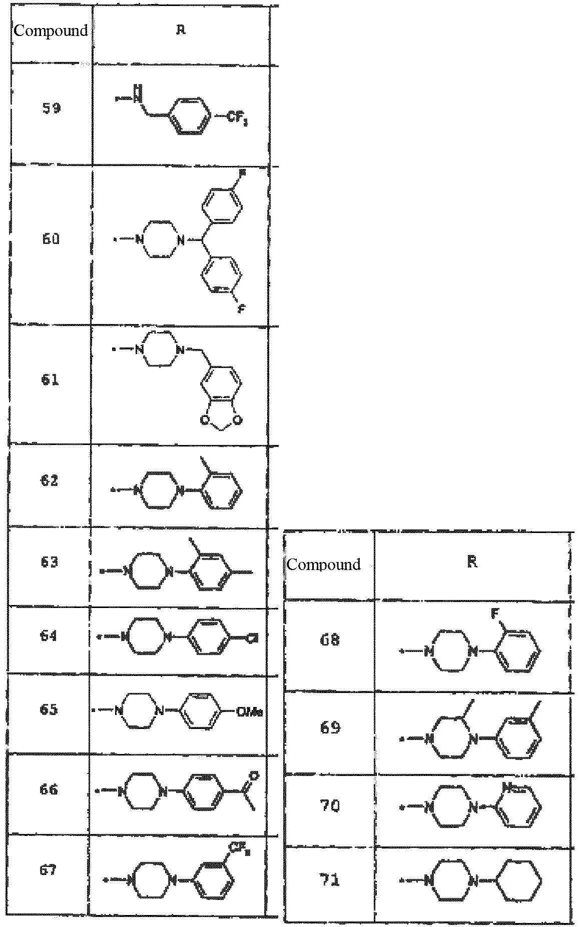

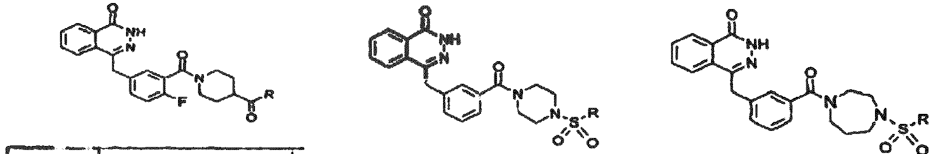

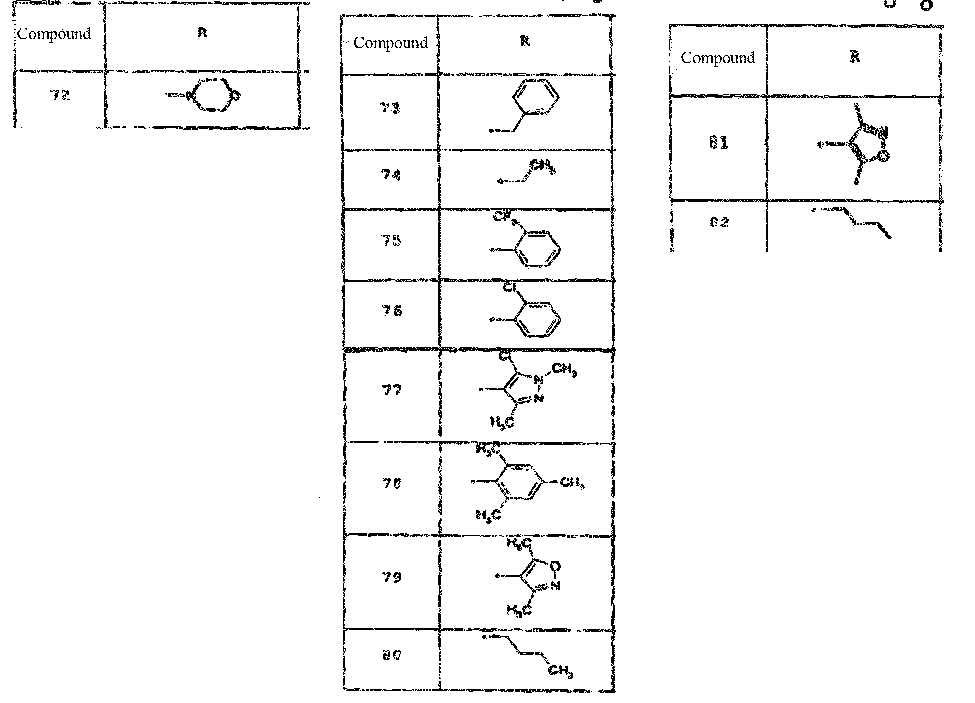

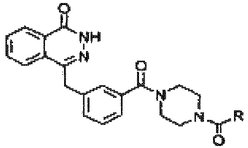

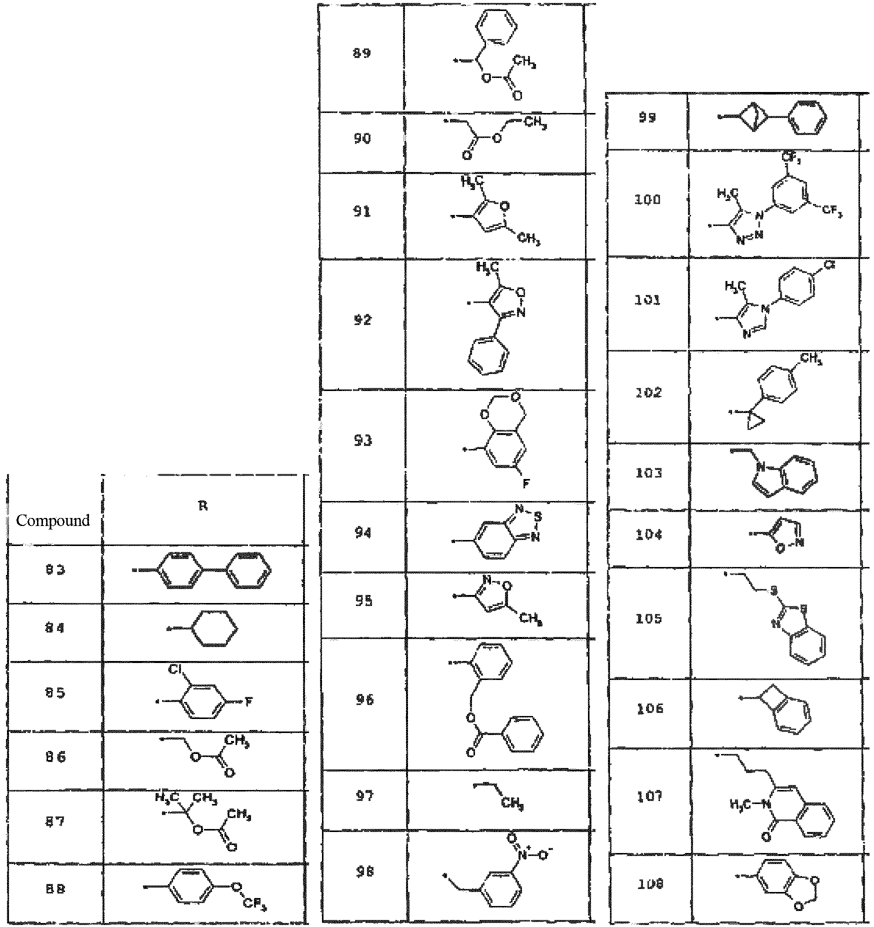

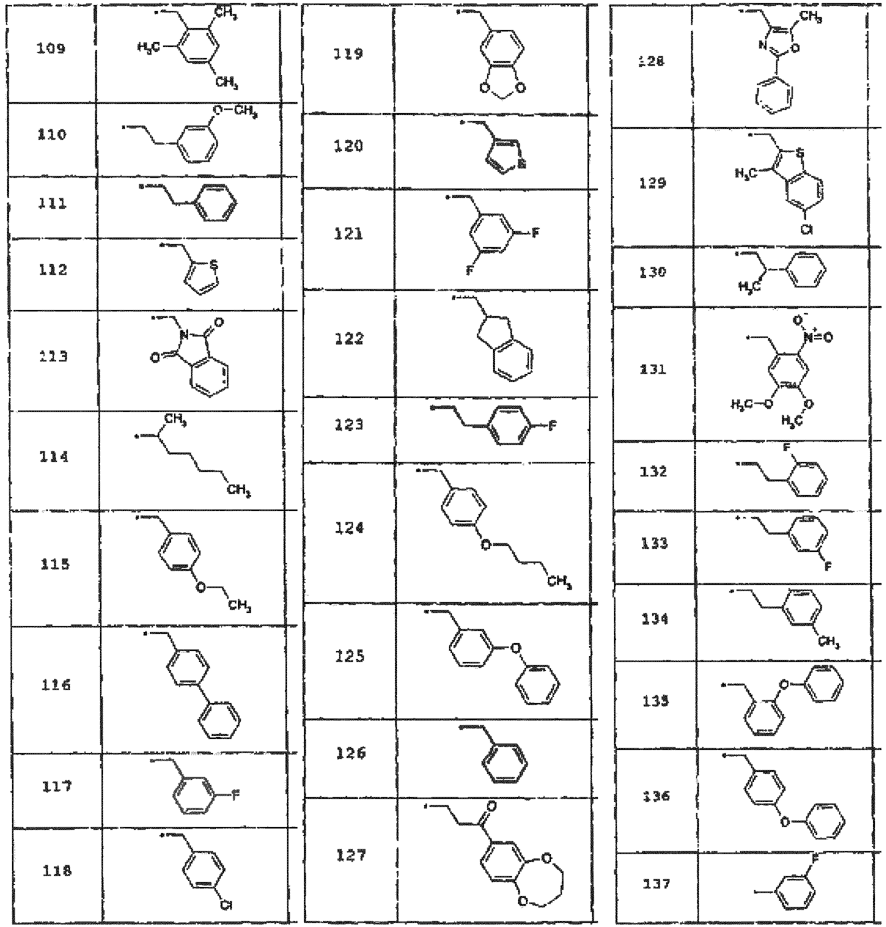

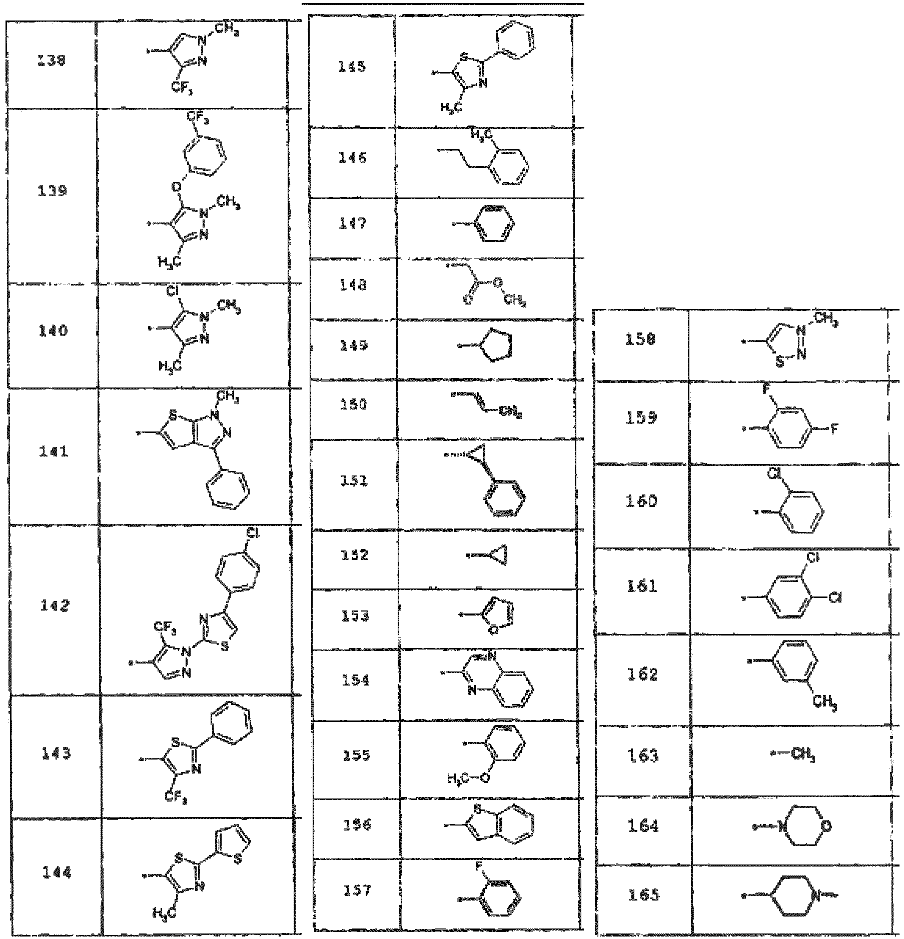

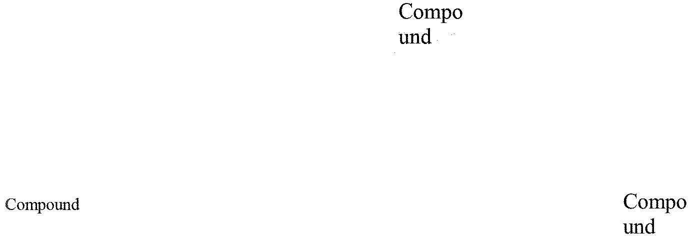

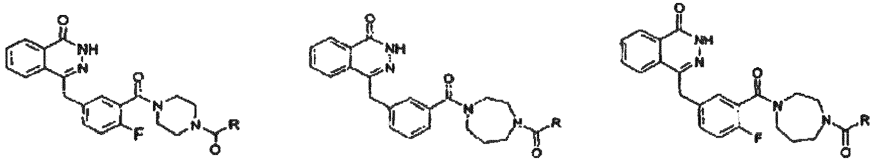

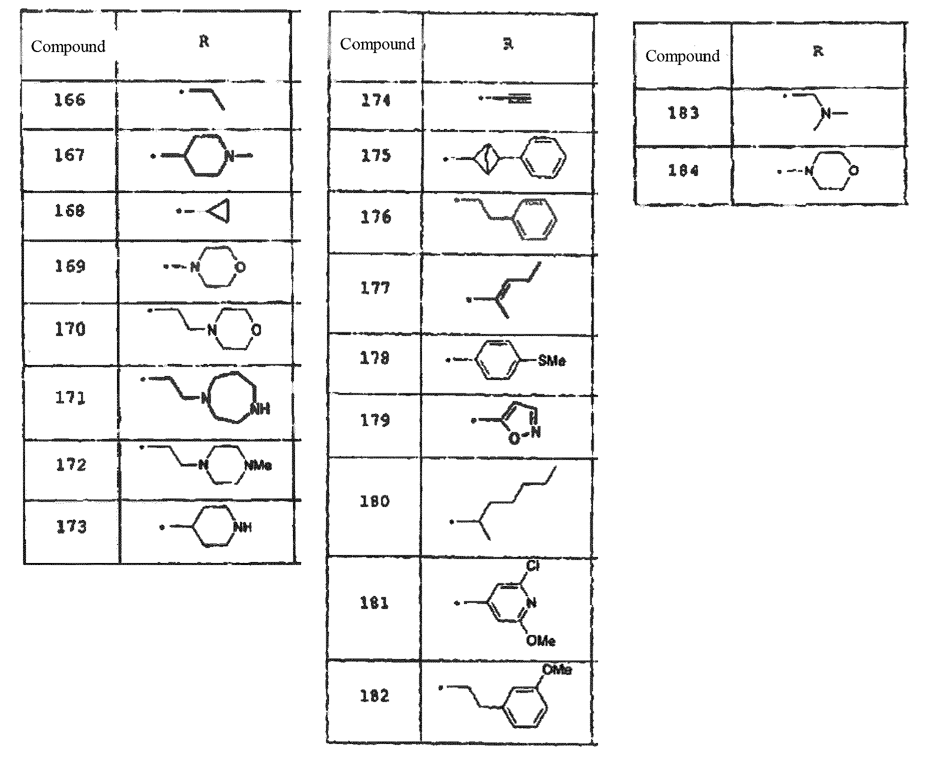

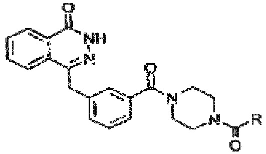

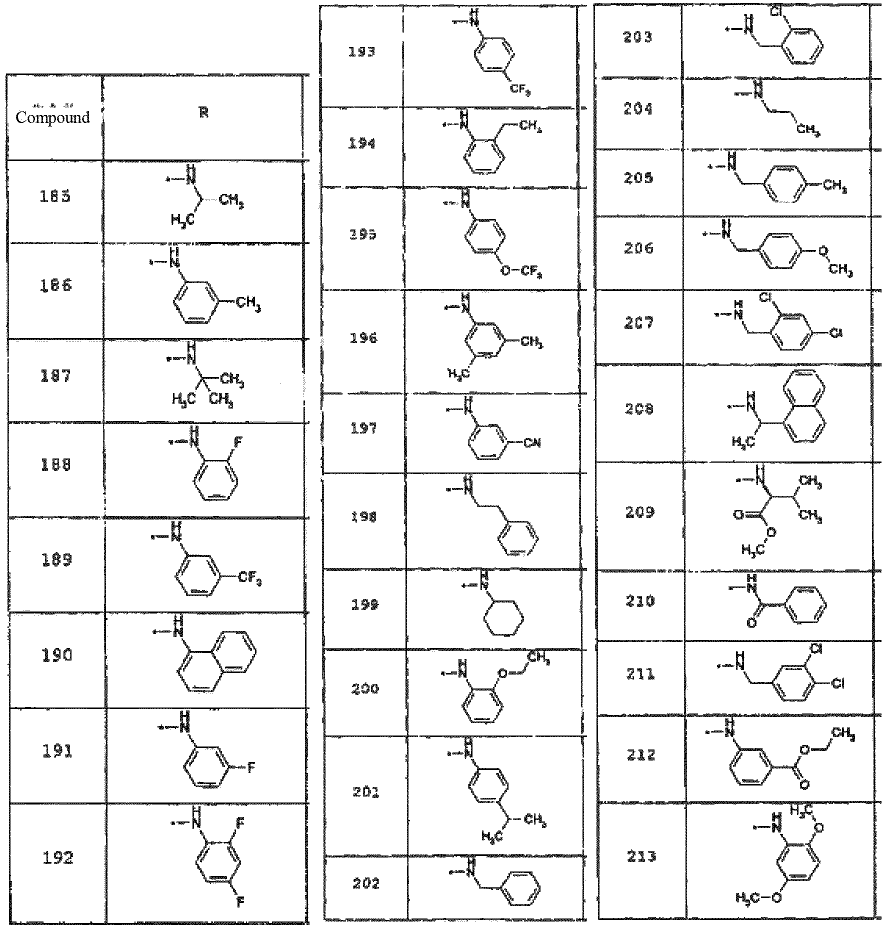

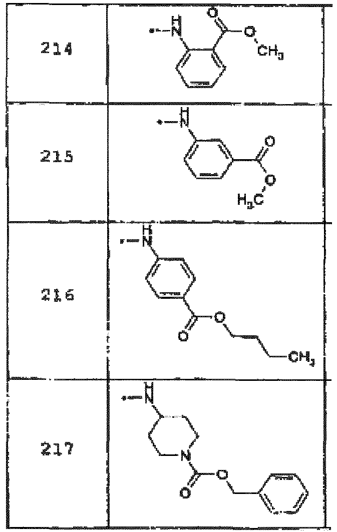

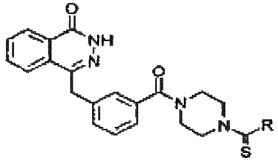

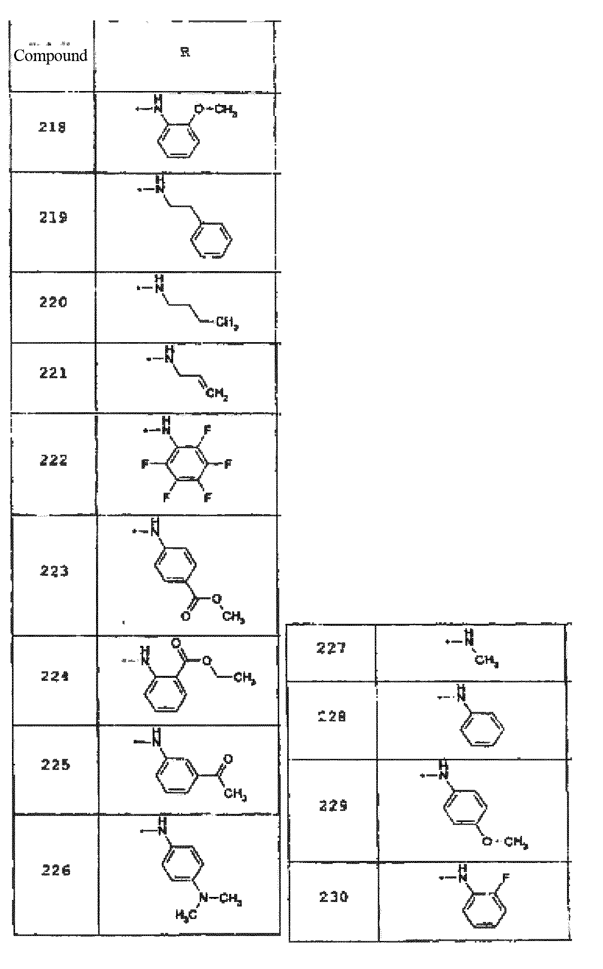

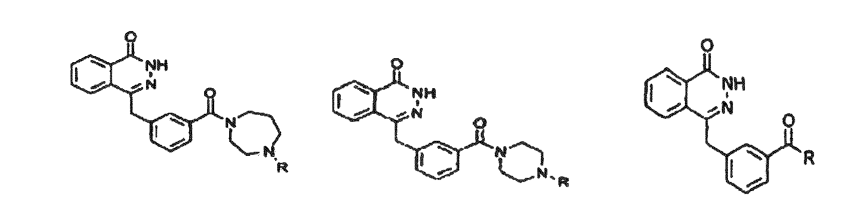

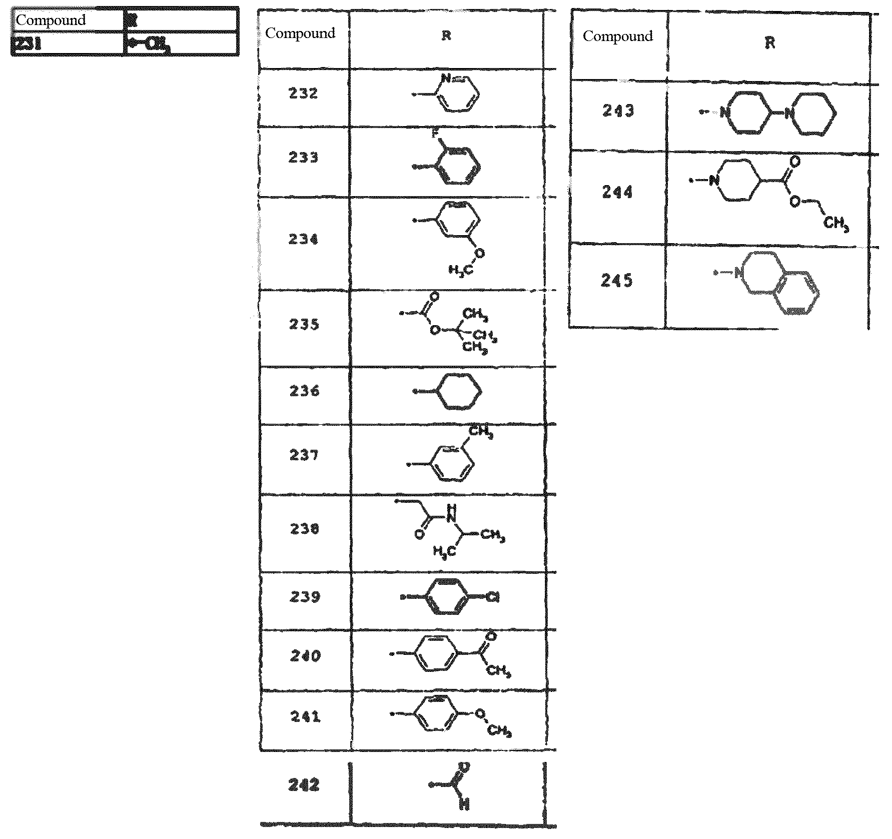

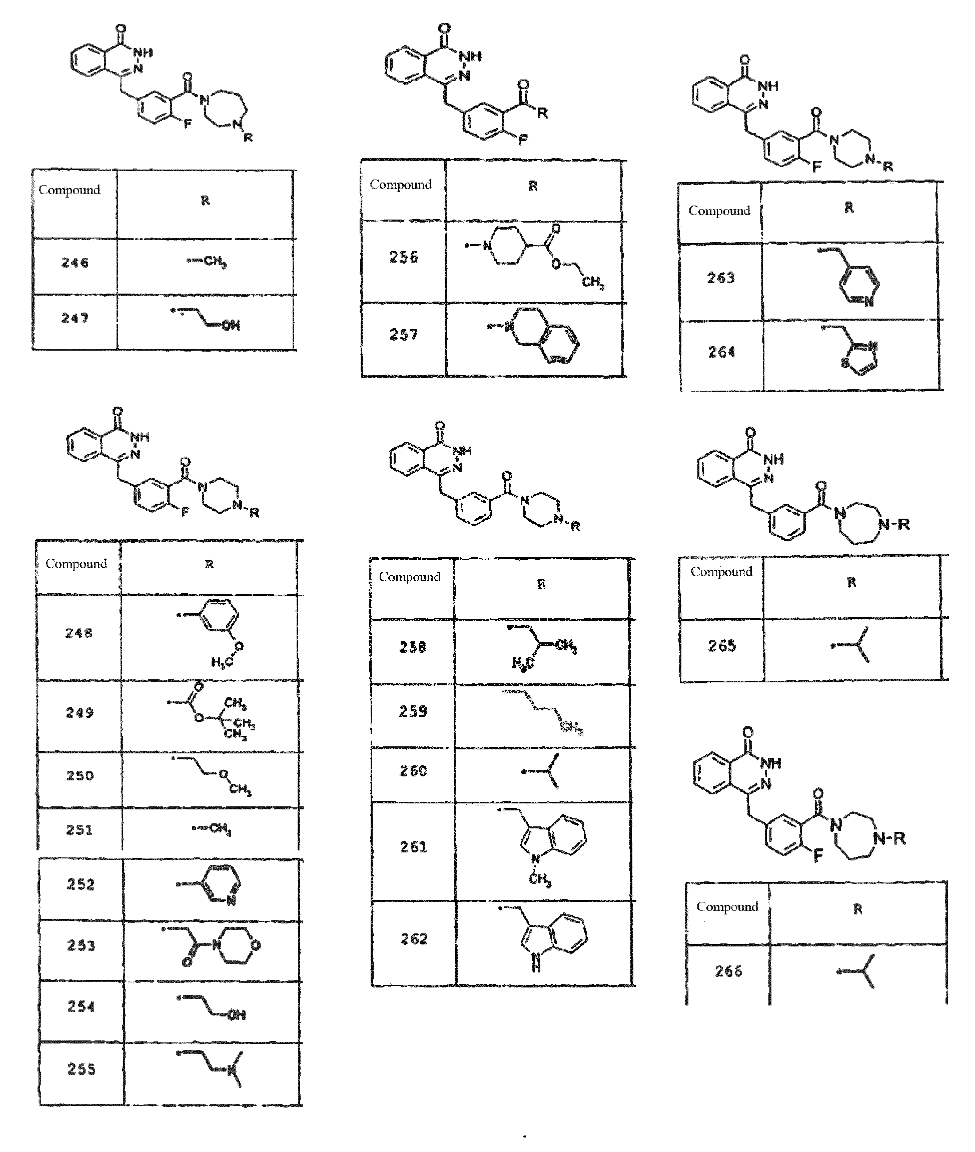

In an embodiment, the compound shown in formula **III** is any of the following compounds:

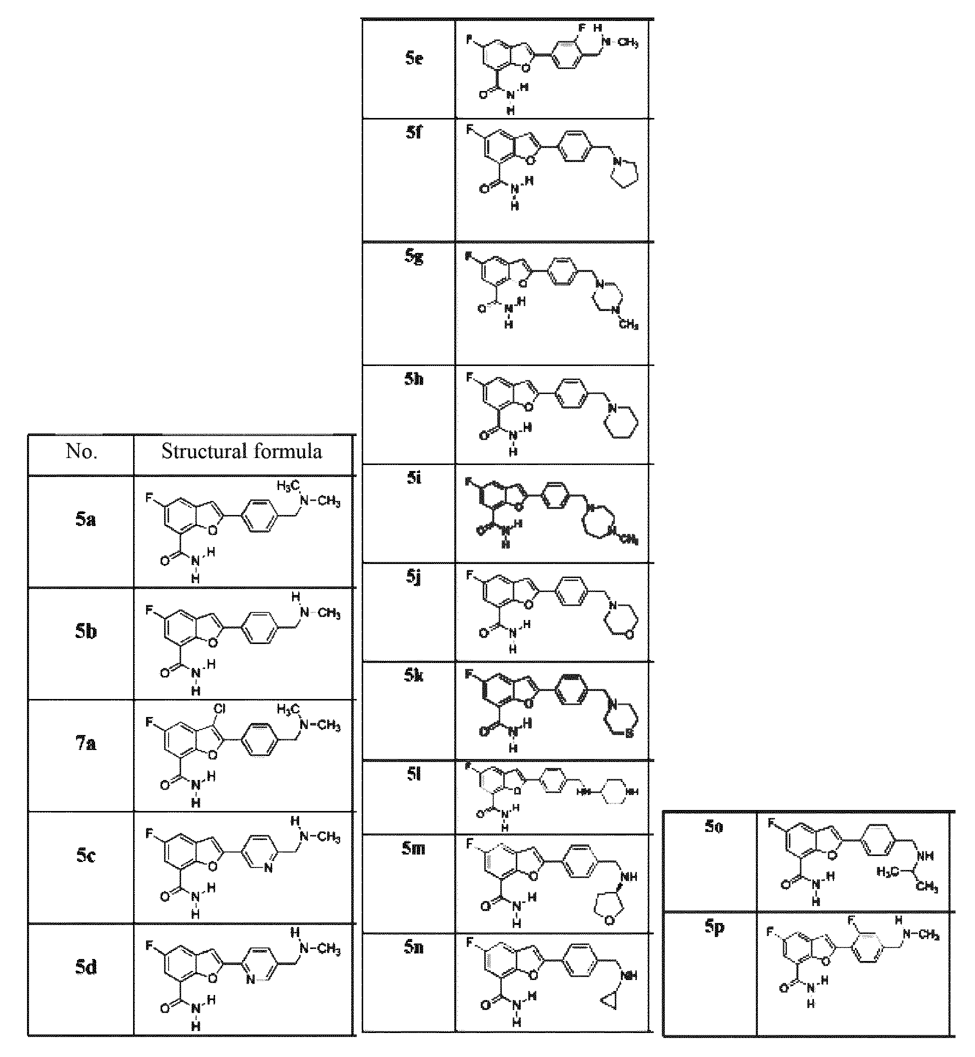

In an embodiment, the compound shown in formula **IV** is any of the following compounds:
2-Phenyl-2H-indazole-7-carboxamide;
2-(3-chlorophenyl)-2H-indazole-7-carboxamide; and
2-{4-[(dimethylamino)methyl]phenyl}-2H-indazole-7-carboxamide;
2- {4-[(N,N-dimethylglycyl)amino]phenyl} -2H-indazole-7-carboxamide;
2-benzyl-2H-indazole-7-carboxamide; 2-(4-chlorophenyl)-2H-indazole-7-carboxamide;
2-(2-chlorophenyl)-2H-indazole-7-carboxamide;
2-{4-[(4-methylpiperazin-1-yl)methyl]phenyl} -2H-indazole-7-carboxamide;
2- [4-(morpholine-4-ylmethyl)phenyl] -2H-indazole-7-carboxamide;
2- {4-[(methylamino)methyl]phenyl} -2H-indazole-7-carboxamide;
2-[4-(pyrrolidin-1-ylmethyl)phenyl]-2H-indazole-7-carboxamide;
2-[4-(piperidin-1-ylmethyl)phenyl]-2H-indazole-7-carboxamide;
{4-[7-(aminocarbonyl)-2H-indazole-2-yl]phenyl} -N,N-dimethylmethane ammonium chloride;
trifluoroacetate 4-[((4-[7-(aminocarbonyl)-2H-indazole-2-yl]benzoyl}amino)methyl]pyridinium;
2-{4-[1-(methylamino)ethyl]phenyl}-2H-indazole-7-carboxamide;
N- {4-[7-(aminocarbonyl)-2H-indazole-2-yl]benzyl} cyclohexanetrifluoroacetate;
{4-[7-(aminocarbonyl)-4-chloro-2H-indazole-2-yl]phenyl}-N-methylmethane ammonium trifluoroacetate; 2-phenyl-2H-1,2,3 -benzotriazole-4-carboxamide;
2-benzyl-2H-1,2,3-benzotriazole-4-carboxamide;
2- {3-[(methylamino)methyl]phenyl} -2H-indazole-7-carboxamide; trifluoroacetate 4-({3-[7-(aminocarbonyl)-2H-indazole-2-yl]benzoyl}amino)piperidinium;
{4-[7-(aminocarbonyl)-2H-Indazole-2-yl]phenyl} -N-methylmethane ammonium chloride;
2- {3-chloro-4-[(dimethylamino)methyl]phenyl} -2H-indazole-7-carboxamide; trifluoroacetate 1-[2-({4-[7-(aminocarbonyl)-2H-indazole-2-yl]phenyl}amino)-2-oxoethyl]-4-methylpiperazine -1-onium;
2-(4-{[(4-pyrrolidine-1-ylpiperidin-1-yl)acetyl]amino}phenyl)-2H-indazole-7-carboxamide;
2-{4-[(pyrrolidin-1-ylacetyl)amino]phenyl}-2H-indazole-7-carboxamide;
2-{4-[(piperidin-1-ylacetyl)amino]phenyl}-2H-indazole-7-carboxamide;
2-{4-[(morpholin-4-ylacetyl)amino]phenyl}-2H-indazole-7-carboxamide; chloride
4-[2-({4-[7-(aminocarbonyl)-2H-indazole-2-yl]phenyl}amino)-2-oxoethyl]morpholine-4-oniu
m; 2- {4-[(ethylamino)methyl]phenyl} -2H-indazole-7-carboxamide;
2- {4-[(isopropylamino)methyl]phenyl} -2H-indazole-7-carboxamide;
N- {4-[7-(aminocarbonyl)-2H-indazole-2-yl]benzyl} propane-2-ammonium chloride;
2-(4-{[(2-fluoroethyl)amino]methyl}phenyl)-2H-indazole-7-carboxamide;
2-(4-{[(2,2-difluoroethyl)amino]methyl}phenyl)-2H-indazole-7-carboxamide;
2- {4-[(cyclopropylamino)methyl]phenyl}-2H-Indazole-7-carboxamide;
4- {4-[7-(aminocarbonyl)-2H-indazole-2-yl]benzoyl} -1,4-diazabicycloheptane-1-onium;
2-({4-[7-(aminocarbonyl)-2H-indazole-2-yl]benzoyl}amino)-N,N-dimethylethane ammonium trifluoroacetate; trifluoroacetate 4-[({4-[7-(aminocarbonyl)-2H-indazole-2-yl]benzoyl}amino)methyl]piperidinium;
N-{4-[7-(aminocarbonyl)-2H-indazole-2-yl]benzyl}-N,N',N'-trimethylethane-1,2-diammonium dichloride; trifluoroacetate 4-{4-[7-(aminocarbonyl)-2H-indazole-2-yl]benzoyl}-1-methylpiperazine-1-onium;
trifluoroacetate 3-[({4-[7-(aminocarbonyl)-2H-indazole-2-yl]phenyl}amino)carbonyl]azacyclobutanium;
trifluoroacetate (2S)-2-[({4-[7-(aminocarbonyl)-2H-indazole-2-yl]phenyl}amino)carbonyl]-1-methylpyrrolidin ium; trifluoroacetate 3-[({4-[7-(aminocarbonyl)-2H-indazole-2-yl]phenyl}amino)carbonyl]-1-methylpiperidinium;
trifluoroacetate 4-[({4-[7-(aminocarbonyl)-2H-indazole-2-yl]phenyl}amino)carbonyl]-1-methylpiperidinium;
trifluoroacetate 4-({4-[7-(aminocarbonyl)-2H-indazole-2-yl]benzoyl}amino)-1-benzylpiperidinium;
2- {4-[(pyridin-4-ylamino)carbonyl]phenyl} -2H-indazole-7-carboxamide;
2- {4-[(4-phenylpiperazin-1-yl)carbonyl]phenyl}-2H-indazole-7-carboxamide;
2-(4-{[methyl(quinoxalin-6-ylmethyl)amino]carbonyl}phenyl)-2H-indazole-7-carboxamide;
2-(4-formylphenyl)-2H-indazole-7-carboxamide; trifluoroacetate
1-[2-({4-[7-(aminocarbonyl)-2H-indazole-2-yl]benzoyl}amino)ethyl]pyrrolidinium;
trifluoroacetate 1-[2-({4-[7-(aminocarbonyl)-2H-indazole-2-yl]benzoyl}amino)ethyl]piperidinium;
trifluoroacetate 4-[2-({4-[7-(aminocarbonyl)-2H-indazole-2-yl]benzoyl}amino)ethyl]morpholine-4-onium;
trifluoroacetate
4-({4-[7-(aminocarbonyl)-2H-indazole-2-yl]benzoyl}amino)-1-methylpiperidinium;
2-[4-[(4-methylpiperazin-1-yl)methyl]-3-(trifluoromethyl)phenyl]-2H-indazole-7-carboxamide;
2-[4-[(methylamino)methyl]-3-(trifluoromethyl)phenyl]-2H-indazole-7-carboxamide;
1-{4-[7-(aminocarbonyl)-2H-indazole-2-yl]phenyl} -N-methylethane ammonium chloride; 2-[4-(pyrrolidin-1-ylmethyl)-3-(trifluoromethyl)phenyl]-2H-indazole-7-carboxamide;
2-[4-(piperidin-1-ylmethyl)-3-(trifluoromethyl)phenyl]-2H-indazole-7-carboxamide;
2-[4-[(ethylamino)methyl]-3-(trifluoromethyl)phenyl]-2H-indazole-7-carboxamide;
trifluoroacetate 4-{4-[7-(aminocarbonyl)-4-chloro-2H-indazole-2-yl]benzyl}-1-methylpiperazine-1-onium;
bis(trifluoroacetic acid) 1-[2-({4-[7-(aminocarbonyl)-2H-indazole-2-yl]benzyl}ammonio)ethyl]piperidinium;
bis(trifluoroacetic acid) 4-[2-({4-[7-(aminocarbonyl)-2H-indazole-2-yl]benzyl}ammonio)ethyl]morpholine-4-onium;
bis(trifluoroacetic acid)l-[2-({4-[7-(aminocarbonyl)-2H-indazole-2-yl]benzyl}ammonio)ethyl]pyrrolidinium;
bis(trifluoroacetic acid) 4-({4-[7-(aminocarbonyl)-2H-indazole-2-yl]benzyl}ammonio)-1-methylpiperidinium;
bis(trifluoroacetic acid)4-({4-[7-(aminocarbonyl)-2H-indazole-2-yl]benzyl}ammonio)-1-benzylpiperidinium;
bis(trifluoroacetic acid)1-{4-[7-(aminocarbonyl)-2H-indazole-2-yl]benzyl}-4-benzylpiperidinium;
N- {4-[7-(aminocarbonyl)-2H-indazole-2-yl]benzyl} -2-(dimethylamino)-2-oxoethyl alkyltrifluoroacetate ammonium; bis(trifluoroacetic acid) 2-[({4-[7-(aminocarbonyl)-2H-indazole-2-yl]benzyl}ammonio)methyl]pyridinium;
bis(trifluoroacetic acid) 4-[({4-[7-(aminocarbonyl)-2H-indazole-2-yl]benzyl}ammonio)methyl]pyridinium;
N-{4-[7-(aminocarbonyl)-2H-indazole-2-yl]benzyl)-2-methylpropane-2-ammonium trifluoroacetate; bis(trifluoroacetic acid)N'-{4-[7-(aminocarbonyl)-2H-indazole-2-yl]benzyl}-N,N-dimethylethane-1,2-diammoniu m; {4-[7-(aminocarbonyl)-2H-indazole-2-yl]phenyl}-N-(1,3-oxazol-2-ylmethyl)methane ammonium trifluoroacetate; chloride 7-[7-(aminocarbonyl)-2H-indazole-2-yl]-1,2,3,4-tetrahydroisoquinolinium; chloride 6-[7-(aminocarbonyl)-2H-indazole-2-yl]-1,2,3,4-tetrahydroisoquinolinium; trifluoroacetate 5-[7-(aminocarbonyl)-2H-indazole-2-yl]-1,2,3,4-tetrahydroisoquinolinium; trifluoroacetate 3-[({4-[7-(aminocarbonyl)-5-fluoro-2H-indazole-2-yl]phenyl}amino)carbonyl]azacyclobutaniu m; 2-(4-{[(azetidin-3-ylcarbonyl)(methyl)amino]methyl}phenyl)-2H-indazole-7-carboxamide;
trifluoroacetate 3-[({4-[7-(aminocarbonyl)-2H-indazole-2-yl]benzyl} amino)carbonyl]azacyclobutanium; 2-(4-bromophenyl)-5-fluoro-2H-indazole-7-carboxamide;
5-fluoro-2-(4-pyridine-3-ylphenyl)-2H-indazole-7-carboxamide;
2-(4-pyridin-3-ylphenyl)-2H-indazole-7-carboxamide; trifluoroacetate 4-{4-[7-(aminocarbonyl)-2H-indazole-2-yl]phenyl}-1-methylpiperazine-1-onium;
trifluoroacetate 4- {4-[7-(aminocarbonyl)-2H-indazole-2-yl]phenyl} piperidinium;
2- {4-[7-(aminocarbonyl)-2H-indazole-2-yl]phenyl} -N-methylethane ammonium trifluoroacetate; trifluoroacetate 2-[({4-[7-(aminocarbonyl)-2H-indazole-2-yl]phenyl}amino)carbonyl]azacyclobutanium;
2-{5-[(methylamino)methyl]pyridin-2-yl}-2H-indazole-7-carboxamide;
5-fluoro-2-{3-fluoro-4-[(methylamino)methyl]phenyl}-2H-indazole-7-carboxamide;
5-fluoro-2- {4-[(methylamino)methyl]phenyl} -2H-indazole-7-carboxamide trifluoroacetate; 2-{4-[7-(aminocarbonyl)-2H-indazole-2-yl]phenyl}-N-methylpropane-2-ammonium trifluoroacetate; 2-(6-phenylpyridazine-3-yl)-2H-indazole-7-carboxamide;
{4-[7-(aminocarbonyl)-5-fluoro-2H-indazole-2-yl]phenyl}-N-{[1-(hydroxylmethyl)cyclohexyl ]methyl}methane ammonium trifluoroacetate;
5-chloro-2-(4-formylphenyl)-2H-indazole-7-carboxamide;
2-{3-methoxy-4-[(4-methylpiperazin-1-yl)methyl]phenyl} -2H-indazole-7-carboxamide;
2- {3-methoxy-4-[(methylamino)methyl]phenyl} -2H-indazole-7-carboxamide;
5-chloro-2- {4-[(4-methylpiperazin-1-yl)methyl]phenyl} -2H-indazole-7-carboxamide;
5-chloro-2-{4-[(methylamino)methyl]phenyl}-2H-indazole-7-carboxamide;
{4-[7-(aminocarbonyl)-4-fluoro-2H-indazole-2-yl]phenyl} -N-methylmethane ammonium chloride; {4-[7-(aminocarbonyl)-5-fluoro-2H- indazole-2-yl]phenyl} -N-methylmethane ammonium chloride; chloride 1-{4-[7-(aminocarbonyl)-4-fluoro-2H-indazole-2-yl]benzyl}-4-methylpiperazine-1-onium;
chloride 1-{4-[7-(aminocarbonyl)-5-fluoro-2H-indazole-2-yl]benzyl} -4-methylpiperazine-1-onium;
bis(trifluoroacetic acid) 1-{3-[7-(aminocarbonyl)-2H-indazole-2-yl]benzyl} -4-methylpiperazinium;
2-[4-(1-hydroxy-1-methylethyl)phenyl]-2H-indazole-7-carboxamide;
2-(4-acetylphenyl)-2H-indazole-7-carboxamide; trifluoroacetate 3-{[{4-[7-(aminocarbonyl)-2H-indazole-2-yl]benzyl}(methyl)amino]carbonyl}-1-methylpiperi dinium; 2-{4-[1-(formylamino)-1-methylylethyl]phenyl} -2H-indazole-7-carboxamide;
2-[3-(1,4-diazabicycloheptane-1-ylcarbonyl)phenyl]-2H-indazole-7-carboxamide;
trifluoroacetate 3-[({4- [7-(aminocarbonyl)-2H-indazole-2-yl]benzyl}amino)carbonyl]-1-methylpiperidinium;
trifluoroacetate (2S)-2-[({4-[7-(aminocarbonyl)-2H-indazole-2-yl]phenyl}amino)carbonyl]pyrrolidinium;
trifluoroacetate 3-[({4- [7-(aminocarbonyl)-2H-indazole-2-yl]phenyl} amino)carbonyl]pyrrolidinium;
trifluoroacetate (2R)-2-[({4-[7-(aminocarbonyl)-2H-indazole-2-yl]phenyl}amino)carbonyl]pyrrolidinium;
trifluoroacetate 3-[({4-[7-(aminocarbonyl)-2H-indazole-2-yl]phenyl} amino)carbonyl]piperidinium;
trifluoroacetate (3R)-3-[({4-[7-(aminocarbonyl)-2H-indazole-2-yl]phenyl}amino)carbonyl]-1-methylpyrrolidin ium; trifluoroacetate 2-[({4-[7-(aminocarbonyl)-2H-indazole-2-yl]phenyl}amino)carbonyl]-1-methylpiperidinium;
4-chloro-2-(4-formylphenyl)-2H-indazole-7-carboxamide; trifluoroacetate (3S)-3-[({4-[7-(aminocarbonyl)-2H-indazole-2-yl]phenyl}amino)carbonyl]-1-methylpyrrolidin ium; (R)-1-{4-[7-(aminocarbonyl)-2H-indazole-2-yl]phenyl}-N-methylethane ammonium chloride; (S)-1-{4-[7-(aminocarbonyl)-2H-indazole-2-yl]Phenyl}-N-methylethaneammonium chloride; 2- {3-fluoro-4-[(methylamino)methyl]phenyl} -2H-indazole-7-carboxamide;
{4-[7-(aminocarbonyl)-2H-indazole-2-yl]-2-fluorophenyl} -N-methane ammonium trifluoroacetate; 2-{4-[1-methyl-1-(methylamino)ethyl]phenyl} -2H-indazole-7-carboxamide;
trifluoroacetate 1- {4-[7-(aminocarbonyl)-2H-indazole-2-yl]-2-hydroxybenzyl}-4-methylpiperazine-1-onium;
chloride (3R)-3-[({4-[7-(aminocarbonyl)-2H-indazole-2-yl]phenyl}amino)carbonyl]-1-methylpiperidini um; chloride (3S)-3-[({4-[7-(aminocarbonyl)-2H-indazole-2-yl]phenyl}amino)carbonyl]-1-methyl chloride piperidinium; bis(trifluoroacetic acid)1-(2-{4-[7-(aminocarbonyl)-2H-indazole-2-yl]phenyl} ethyl)-4-methylpiperazinedinium;
{4-[7-(aminocarbonyl)-4-hydroxy-2H-indazole-2-yl]phenyl} -N-ammonium methylmethane trifluoroacetate; trifluoroacetate 2-[({4-[7-(aminocarbonyl)-2H-indazole-2-yl]phenyl}amino)carbonyl]-4-phenylpyrrolidinium;
(1R,3S)-3-[({4-[7-(aminocarbonyl)-2H-indazole-2-yl]phenyl}amino)carbonyl]cyclopentane ammonium trifluoroacetate;
(1R,3R)-3-[({4-[7-(aminocarbonyl)-2H-indazole-2-yl]phenyl}amino)carbonyl]cyclopentane ammonium trifluoroacetate;
(1S,3R)-3-[({4-[7-(aminocarbonyl)-2H-indazole-2-yl]phenyl}amino)carbonyl]cyclopentane ammonium trifluoroacetate; trifluoroacetate 2-[({4-[7-(aminocarbonyl)-2H-indazole-2-yl]phenyl}amino)carbonyl]-2-methylazetidinium; trifluoroacetate 4-[2-({4-[7-(aminocarbonyl)-2H-indazole-2-yl]phenyl}amino)-2-oxoethyl]-1-methylpiperidini um;
9-[2-({4-[7-(aminocarbonyl)-2H-indazole-2-yl]phenyl}amino)-2-oxoethyl]-3-azaspiro[5.5]und ecane trifluoroacetate; trifluoroacetate 4-[2-({4-[7-(aminocarbonyl)-2H-indazole-2-yl]phenyl}amino)-2-oxoethyl]-4-phenylpiperidini um; trifluoroacetate 2-[({4-[7-(aminocarbonyl)-2H-indazole-2-yl]phenyl}amino)carbonyl]pyridinium;
trifluoroacetate 4-{3-[({4-[7-(aminocarbonyl)-2H-indazole-2-yl]phenyl}amino)carbonyl]pyridin-2-yl}piperazi ne-1-onium; trifluoroacetate 3-[({4-[7-(aminocarbonyl)-2H-indazole-2-yl]phenyl}amino)carbonyl]pyridinium;
trifluoroacetate 4-[({4-[7-(aminocarbonyl)-2H-indazole-2-yl]phenyl}amino)carbonyl]pyridinium;
trifluoroacetate 4-[({4-[7-(aminocarbonyl)-2H-indazole-2-yl]phenyl}amino)carbonyl]quinolinium;
trifluoroacetate 4-[({4-[7-(aminocarbonyl)-2H-indazole-2-yl]phenyl}amino)carbonyl]isoquinolinium;
trifluoroacetate 2-[({4-[7-(aminocarbonyl)-2H-indazole-2-yl]phenyl}amino)carbonyl]-1-methylazepanium;
trifluoroacetate 3-[({4-[7-(aminocarbonyl)-2H-indazole-2-yl]phenyl}amino)carbonyl]-2-methyl-1,2,3,4-tetrahy droisoquinolinium; trifluoroacetate 2-{4-[({4-[7-(aminocarbonyl)-2H-indazole-2-yl]phenyl}amino)carbonyl]piperidin-1-yl}pyrimi dine-1-onium; chloride 1-{4-[7-(aminocarbonyl)-5-fluoro-2H-indazole-2-yl]benzyl} -4-methylpiperazine-1-onium;
5-[({4-[7-(aminocarbonyl)-2H-indazole-2-yl]phenyl}amino)carbonyl]-3-oxoswainsonine-2-ammoniu m trifluoroacetate; trifluoroacetate 2-{3-((4-[7-(aminocarbonyl)-2H-indazole-2-yl]phenyl}amino)carbonyl]piperidin-1-yl}pyridini um; trifluoroacetate 2-[({4-[7-(aminocarbonyl)-2H-indazole-2-yl]phenyl}amino)carbonyl]-4-methylmorpholin-4-o nium;
(1R,4R)-N-{4-[7-(aminocarbonyl)-2H-indazole-2-yl]phenyl}-1'-(methylsulfonyl)-1',2'-dihydro spiro[cyclohexane-1,3'-indole]-4-carboxamide; trifluoroacetate 1-[({4-[7-(aminocarbonyl)-2H-indazole-2-yl]phenyl}amino)carbonyl]octahydro-1H-isoindoliu m; trifluoroacetate 2-[({4-[7-(aminocarbonyl)-2H-indazole-2-yl]phenyl}amino)carbonyl]-4-benzylmorpholine-4-o nium; trifluoroacetate (3S, 4R)-3-[({4-[7-(aminocarbonyl)-2H-indazole-2-yl]phenyl}amino)carbonyl]-4-(methoxycarbony l)pyrrolidinium; bis(trifluoroacetic acid) 4-{(2S)-2-[({4-[7-(aminocarbonyl)-2H-indazole-2-yl]phenyl}amino)carbonyl]pyrrolidinium-1-yl} piperidinium;
(1S,3S)-3-[({4-[7-(aminocarbonyl)-2H-indazole-2-yl]phenyl}amino)carbonyl]cyclopentane ammonium trifluoroacetate; trifluoroacetate 3-[({4- [7-(aminocarbonyl)-2H-indazole-2-yl]phenyl}amino)carbonyl]-1-methylpyrrolidinium;
trifluoroacetate 2-{4-[({4-[7-(aminocarbonyl)-2H-indazole-2-yl]phenyl}amino)carbonyl]piperidin-1-yl}pyrimi dine-1-onium; bis(trifluoroacetic acid)2-(1-{4-[7-(aminocarbonyl)-2H-indazole-2-yl]benzyl}pyrrolidinium-3-yl)pyridinium;
bis(trifluoroacetic acid) 3-(1-{4-[7-(aminocarbonyl)-2H-indazole-2-yl]benzyl}pyrrolidinium-3-yl)pyridinium;
trifluoroacetate (3S,4S)-1-{4-[7-(amino carbonyl)-2H-indazole-2-yl]benzyl}-3,4-difluoropyrrolidinium;
3- {4-[7-(aminocarbonyl)-2H-indazole-2-yl]benzyl} -6-amino-3-azabicyclo[3.1.0]hexanebis(trif luoroacetate); 2-{4-[7-(aminocarbonyl)-2H-indazole-2-yl]benzyl} -7-methyl-2,7-diazonium heterospiro[4.4]nonane bis(trifluoroacetate); bis(trifluoroacetic acid) 1-{4-[7-(aminocarbonyl)-2H-indazole-2-yl]benzyl} -3-[4-(dimethylammonio)phenyl]pyrrolidin ium; bis(trifluoroacetic acid)5-{4-[7-(aminocarbonyl)-2H-indazole-2-yl]benzyl}-1-methyl-1,2,4,5,6,6a-hexahydropyrr olo[3,4-b]pyrrolidinium; bis(trifluoroacetic acid) 3-{[{4-[7-(aminocarbonyl)-2H-indazole-2-yl]benzyl}(methyl)amino]methyl}-1-methylpiperidi nium;
(1R,4S)-5-{4-[7-(aminocarbonyl)-2H-indazole-2-yl]benzyl}-2-oxa-5-azoniabicyclo[22.1]hept ane trifluoroacetate;
N-{4-[7-(aminocarbonyl)-2H-indazole-2-yl]benzyl}-2-hydroxy-2-methylpropane-1-ammoniu m trifluoroacetate;
N- {4-[7-(aminocarbonyl)-2H-indazole-2-yl]benzyl} -3,3-difluorocyclobutane ammonium trifluoroacetate; trifluoroacetate 4- {4-[7-(aminocarbonyl)-2H-indazole-2-yl]benzyl} -6-fluoro-1,4-diazabicycloheptane-1-onium; bis(trifluoroacetic acid) 1-{4-[7-(aminocarbonyl)-2H-indazole-2-yl]benzyl}-4-pyrimidin-1-onium-2-yl-1,4-diazabicycl oheptane-1-onium; bis(trifluoroacetic acid) 3-({4-[7-(aminocarbonyl)-2H-indazole-2-yl]benzyl)ammonio)-1-benzylpyrrolidinium;
bis(trifluoroacetic acid) 3-[({4- [7-(aminocarbonyl)-2H-indazole-2-yl]benzyl} ammonio)methyl]-1-methylpyrrolidinium; bis(trifluoroacetic acid) 3-[({4-[7-(aminocarbonyl)-2H-indazole-2-yl]benzyl}ammonio)methyl]-1-benzylpyrrolidinium;
2-{4-[7-(aminocarbonyl)-2H-indazole-2-yl]benzyl}-7-benzyl-2,7-diazaspiro[4.4]nonanebis(trif luoroacetate); 2- {4-[7-(aminocarbonyl)-2H-indazole-2-yl]benzyl} -8-benzyl-2,8-diazonium heterospiro[5.5]undecanbis(trifluoroacetate);
2- {4-[7-(aminocarbonyl)-2H-indazole-2-yl]benzyl} -2,6-diazonium heterospiro[3.3]heptanebis(trifluoroacetate);
7- {4-[7-(aminocarbonyl)-2H-indazole-2-yl]benzyl} -2,7-diazonium heterospiro[3.5]nonanebis(trifluoroacetate);
2- {4-[7-(aminocarbonyl)-2H-indazole-2-yl]benzyl} -2,6-diazonium heterospiro[3.5]nonanebis(trifluoroacetate);
2-{4-[7-(aminocarbonyl)-2H-indazole-2-yl]benzyl}-2,8-diazonium heterospiro[5.5]undecanbis(trifluoroacetate);
2-{4-[7-(aminocarbonyl)-2H-indazole-2-yl]benzyl}-2,8-diazonium heterospiro[4.5]decanebis(trifluoroacetate);
2- {4-[7-(aminocarbonyl)-2H-indazole-2-yl]benzyl} -2,7-diazonium heterospiro[4.5]decanebis(trifluoroacetate);
8- {4-[7-(aminocarbonyl)-2H-indazole-2-yl]benzyl} -2,8-diazonium heterospiro[4.5]decanebis(trifluoroacetate);
3- {4-[7-(aminocarbonyl)-2H-indazole-2-yl]benzyl} -3,9-diazonium heterospiro[5.5]undecanbis(trifluoroacetate); bis(trifluoroacetic acid) 2-{4-[7-(aminocarbonyl)-2H-indazole-2-yl]benzyl}octahydropyrrolo[3,4-c]pyrrolidinium;
bis(trifluoro acetic acid) 5-{4-[7-(aminocarbonyl)-2H-indazole-2-yl]benzyl}octahydropyrrolo[3,4-b]pyrrolidinium;
bis(trifluoroacetic acid) 4-({4-[7-(Aminocarbonyl)-2H-indazole-2-yl]benzyl}ammonio)octahydrocyclopentadieno[c]py rrolidinium; N²-{4-[7-(Amino) carbonyl)-2H-indazole-2-yl]benzyl}-N¹,N¹-dimethyl-1-pyridin-2-ylethane-1,2-bis(trifluoroacet ic acid)diammonium; bis(trifluoroacetic acid) 7-(aminocarbonyl)-2-[4-({[2-(2,3-dihydro-1H-indol-1-yl)ethyl]ammonium}methyl)phenyl]-2H -indazole-1-onium; bis(trifluoroacetic acid)(3S,4S)-1-[2-({4-[7-(aminocarbonyl)-2H-indazole-2-yl]benzyl}ammonio)ethyl]-3,4-diflu oropyrrolidinium; trifluoroacetate 5-({4-[7-(aminocarbonyl)-2H-indazole-2-yl]benzyl}amino)-1,3-benzothiazole-3-onium;
1-({4-[7-(aminocarbonyl)-2H-indazole-2-yl]benzyl}ammonio)-8-diazonium heterospiro[4.5]decanebis(trifluoroacetate); bis(trifluoroacetic acid) 4-[({4-[7-(aminocarbonyl)-2H-indazole-2-yl]benzyl}ammonio)methyl]-1-methylpiperidinium;
N- {4-[7-(aminocarbonyl)-2H-indazole-2-yl]benzyl} -2-hydroxyethane ammonium trifluoroacetate; trifluoroacetate 7-[7-(aminocarbonyl)-2H-indazole-2-yl]-1,2,3,4-tetrahydroisoquinolinium; trifluoroacetate 3-[2-({4-[7-(aminocarbonyl)-2H-indazole-2-yl]phenyl}amino)-2-oxoethyl]azacyclobutanium;
bis(trifluoroacetic acid)4-({4-[7-(aminocarbonyl)-2H-indazole-2-yl]benzyl} ammonio)piperidinium;
bis(trifluoroacetic acid)(3R,4R)-4-({4-[7-(aminocarbonyl)-2H-indazole-2-yl]benzyl}ammonio)-3-fluoropiperidin ium; bis(trifluoroacetic acid)(3S,4R)-4-({4-[7-(aminocarbonyl)-2H-indazole-2-yl] benzyl}ammonio)-3-benzyl-1-methylpiperidinium;
N- {4-[7-(aminocarbonyl)-2H-indazole-2-yl]benzyl}-1-isobutylpiperidine-4-ammonium trifluoroacetate; bis(trifluoroacetic acid) 2-[4-({4-[7-(aminocarbonyl)-2H-indazole-2-yl]benzyl}ammonium)piperidine-1-yl]-3-methylp yridinium; bis(trifluoroacetic acid) 3-({4-[7-(aminocarbonyl)-2H-indazole-2-yl]benzyl}ammonio) pyridinium; bis(trifluoroacetic acid) 3-({4-[7-(aminocarbonyl)-2H-indazole-2-yl]benzyl}ammonio)-1-benzylpiperidinium;
5- {4-[7-(aminocarbonyl)-2H-indazole-2-yl]benzyl} -5-aza-2-azonium heterobicyclo[2.2.2]octane trifluoroacetate;
(1S,4S)-2-{4-[7-(aminocarbonyl)-2H-indazole-2-yl]benzyl}-5-methyl-2,5-diazonium heterobicyclo[22.1]heptane bis(trifluoroacetate); bis(trifluoroacetic acid) 1-{4-[7-(aminocarbonyl)-2H-indazole-2-yl]benzyl}-4-(pyridin-2-ylmethylbase)dinium piperazine; 5- {4-[7-(aminocarbonyl)-2H-indazole-2-yl]benzyl} -2-benzyl-5-aza-2-azonium heterobicyclo[2.2. 2] octane trifluoroacetate;
8-{4-[7-(aminocarbonyl)-2H-indazole-2-yl]benzyl}-3-benzyl-8-aza-3-azonium heterobicyclo[3.2.1]octane trifluoroacetate;
(1S,4S)-5-{4-[7-(aminocarbonyl)-2H-indazole-2-yl]benzyl}-2-benzyl-5-aza-2-azonium heterobicyclo[2.2.1]heptane trifluoroacetate; bis(trifluoroacetic acid)3-({4-[7-(aminocarbonyl)-2H-indazole-2-yl]benzyl} ammonio)pyrrolidinium;
6-({4-[7-(aminocarbonyl)-2H-indazole-2-yl]benzyl}ammonio)-3-diazonium heterobicyclo[3.1.0]hexanebis(trifluoroacetate); trifluoroacetate (3S,4S)-N-{4-[7-(aminocarbonyl)-2H-indazole-2-yl]benzyl}-4-hydroxytetrahydrothiophene-3-ammonium 1,1-dioxide; bis(trifluoroacetic acid) 4-[({4-[7-(aminocarbonyl)-2H-indazole-2-yl] benzyl}ammonio)methyl]-4-hydroxy-1-methylpiperidinium;
N-{4-[7-(aminocarbonyl)-2H-indazole-2-yl]benzyl}-1-cyclopropyl-2-hydroxyethane ammonium trifluoroacetate;
{4-[7-(aminocarbonyl)-2H-indazole-2-yl]phenyl}-N-{[1-(hydroxymethyl)cyclopentyl]methyl} methane trifluoroacetate; bis(trifluoroacetic acid) 2-{4-[7-(aminocarbonyl)-2H-indazole-2-yl]benzyl}-1,2,3,4-tetrahydro-2,7-diazanaphthalene;
bis(trifluoroacetic acid) 1-{4-[7-(aminocarbonyl)-2H-indazole-2-yl]benzyl} -3-[(dimethylammonio)methyl]piperidiniu m; bis(trifluoroacetic acid)4-(1-{4-[7-(aminocarbonyl)-2H-indazole-2-yl]benzyl}piperidinium-4-yl)thiomorpholine-4-onium; trifluoroacetate 1-{4-[7-(aminocarbonyl)-2H-indazole-2-yl]benzyl} -4-[(methylsulfonyl)amino]piperidinium;
bis(trifluoroacetic acid)1-{4-[7-(aminocarbonyl)-2H-indazole-2-yl]benzyl}-4-(1H-imidazol-3 -onium-1 -ylmethyl) piperidinium; 7-{4-[7-(aminocarbonyl)-2H-indazole-2-yl]benzyl}-1-oxa-7-aziumhespiro [4.5] decane trifluoroacetate; trifluoroacetate 1-{4-[7-(aminocarbonyl)-2H-indazole-2-yl]benzyl}-4-(1 -hydroxy-1 -methylethyl) piperidinium; trifluoroacetate 2-[({4-[7-(aminocarbonyl)-2H-indazole-2-yl]phenyl}amino)carbonyl]-1-benzylpiperidinium;
trifluoroacetate 2-[({4-[7-(aminocarbonyl)-2H-indazole-2-yl]phenyl}amino)carbonyl]-1-ethylpiperidinium;
trifluoroacetate 3-[({4-[7-(aminocarbonyl)-2H-indazole-2-yl]phenyl}amino)carbonyl]-1-ethylpiperidinium;
2-[3-(1,4-diazabicycloheptane-1-ylcarbonyl)-4-fluorophenyl]-2H-indazole-7-carboxamide trifluoroacetate; {4-[7-(aminocarbonyl)-4-chloro-2H-indazole-2-yl]benzyl}methylcarbamate tert-butyl ester; trifluoroacetate 6-[7-(aminocarbonyl)-2H-indazole-2-yl]-1,2,3,4-tetrahydroisoquinolinium; trifluoroacetate 2-{4-[7-(aminocarbonyl)-2H-indazole-2-yl]phenyl}pyrrolidinium;
6-fluoro-2- {4-[(methylamino)methyl]phenyl} -2H-indazole-7-carboxamide;
5-fluoro-2-{2-fluoro-4-[(methylamino)methyl]phenyl}-2H-indazole-7-carboxamide;
2- {3-hydroxy-4-[(methylamino)methyl]phenyl} -2H-indazole-7-carboxamide trifluoroacetate; 2-(4-{[formyl(methyl)amino]methyl}-3-hydroxyphenyl)-2H-indazole-7-carboxamide;
2- {2-chloro-4-[(methylamino)methyl]benzene yl} -5-fluoro-2H-indazole-7-carboxamide;
5-fluoro-2- {3-fluoro-4-[(methylamino)methyl]phenyl} -2H-indazole-7- carboxamide trifluoroacetate;
2-{2,5-difluoro-4-[(methylamino)methyl]phenyl}-5-fluoro-2H-indazole-7-carboxamide trifluoroacetate; 2-(4-bromophenyl)-2H-indazole-7-carboxamide; chloride (3R)-3-[({4-[7-(aminocarbonyl)-5-fluoro-2H-indazole-2-yl]phenyl}amino)carbonyl]-1-methyl piperidinium; trifluoroacetate (3R)-3-[({4-[7-(aminocarbonyl)-5-fluoro-2H-indazole-2-yl]phenyl}amino)carbonyl]-1-methyl piperidinium; 2-(1,2,3,4-tetrahydroisoquinolin-7-yl)-2H-indazole-7-carboxamide;
(R)-2-[4-({3-[(dimethylamino)methyl]piperidin-1-yl}methyl)phenyl]-2H-indazole-7-carboxam ide;
(S)-2-[4-({3-[(dimethylamino)methyl]piperidin-1-yl}methyl)phenyl]-2H-indazole-7-carboxami de;
3-({4-[7-(aminocarbonyl)-5-fluoro-2H-indazole-2-yl]phenyl}amino)-2-(chloromethyl)-3-oxopr opane-1-ammonium trifluoroacetate; 5-fluoro-2- {3-fluoro-4-[(methylamino)methyl]phenyl} -2H-indazole-7-carboxamide hydrochloride;
2-{4-[(dimethylamino)methyl]-3-fluorophenyl}-5-fluoro-2H-indazole-7-carboxamide trifluoroacetate;
2-{4-[(azacyclobutane-3-ylcarbonyl)amino]phenyl}-5-fluoro-2H-indazole-7-carboxamide;
2-[4-(2,7-diazaspiro[4.5]dec-2-ylmethyl)phenyl]-2H-indazole-7-carboxamide;
(1S,4S)-5-{4-[7-(aminocarbonyl)-2H-indazole-2-yl]benzyl}-2-(4-chlorobenzyl)-5-aza-2-azoniu m heterobicyclo[2.2.1]heptane trifluoroacetate;
(1S,4S)-5-{4-[7-(aminocarbonyl)-2H-indazole-2-yl]benzyl}-2-(3-chlorobenzyl)-5-aza-2-azonium heterobicyclo[2.2.1]heptane trifluoroacetate; trifluoroacetate 1-{4-[7-(aminocarbonyl)-2H-indazole-2-yl]benzyl}-4-[(methylamino)carbonyl]piperazine-1-o nium; N- {4-[7-(aminocarbonyl)-2H-indazole-2-yl]benzyl}-2-hydroxy-2-pyridin-3-ylethane ammonium trifluoroacetate;
N- {4-[7-(aminocarbonyl)-2H-indazole-2-yl]benzyl} -2-cyclohexyl-2-hydroxyethane ammonium trifluoroacetate; 4- {4-[7-(aminocarbonyl)-2H-indazole-2-yl]benzyl} -6-(hydroxymethyl)-trifluoroacetate 1,4-oxaazacycloheptane-4-onium;
{4-[7-(aminocarbonyl)-2H-indazole-2-yl]phenyl}-N-{[1-(hydroxymethyl) cyclobutyl]methyl}methane ammonium trifluoroacetate;
{4-[7-(aminocarbonyl)-2H-indazole-2-yl]phenyl}-N-{[1-(hydroxymethyl)cyclohexyl]methyl} methane ammonium trifluoroacetate; trifluoroacetate 1-{4-[7-(aminocarbonyl)-2H-indazole-2-yl]benzyl}-4-(5-methyl-1H-benzimidazol-2-yl)piperid inium; bis(trifluoroacetic acid)2-(1- {4-[7-(aminocarbonyl)-2H-indazole-2-yl]benzyl} -4-hydroxy piperidinium-4-yl)pyridinium; trifluoroacetate 1-{4-[7-(aminocarbonyl)-2H-indazole-2-yl]benzyl} -3,3-difluoropyrrolidinium;
2-(4-{[(2R)-2-(fluoromethyl)pyrrolidin-1-yl]methyl}phenyl)-2H-indazole-7-carboxamide;
N-{4-[7-(aminocarbonyl)-2H-indazole-2-yl]benzyl}-2-oxopyrrolidine-3-ammonium trifluoroacetate; 5-fluoro-2-(4-formylphenyl)-2H-indazole-7-carboxamide; trifluoroacetate 3-[({4-[7-(aminocarbonyl)-5-fluoro-2H-indazole-2-yl]phenyl}amino)carbonyl]-1-methyl azacyclobutanium; bis(trifluoroacetic acid)1-{4-[7-(ammocarbonyl)-5-fluoro-2H-indazole-2-yl]benzyl}-3-[(dimethylamino)methyl]p iperidinium; trifluoroacetate 3-[({4-[7-(aminocarbonyl)-5-fluoro-2H-indazole-2-yl]-2-fluorophenyl}amino)carbonyl]azacyc lobutanium; 2- {4-[7-(aminocarbonyl)-5-fluoro-2H-indazole-2-yl]benzyl} -2,7-diazonium heterospiro[4.5] decanebis(trifluoroacetate);
4,5-difluoro-2-{4-[(methylamino)methyl]phenyl}-2H-indazole-7-carboxamide trifluoroacetate; 5-fluoro-2-(3-fluoro-4-{[(1-methylazetidine-3-yl)carbonyl]amino}phenyl)-2H-indazole-7-carb oxamide trifluoroacetate; 5-fluoro-2-(3-fluoro-4-formylphenyl)-2H-indazole-7-carboxamide; 5-fluoro-2-(5-fluoro-2-formylphenyl)-2H-indazole-7-carboxamide;
{4-[7-(aminocarbonyl)-5-fluoro-2H-indazole-2-yl]-2-fluorophenyl}-N-{[1-(hydroxymethyl)cy clopentyl]methyl} ammonium trifluoroacetate;
5-fluoro-2-[3-fluoro-4-({[(3R)-1-methylpiperidin-3-yl]carbonyl}amino)phenyl]-2H-indazole-7 -carboxamide trifluoroacetate; bis(trifluoroacetic acid)1-{4-[7-(aminocarbonyl)-5-fluoro-2H-indazole -2-yl]-2-fluorobenzyl} -4-methylpiperazine dinium; bis(trifluoroacetic acid) 4-[({4-[7-(aminocarbonyl)-5-fluoro-2H-indazole-2-yl]benzyl}ammonio)methyl]-1-methylpiper idinium; bis(trifluoroacetic acid)4-[({4-[7-(aminocarbonyl)-5-fluoro-2H-indazole-2-yl]-2-fluorobenzyl}ammonio)methyl]-1-methylpiperidinium;
And pharmaceutically acceptable salts or tautomers thereof.

Other particular compounds within the scope of the present invention are:
Trifluoroacetate 7-[7-(aminocarbonyl)-2H-indazole-2-yl]-1-methyl-1,2,3,4-tetrahydroisoquinolinium;
trifluoroacetate 3- {4-[7-(aminocarbonyl)-5-fluoro-2H-indazole-2-yl]phenyl} -1 -ethylpiperidinium;
2-(4-cyanophenyl)-5-fluoro-2H-indazole-7-carboxamide;
5-fluoro-2-[4-(1H-tetrazol-5-yl)phenyl]-2H-indazole-7-carboxamide;
2-(4-aminophenyl)-5-fluoro-2H-indazole-7-carboxamide hydrochloride;
3-[7-(aminocarbonyl)-5-fluoro-2H-indazole-2-yl]pyrrolidine-1-carboxylate tert-butyl ester;
trifluoroacetate 3-[7-(aminocarbonyl)-5-fluoro-2H-indazole-2-yl]pyrrolidinium;
trifluoroacetate 3-[7-(aminocarbonyl)-5-fluoro-2H-indazole-2-yl]-1-methylpyrrolidinium;
trifluoroacetate 3-[7-(aminocarbonyl)-5-fluoro-2H-indazole-2-yl]-1-ethylpyrrolidinium;
trifluoroacetate 3-[7-(aminocarbonyl)-5-fluoro-2H-indazole-2-yl]-1-propylpyrrolidinium;
trifluoroacetate 3-[7-(aminocarbonyl)-5-fluoro-2H-indazole-2-yl]-1-isopropylpyrrolidinium;
trifluoroacetate 3-[7-(aminocarbonyl)-5-fluoro-2H-indazole-2-yl]-1-cyclohexylpyrrolidinium;
trifluoroacetate 3-[7-(aminocarbonyl)-5-fluoro-2H-indazole-2-yl]-1-cyclobutylpyrrolidinium;
4-[7-(aminocarbonyl)-2H-indazole-2-yl]-4-methylpiperidine-1-carboxylate tert-butyl ester;
trifluoroacetate 4-[7-(aminocarbonyl)-2H-indazole-2-yl]-4-methylpiperidinium;
trifluoroacetate 2-{4-[7-(aminocarbonyl)-5-fluoro-2H-indazole-2-yl]phenyl}pyrrolidinium;
2-[4-(4,5-dihydro-1H-imidazol-2-yl)phenyl]-5-fluoro-2H-indazole-7-carboxamide trifluoroacetate; chloride 6-[7-(aminocarbonyl)-5-fluoro-2H-indazole-2-yl]-1,2,3,4-tetrahydroisoquinolinium; chloride 2-{4-[7-(aminocarbonyl)-5-fluoro-2H-indazole-2-yl]phenyl}piperidinium;
5-fluoro-2-[4-(1H-pyrazol-1-yl)phenyl]-2H-indazole-7-carboxamide;
5-fluoro-2-(3-piperidin-3-ylphenyl)-2H-indazole-7-carboxamide;
2-[4-(aminosulfonyl)phenyl]-5-fluoro-2H-indazole-7-carboxamide;
5-fluoro-2-(5,6,7,8-tetrahydro-1,7-naphthalen-3-yl)-2H-indazole-7-carboxamide;
5-fluoro-2-(4-piperazin-2-ylphenyl)-2H-indazole-7-carboxamide;
4-[7-(aminocarbonyl)-5-fluoro-2H-indazole-2-yl]benzene methyl formate;
5-fluoro-2-(1-methylpiperidin-3-yl)-2H-indazole-7-carboxamide;
5-fluoro-2-(1-ethylpiperidin-3-yl)-2H-indazole-7-carboxamide;
5-fluoro-2-(1-propylpiperidin-3-yl)-2H-indazole-7-carboxamide;
5-fluoro-2-(1-isopropylpiperidin-3-yl)-2H-indazole-7-carboxamide;
2-(1-cyclohexylpiperidin-3-yl)-5-fluoro-2H-indazole-7-carboxamide;
5-fluoro-2-(1-methylpiperidin-4-yl)-2H-indazole-7-carboxamide;
5-fluoro-2-(1 -ethylpiperidin-4-yl)-2H-indazole-7-carboxamide;
5-fluoro-2-(1-propylpiperidin-4-yl)-2H-indazole-7-carboxamide;
5-fluoro-2-(1-isopropylpiperidine-4-yl)-2H-indazole-7-carboxamide;
2-(1-cyclohexylpiperidin-4-yl)-5-fluoro-2H-indazole-7-carboxamide;
2-(1-cyclobutylpiperidin-4-yl)-5-fluoro-2H-indazole-7-carboxamide;
2-(1-cyclobutylpiperidin-3-yl)-2H-indazole-7-carboxamide;
2-[7-(aminocarbonyl)-5-fluoro-2H-indazole-2-yl]-N,N-dimethylethane ammonium trifluoroacetate; 2-[7-(aminocarbonyl)-5-fluoro-2H-indazole-2-yl]-N,N-diethylethane ammonium trifluoroacetate; N-{2-[7-(aminocarbonyl)-5-fluoro-2H-indazole-2-yl]ethyl}propane-2-trifluoroacetate;
N-{2-[7-(aminocarbonyl)-5-fluoro-2H-indazole-2-yl]ethyl}cyclohexanetrifluoroacetic acid ammonium; 2-[2-(dicyclobutylamino)ethyl]-5-fluoro-2H-indazole-7-carboxamide;
3-[7-(aminocarbonyl)-5-fluoro-2H-indazole-2-yl]piperidine-1-carboxylate tert-butyl ester;
4-[7-(aminocarbonyl)-5-fluoro-2H-indazole-2-yl]piperidine-1-carboxylate tert-butyl ester;
trifluoroacetate 3 - [7-(aminocarbonyl)-5 -fluoro-2H-indazole-2-yl]piperidinium; trifluoroacetate 4-[7-(aminocarbonyl)-5-fluoro-2H-indazole-2-yl]piperidinium;
3-[7-(aminocarbonyl)-2H-indazole-2-yl]piperidine-1-carboxylate tert-butyl ester;
{2-[7-(aminocarbonyl)-5-fluoro-2H-indazole-2-yl]ethyl}carboxylate tert-butyl ester;
2-[7-(aminocarbonyl)-5-fluoro-2H-indazole-2-yl]ethane ammonium trifluoroacetate;
trifluoroacetate 3-[7-(aminocarbonyl)-2H-indazole-2-yl]piperidinium; trifluoroacetate 3-[7-(aminocarbonyl)-2H-indazole-2-yl]-1-methylpiperidinium; trifluoroacetate 3-[7-(aminocarbonyl)-2H-indazole-2-yl]-1-ethylpiperidinium; trifluoroacetate 3-[7-(aminocarbonyl)-2H-indazole-2-yl]-1-propylpiperidinium; trifluoroacetate 3-[7-(aminocarbonyl)-2H-indazole-2-yl]-1-isopropylpiperidinium; trifluoroacetate 3-[7-(aminocarbonyl)-2H-indazole-2-yl]-1-cyclohexylpiperidinium; trifluoroacetate 3-[7-(aminocarbonyl)-5-fluoro-2H-indazole-2-yl]-1-cyclobutylpiperidinium;
N-{2-[7-(aminocarbonyl)-5-fluoro-2H-indazole-2-yl]ethyl}-N-propylpropane-1-ammonium trifluoroacetate;
2-(4-piperidin-3-ylphenyl)-2H-indazole-7-carboxamide;
2-{4-[(3R)-piperidin-3-yl]phenyl}-2H-indazole-7-carboxamide;
2-{4-[(3S)-piperidin-3-yl]phenyl}-2H-indazole-7-carboxamide;
5-fluoro-2-(4-piperidin-3-ylphenyl)-2H-indazole-7-carboxamide;
5-fluoro-2-{4-[(3S)-piperidin-3-yl]phenyl}-2H-indazole-7-carboxamide;
5-fluoro-2-{4-[(3R)-piperidin-3-yl]phenyl}-2H-indazole-7-carboxamide;
5-fluoro-2-(3-fluoro-4-piperidin-3-ylphenyl)-2H-indazole-7-carboxamide;
5-fluoro-2-{3-fluoro-4-[(3R)-piperidin-3-yl]phenyl}-2H-indazole-7-carboxamide;
5-fluoro-2-(3-fluoro-4-[(3S)-piperidin-3-yl]phenyl}-2H-indazole-7-carboxamide;
2-{4-[(3R)-piperidin-3-yl]phenyl}-2H-indazole-7-carboxamide;
2-{4-[(3S)-piperidin-3-yl]phenyl}-2H-indazole-7-carboxamide;
In an embodiment, the compound shown in formula I is

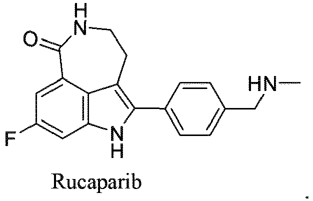

In an embodiment, the compound shown in formula II is

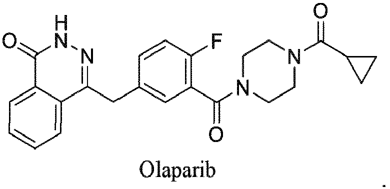

In an embodiment, the compound shown in formula III is

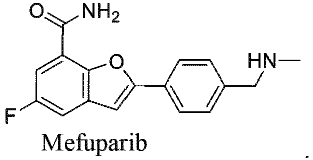

In an embodiment, the compound shown in formula IV is

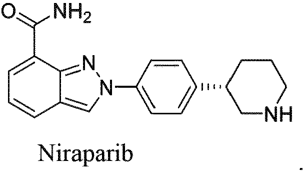

In an embodiment, the Substance A is selected from one or more of niraparib, tarazoparib, fluzoparib, simmiparib, IMP4297, BGB-290, ABT-888, rucaparib, olaparib and mefuparib;

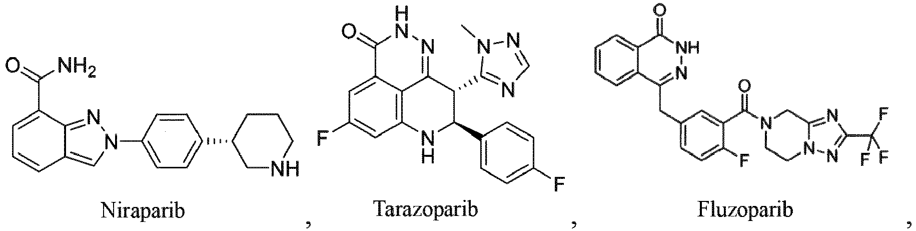

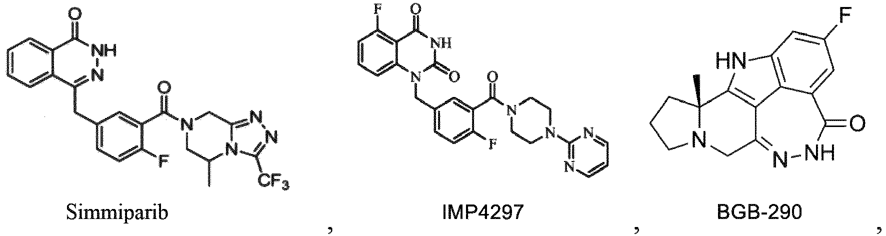

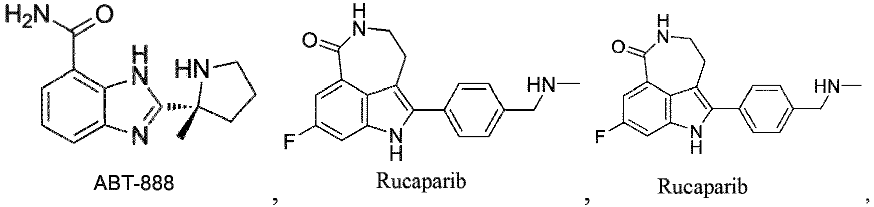

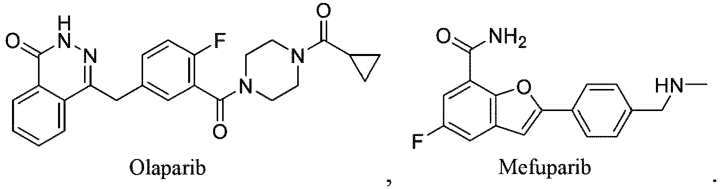

In an embodiment, the pharmaceutically acceptable salt is a hydrochloride salt.

In an embodiment, the pharmaceutically acceptable salt of substance A is

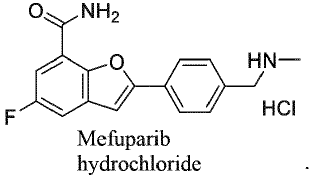

In an embodiment, the poly ADP ribose polymerase inhibitor or a pharmaceutically acceptable salt thereof is present in the form of a pharmaceutical composition comprising the same. Preferably, the pharmaceutical composition uses the poly ADP ribose polymerase inhibitor or a pharmaceutically acceptable salt thereof as the only active ingredient of the pharmaceutical composition; and/or, the pharmaceutical composition further comprises pharmaceutical acceptable carriers, such as pharmaceutically acceptable excipients.

In an embodiment, the poly ADP ribose polymerase inhibitor or a pharmaceutically acceptable salt thereof is present in the form of a kit composition comprising the same, and the kit also contains drugs to treat coronaviruses-related diseases and/or drugs to treat diseases caused by other viruses.

In order to solve the above technical problems, the second aspect of the present invention provides a compound represented by formula **III** and/or a compound represented by formula **IV** or a pharmaceutically acceptable salt thereof in the preparation of antiviral agents or in the preparation of medicines for treatment of diseases caused by viruses according to any one of claims 3-6, the viruses being HIV, HPV, EBV, IFV and/or coronaviruses, preferably the subfamily Orthocoronavirinae viruses.

In an embodiment, the pharmaceutically acceptable salt is a hydrochloride salt. In a preferred example of the present invention, it is preferred that the pharmaceutically acceptable salt is mefuparib hydrochloride.

In an embodiment, the compound represented by formula **III** and/or a compound represented by formula **IV** or a pharmaceutically acceptable salt thereof is present in the form of a pharmaceutical composition comprising the same; preferably, the pharmaceutical composition uses the compound represented by formula **III** and/or a compound represented by formula **IV** or a pharmaceutically acceptable salt thereof as the only active ingredient of the pharmaceutical composition; and/or, the pharmaceutical composition further comprises pharmaceutical acceptable carriers, such as pharmaceutically acceptable excipients.

In an embodiment, the compound represented by formula **III** and/or a compound represented by formula **IV** or a pharmaceutically acceptable salt thereof is present in the form of a kit composition comprising the same, and the kit also contains drugs for other anti-coronavirus-induced diseases.

In an embodiment, viruses of the subfamily Orthocoronavirinae are α coronavirus, β coronavirus, γ coronavirus and/or δ coronavirus, preferably coronaviruses that cause upper respiratory tract infections, and viruses that cause acute respiratory syndrome such as SARS-related coronavirus and/or Middle East respiratory syndrome coronavirus (MERS-CoV).

Preferably, the coronaviruses that cause upper respiratory tract infections are human coronavirus 229E, human coronavirus HKU1 (HCoV-HKU1), human coronavirus OC43 (HCoV-OC43), human coronavirus NL63 (HCoV-NL63) and/or mouse hepatitis virus A59 (MHV-A59).

Preferably, the SARS-related coronavirus is SARS-CoV (severe acute respiratory syndrome coronavirus) or SARS-CoV-2 (severe acute respiratory syndrome coronavirus-2).

In a preferred embodiment of the present invention, the coronaviruses include severe acute respiratory syndrome coronavirus (SARS-CoV) and severe acute respiratory syndrome coronavirus-2 (SARS-CoV-2).

In a preferred embodiment of the present invention, the coronavirus is severe acute respiratory syndrome coronavirus-2 (SARS-CoV-2).

In addition, in order to solve the above technical problems, the present invention also provides the application of a poly ADP ribose polymerase inhibitor or a pharmaceutically acceptable salt thereof in the preparation of a medicine for virus-related diseases.

Preferably, the poly ADP ribose polymerase inhibitor or a pharmaceutically acceptable salt thereof is as described in the first aspect of the present invention.

Preferably, the medicine for virus-related diseases is as described in the second aspect of the present invention.

To solve the above technical problems, the present invention also provides a medicine for treatment of virus-related diseases as described in the second aspect of the present invention, the medicine comprising the poly ADP ribose polymerase inhibitor or a pharmaceutically acceptable salt thereof as described in the first aspect of the present invention.

To solve the above technical problems, the present invention also provides a viral inhibitor, which comprises the poly ADP ribose polymerase inhibitor or a pharmaceutically acceptable salt thereof as described in the first aspect of the present invention. The viruses are those as described in the second aspect of the present invention

To solve the above technical problems, the present invention also provides use of a poly ADP ribose polymerase inhibitor or a pharmaceutically acceptable salt thereof as described in the first aspect of the present invention for the treatment of the virus-related diseases as described in the second aspect of the present invention.

The term "pharmaceutically acceptable" refers to salts, solvents, excipients and the like that are generally non-toxic, safe, and suitable for use in patients. The "patient" is preferably a mammal, more preferably a human.

The term "pharmaceutically acceptable salts" refers to salts obtained by preparing the compounds of the present invention with relatively non-toxic, pharmaceutically acceptable acids or bases. When compounds of the present invention contain relatively acidic functional groups, base addition salts can be obtained by contacting neutral forms of such compounds with a sufficient amount of a pharmaceutically acceptable base in pure solution or in a suitable inert solvent. Pharmaceutically acceptable base addition salts include, but are not limited to, lithium, sodium, potassium, calcium, aluminum, magnesium, zinc, bismuth, ammonium, diethanolamine salts. When compounds of the present invention contain relatively basic functional groups, acid addition salt can be obtained by contacting neutral forms of such compounds with a sufficient amount of a pharmaceutically acceptable acid in pure solution or in a suitable inert solvent. The pharmaceutically acceptable acids include inorganic acids, including but not limited to: hydrochloric acid, hydrobromic acid, hydroiodic acid, nitric acid, carbonic acid, phosphoric acid, phosphorous acid, sulfuric acid, and the like. The pharmaceutically acceptable acids include organic acids, including but not limited to: acetic acid, propionic acid, oxalic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, salicylic acid, tartaric acid, methanesulfonic acid, isonicotinic acid, acid citrate, oleic acid, tannic acid, pantothenic acid, hydrogen tartrate, ascorbic acid, gentisic acid, fumaric acid, gluconic acid, sugar acid, formic acid, ethanesulfonic acid, pamoic acid (i.e. 4,4'-methylene-bis(3-hydroxy-2-naphthoic acid), amino acids (for example, glutamic acid, arginine), and the like. When the compounds of the present invention contain relatively acidic and relatively basic functional groups, they can be converted into base addition salts or acid addition salts. For details, see Berge et al., "Pharmaceutical Salts", Journal of Pharmaceutical Science 66: 1-19 (1977) or Handbook of Pharmaceutical Salts: Properties, Selection, and Use (P. Heinrich Stahl and Camille G. Wermuth, ed., Wiley-VCH, 2002).

The term "solvate" refers to a substance formed by combining a compound of the present invention with a stoichiometric or non-stoichiometric amount of a solvent. Solvent molecules in solvates can exist in the form of ordered or non-ordered arrangement. The solvent includes, but is not limited to, water, methanol, ethanol, and the like.

"Pharmaceutically acceptable salts" and "solvates" in the term "solvates of pharmaceutically acceptable salts" are as described above and refer to the substances formed by combining the compounds of the present invention with 1, with 2 obtained by preparing a relatively non-toxic, pharmaceutically acceptable compound, and with a stoichiometric or non-stoichiometric solvent. The "solvates of pharmaceutically acceptable salts" include, but are not limited to, hydrochloric acid monohydrates of the compounds of the present invention.

The term "plurality" refers to 2, 3, 4 or 5.

When any variable (for example, R¹¹⁻¹) appears multiple times in the definition of a compound, the definition that appears at each position of the variable is independent of the definitions that appear at other positions, and their meanings are independent of each other and do not affect each other. Therefore, if a group is substituted by 1, 2 or 3 R¹¹⁻¹ groups, that is, the group may be substituted by up to 3 R¹¹⁻¹ groups, the definition of R¹¹⁻¹ at this position will be independent of the definition of R¹¹⁻¹ at the remaining positions. In addition, combinations of substituents and/or variables are allowed only if such combinations produce stable compounds.

The term "halogen" refers to fluorine, chlorine, bromine or iodine.

The term "hydrocarbyl" refers to a monovalent moiety obtained by removing a hydrogen atom from a carbon atom of a hydrocarbon compound having 1 to 20 carbon atoms (unless otherwise specified), which may be aliphatic or cycloaliphatic and may be saturated or unsaturated (e.g., partially unsaturated, fully unsaturated). Thus, the term "hydrocarbyl" includes the subclasses of alkyl, alkenyl, alkynyl, cycloalkyl, cycloalkenyl, cycloalkynyl, and the like.

The term "alkyl" refers to a straight or branched chain alkyl group having the specified number of carbon atoms. Examples of alkyl groups include methyl, ethyl, n-propyl, isopropyl, n-butyl, tert-butyl, isobutyl, sec-butyl, n-pentyl, n-hexyl, n-heptyl, n-octyl, and the similar alkyl groups thereof.

The term "alkenyl" refers to a straight or branched chain alkenyl group having the specified number of carbon atoms.

The term "alkynyl" refers to a straight or branched chain alkynyl group having the specified number of carbon atoms.

The term "alkoxy" refers to the group -O-R^{X}, wherein R^{X} is an alkyl group as defined above.

The term "cycloalkyl" refers to a monovalent saturated cyclic alkyl group, preferably a monovalent saturated cyclic alkyl group having 3-7 ring carbon atoms, more preferably 3-6 carbon atoms, such as cyclopropyl, cyclobutyl, cyclopentyl or cyclohexyl.

The term "heterocyclyl" or "heterocycle" refers to a monovalent moiety obtained by removing a hydrogen atom from a ring atom of a heterocyclic compound, the moiety having 3 to 20 ring atoms (unless otherwise specified), wherein 1 to 10 are ring heteroatoms and can be aromatic or non-aromatic. Preferably, each ring has 3 to 7 ring atoms, wherein 1 to 4 are ring heteroatoms.

The term "heterocycloalkyl" refers to a saturated monocyclic group having a heteroatom, preferably a 3-7 membered saturated monocyclic group containing 1, 2 or 3 ring heteroatoms independently selected from N, O and S. Examples of heterocycloalkyl groups are: pyrrolidinyl, tetrahydrofuranyl, tetrahydropyranyl, tetrahydrothienyl, tetrahydropyridyl, tetrahydropyrrolyl, azacyclobutanyl, thiazolidinyl, oxazolidinyl, piperidinyl, morpholinyl, thiomorpholinyl, piperazinyl, azepanyl, diazepanyl, oxazepanyl and the like. Preferred heterocyclyl groups are morpholin-4-yl, piperidin-1-yl, pyrrolidin-1-yl, thiomorpholin-4-yl and 1,1-dioxo-thiomorpholin-4-yl.

The term "heteroaryl" or "heteroaromatic ring" refers to an aromatic group comprising a heteroatom, preferably comprising 1, 2 or 3 aromatic 5-6 membered monocyclic or 9-10 membered bicyclic rings independently selected from nitrogen, oxygen and sulfur, for example, furanyl, pyridyl, pyridazinyl, pyrimidinyl, pyrazinyl, thienyl, isoxazolyl, oxazolyl, diazolyl, imidazolyl, pyrrolyl, pyrazolyl, triazolyl, tetrazolyl , thiazolyl, isothiazolyl, thiadiazolyl, benzimidazolyl, indolyl, indazolyl, benzothiazolyl, benziisothiazolyl, benzoxazolyl, benzisoxazolyl, quinolinyl, isoquinolyl, and the like.

The pharmaceutical compositions involved in the present invention using the compounds of the present invention as active ingredients can be prepared according to methods known in the art. The compounds of the present invention may be formulated in any dosage form suitable for use in humans or animals. The weight content of the compound of the present invention in the pharmaceutical composition thereof is usually 0.1-99.0%.

The pharmaceutically acceptable carrier can be a conventional carrier in the art, and the carrier can be any suitable physiologically or pharmaceutically acceptable excipients. The pharmaceutical excipients are conventional pharmaceutical excipients in the art, preferably including pharmaceutically acceptable vehicles, fillers or diluents and the like. More preferably, the pharmaceutical composition comprises 0.01-99.99% of the above-mentioned protein and/or the above-mentioned antibody-drug conjugate, and 0.01-99.99% of the pharmaceutical carrier, and the percentage being mass percentage of the pharmaceutical composition.

The compound of the present invention or the pharmaceutical composition containing it may be administered in the form of unit dose, and in the route which may be enteral or parenteral, for example, oral, intravenous, intramuscular, subcutaneous, nasal, oral mucosal, intraocular, pulmonary and respiratory, skin, vaginal, rectal administration and the like.

The dosage form for administration may be a liquid dosage form, a solid dosage form or a semi-solid dosage form. Liquid dosage forms can be solutions (including both true and colloidal solutions), emulsions (including o/w, w/o and multiple emulsion), suspension, injections (including aqueous, powdered and infusion), eye drops, nasal drops, lotion, liniment and the like; the solid dosage forms can be tablets (including ordinary tablets, enteric-coated tablets, lozenges, dispersible tablets, chewable tablets, effervescent tablets, orally disintegrating tablets), capsules (including hard capsules, soft capsules, enteric-coated capsules), granules, powders, pellets, dripping pills, suppositories, films, patches, gas (powder) aerosols, sprays and the like; semi-solid dosage forms can be ointments, gels, pastes and the like.

The compound of the present invention can be prepared into ordinary preparations, and also sustained-release preparations, controlled-release preparations, targeted preparations and various microparticle delivery systems.

On the basis of conforming to common knowledge in the art, the above preferred conditions can be combined arbitrarily to obtain the better examples of the present invention.

The reagents and raw materials used in the present invention are all commercially available.

The effect of the present invention terms of positive progress is: the present invention has been confirmed for the first time that the poly ADP ribose polymerase inhibitor can inhibit the infection of host cells by coronaviruses and the replication of the coronaviruses, and the effect is dose-dependent with no significant cytopathic action, and that it can be used for treatment of diseases related to anti-coronavirus infections. The poly ADP ribose polymerase inhibitor of the present invention or a pharmaceutically acceptable salt thereof, as well as a pharmaceutical composition and a kit containing the same, can achieve a lower effective concentration for inhibiting viruses and a higher antiviral activity while ensuring low toxicity and high safety when being used in humans, so that, when used in the clinical treatment of diseases caused by coronaviruses, the present inhibitor can effectively inhibit the viruses. In a preferred example of the present invention, the inhibition rate of the poly ADP ribose polymerase inhibitor against the viruses can be up to 35% (under the same conditions, the inhibition rate of the arbidol against the viruses is only 21%).

### Brief Description of the Drawings

Fig. 1 is a graph showing the results of inhibitory activity of mefuparib hydrochloride (CVL218) and Olaparib against SARS-CoV-2.
Fig. 2 is a graph showing the antiviral activity and cellular activity of mefuparib hydrochloride (CVL218) on SARS-CoV-2, wherein A and B are experiments of different batches, respectively.
Fig. 3 is a graph showing the cytotoxicity results of Olaparib in Vero-E6 cells.

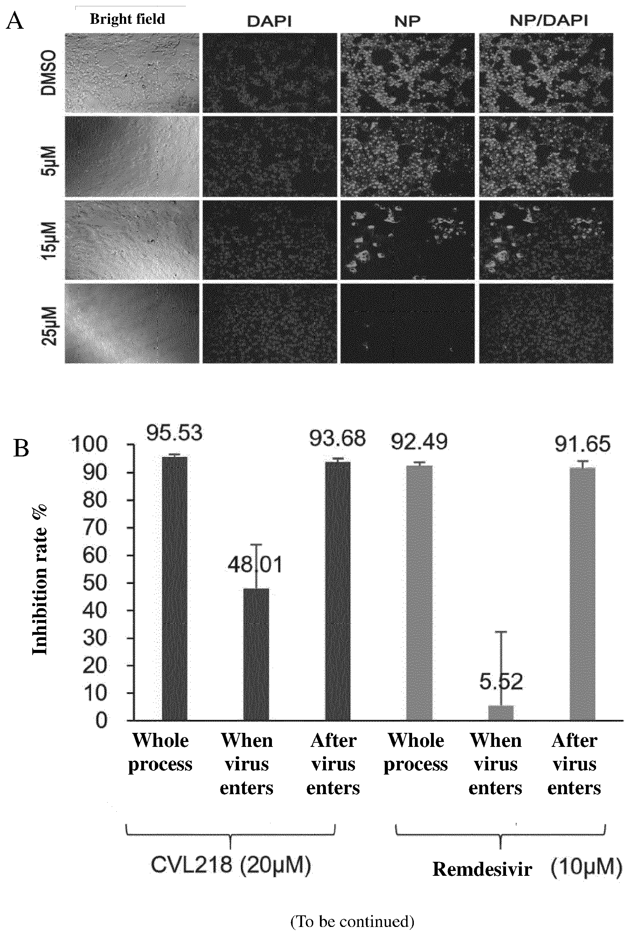

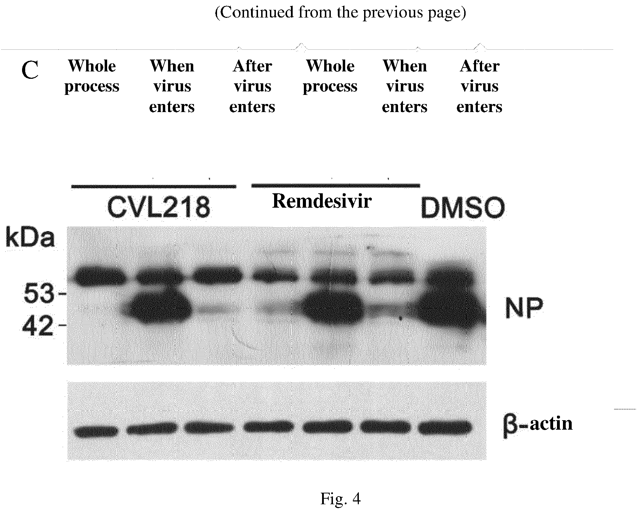

Fig. 4 shows the *in vitro* anti-SARS-CoV-2 activity of the test drug. (A) Viral nucleoprotein (NP) expression in Vero cells treated with CVL218 at 14 hours after SARS-CoV-2 infection was observed using a fluorescence microscope (NP represents nuclear protein staining, DAPI represents nuclear DNA staining, wherein DAPI is the dye 4',6-diamidino-2-phenylindole). (B) Relationship between the inhibitory effects of CVL218 and remdesivir on SARS-CoV-2 *in vitro* and the different duration of action. The viral inhibitory activities of CVL218 and remdesivir were determined at the stages of "whole process", "when virus enters" and "after virus enters", respectively. (C) Western blot analysis of viral NP expression in infected cells treated with CVL218 and remdesivir.
Fig. 5 shows that CVL218 attenuates CpG-induced IL-6 production in a time- and dose-dependent manner.
Fig. 6 shows the effect of CVL218 on body weight in rats (A) and monkeys (B). Rats and monkeys were orally administered CVL218 at 20/60/160 mg/kg and 5/20/80 mg/kg, respectively, for 28 days and then discontinued with drugs for 28 days, showing that CVL218 had good safety.
Fig. 7 shows the model structure of the action of the nucleocapsid protein N-terminus (N-NTD) of SARS-CoV-2 in complex with a PARP1 inhibitor. (A). Simulated structure of SARS-CoV-2-N-NTD in complex with CVL218 and olaparib (both modeled by AutoDock4.2). (B). Mode of interaction between viral N-NTD and PARP1 inhibitors CVL218 and Olaparib. Key residues are shown as sticks. Hydrogen bonds are represented as dashed lines.
Fig. 8 shows the tissue distribution characteristics of CVL218 in rats, with the maximum concentration in lungs. After oral administration of 20 mg/kg to rats, the concentrations of CVL218 in different tissues were determined at 3/6/8 h time points.

### Specific Embodiments

The present invention will be further described in the following examples, nevertheless, without being limited thereto. The experimental methods, specific conditions of which are not indicated in the following examples, are usually performed in accordance with conventional conditions, for example, described in common reference books in the art, such as Molecular Cloning: A Laboratory Manual (Third Edition, Science Publishing House, 2005), or according to the conditions suggested by the reagent manufacturer.
(1) Main instruments (name, number) are shown in Table 1 below.

**Table 1**

| Instrument name | Manufacturer | Instrument number |
|---|---|---|
| Quantitative PCR Instrument QuantStudio Dx | ABI | 04-620 |
| MagNA Pure LC2.0 Automatic Nucleic Acid Extractor | Roche | - |
| Carbon Dioxide Incubator (GALAXYS) | RsBiotech | 04-0303 |
| Inverted Microscope | NiKon | 0393 |

(2) Experimental reagents and virus strains
1) Reagents are shown in Table 2 below.

**Table 2**

| Name | Manufacturer | Item number | Expiry date |
|---|---|---|---|
| DMSO | Beyotime Biotechnology | ST-1276-500ml | - |
| DMEM Medium | Gibco | 12430-054 | 2020-10-30 |
| CCK-8 Kit | Beyotime Biotechnology | C0039 | - |
| Nucleic Acid Extraction Kit | Roche | 03038505001 | 2021-06 |
| 2019 Novel Coronavirus Nucleic Acid Test Kit | Shanghai Bio-germ | SJ-HX-226-2 | 2021-01-16 |

2) SARS-CoV-2 strain
① Obtained from the BSL-3 Laboratory of the Jiangsu Provincial Center for Disease Control and Prevention. The internal number of this strain is: 2019-nCoV-1.
② Obtained from Wuhan Institute of Virology, virus strain CCTCC number: IVCAS 6.7512, preservation unit: Wuhan Institute of Virology, Chinese Academy of Sciences, National Virus Resource Bank of China.
As identified, the genomes of the above two virus strains are completely identical, so they are both SARS-CoV-2 virus strains. The virus strains used in subsequent experiments are mainly 2019-nCoV-1.
(3) Drugs
Zanamivir, oseltamivir, remdesivir, baricitinib, olaparib and arbidol were all provided by MCE (Medchem Express, China). The PARP1 inhibitor mefuparib hydrochloride (CVL218, see also patent application 201210028895.0) has a purity of over 99.0%, which was provided by Pukang (Shanghai) Health Technology Co., Ltd.

### Example 1. Detection of in vitro anti-SARS-CoV-2 activity of mefuparib hydrochloride (CVL218)

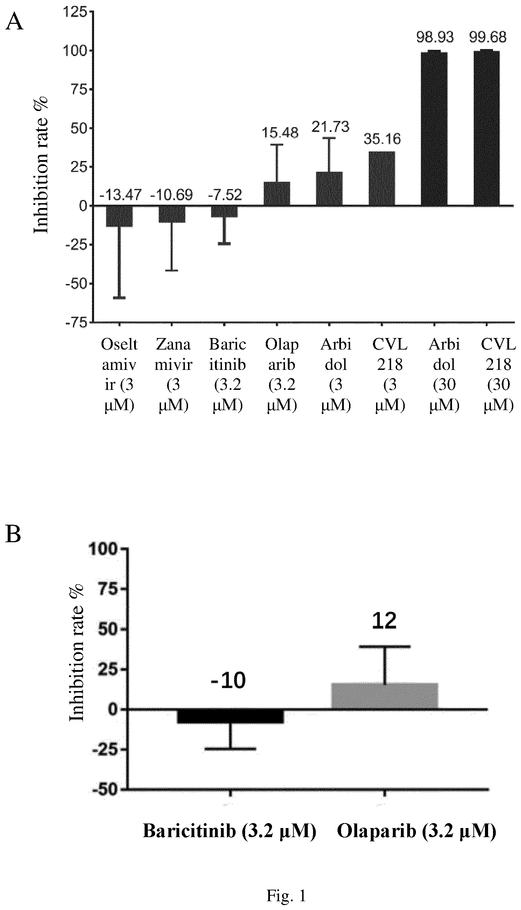

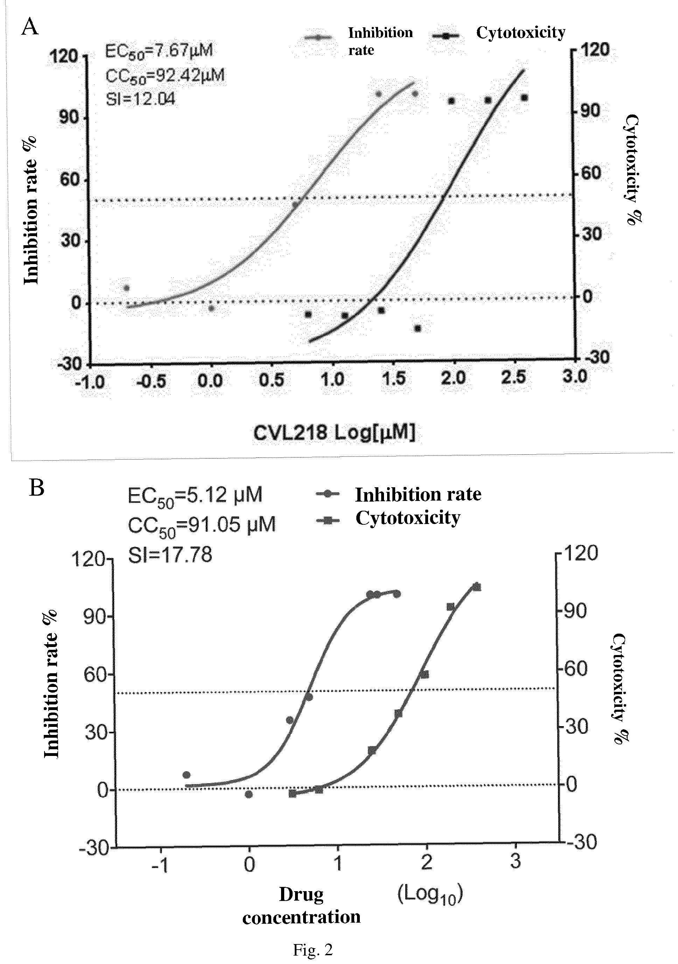

1.1 Experimental groups:
   1) Arbidol groups: 3 µM group and 30 µM group
   2) Mefuparib hydrochloride (CVL218) 3 µM group
   3) Mefuparib hydrochloride (CVL218) 30 µM group
   4) Zanamivir group
   5) Oseltamivir group
   6) Olaparib 3.2 µM group
   7) Baricitinib 3.2 µM group
   8) DMSO control group
   9) Virus control group: only virus added, no drug added
   10) Cell control group: containing only cells, without adding viruses and drugs
1.2 Drug dilution:
   15 µl of 1 mM stock solution was taken for each drug group, to which was added 4985 µl of cell maintenance solution (i.e., DMEM medium) to obtain 3 µM application solution. 3 µl of 20 mM stock solution of CVL218 was taken, to which was added 1997 µl of cell maintenance solution to obtain 30 µM application solution. DMSO was diluted according to the dilution ratio of the corresponding drug.
1.3 Experimental steps:
   1.3.1 Cell preparation and drug pretreatment (cell culture room)
      Vero-E6 cells (purchased from ATCC cell bank) were seeded into 96-well culture plates at 1 × 10⁴ cells/well, and cultured in DMEM containing 10% (v/v) fetal bovine serum for 16 hours to become 80% pellets, then the cell culture medium was aspirated and discarded from each well, the cells were washed once with sterile PBS, and different drugs (50 µl/well) diluted in the cell maintenance solution as described in section 1.2 above were added to each well according to the experimental groups, with 4 duplicate wells set up for each group, and then they were placed in a 37 °C, 5% CO₂ incubator for 1 h pretreatment. Only 50 µl of the cell maintenance solution was added to the virus control group and the cell control group.
   1.3.2 Virus infection and culture (BSL-3 Laboratory):
      After drug pretreatment for 1 h, except for the cell control group, 2 µl of the SARS-CoV-2 strain was added to each well to bring the virus multiplicity of infection to 0.05 (MOI = 0.05), the wells were placed in a 37°C, 5% CO₂ incubator for adsorption for 2 h. After the adsorption was completed, the medium with the viruses was discarded, and a new drug-containing medium was added according to the experimental group, and then cultured in a 37°C, 5% CO₂ incubator for 48 h. After culture, the cytopathic conditions were microscopically observed for each experimental group and recorded. 120 µl of the culture supernatant was pipetted from each well, placed at 56°C, and inactivated for 30 min. After the inactivation was completed, 100 µl was taken from each well and added to the lysate in the reagent tank of the nucleic acid extraction reagent. After the outer surface of the reagent tank was disinfected, the tank was transferred to the BSL-2 Laboratory for viral nucleic acid extraction and genetic testing.
   1.3.3 Nucleic acid extraction:
      Refer to the operating instructions of the automatic nucleic acid extractor and extraction kit for viral nucleic acid RNA extraction.
   1.3.4 Fluorescence quantitative PCR detection
      The PCR reaction system was configured according to the package insert of the Shanghai Bio-germ 2019 Novel Coronavirus Nucleic Acid Detection Kit. The specific reaction systems are: 6 µl of qRT-PCR reaction solution, 2 µl of qRT-PCR enzyme mixture, 2 µl of primer probe, and 2.5 µl viral nucleic acid RNA template extracted as described above; the reaction parameters are: 50°C for 10 min, 95°C for 5 min, 40 cycles of: 95°C for 10s, 55°C for 40s (at this step, fluorescence signals of the FAM channel and VIC channel were collected); viral replication levels were reflected by detecting the SARS-CoV-2 virus genes (ORF1ab and N) transcript levels. The 2^{-△CT} value was calculated according to the CT value given by the PCR instrument to represent the relative virus content of the experimental group relative to the control group. The virus replication inhibition rate (%) = (1-2^{-△CT}) × 100%.
1.4 Experimental results
   Compounds such as mefuparib hydrochloride (CVL218) and olaparib and their inhibitory effects on the SARS-CoV-2 coronavirus are shown in Fig. 1, Fig. 2 and Table 3. No inhibitory activity was detected in each control group. It can be seen from Fig. 1 and Table 3, CVL218 can effectively inhibit the replication of SARS-CoV-2 in Vero-E6 cells. CVL218 has an inhibition rate of 35% against the virus when it is at a concentration of 3 µM, which is higher than that of the control drug Arbidol (21%), while the anti-influenza virus drugs Zanamivir and Oseltamivir have no inhibitory activity against the SARS-CoV-2 virus. When CVL218 had a concentration of 30 µM, it had an inhibition rate of more than 99% against the virus. Fig. 2 shows that the EC₅₀ of CVL218 against SARS-CoV-2 was 7.67 µM and 5.12 µM in different batches (experimental steps are shown in Example 2), in a dose-dependent manner. Fig. 1 and Table 3 also show that the PARP-1 inhibitor Olaparib also has slight activity against SARS-CoV-2, with an inhibition rate of around 12% or 15.8% against the virus at 3.2 µM, while JAK-1 inhibitor Baricitinib shows almost 0% inhibition of the virus.

In conclusion, both PARP 1 inhibitors olaparib and CVL218 have exhibited inhibitory effects on SARS-CoV-2 replication; and CVL218 has a higher inhibition rate than olaparib. It should be noted that the antiviral efficacy of CVL218 exceeds that of Arbidol which is one of the standard treatments for COVID-19 in the *Diagnosis and Treatment Protocol for Novel Coronavirus Pneumonia (6th Edition)* promulgated by Chinese government.

**Table 3**

| Drug | Arbidol 3 µM group | CVL218 3 µM group | Zanamivir 3 µM group | Oseltamivir 3 µM group | CVL218 30 µM group | Olaparib | Baricitinib | Arbidol 30 µM group |
|---|---|---|---|---|---|---|---|---|
| Inhibiting rate, % | 21.73 | 35.16 | No inhibitory activity (-10.69) | No inhibitory activity (-13.47) | 99.68 | 12% (in different batches, the inhibition rate for another batch being 15.48) | No inhibitory activity (-10%) (in different batches, the inhibition rate for another batch being -7.52) | 98.93 |

### Example 2: Cytotoxicity test of mefuparib hydrochloride (CVL218) on Vero-E6 cells

2.1 Experimental groups:
1) Mefuparib hydrochloride (CVL218) group
2) DMSO control group
3) Olaparib group
4) Cell control group: containing only cells, without adding drugs
5) Blank control group (only medium without cells)

2.2 Drug dilution:
Mefuparib hydrochloride was obtained from Pukang (Shanghai) Health Technology Co., Ltd., with a purity of > 99.0%. It was dissolved in DMSO and then diluted in gradients according to Table 4 below (the maintenance solution was DMEM medium). Serial dilutions were performed for DMSO according to the same dilution gradients.

**Table 4**

| Diluent | Combination | Concentration |
|---|---|---|
| Diluent 1 | 0.05ml of mefuparib hydrochloride stock solution | 400 µM |
| | (20 mM) + 2.45ml of maintenance solution | |
| Diluent 2 | 1 ml Diluent 1 +1 ml maintenance solution | 200 µM |
| Diluent 3 | 1 ml Diluent 2 +1 ml maintenance solution | 100 µM |
| Diluent 4 | 1 ml Diluent 3 +1 ml maintenance solution | 50 µM |
| Diluent 5 | 1 ml Diluent 4 +1 ml maintenance solution | 25 µM |
| Diluent 6 | 1 ml Diluent 5 +1 ml maintenance solution | 12.5 µM |
| Diluent 7 | 1 ml Diluent 6 +1 ml maintenance solution | 6.25 µM |
| Diluent 8 | 1 ml Diluent 7 +1 ml maintenance solution | 3.125 µM |

2.3 Experimental steps:
2.3.1 Cell preparation and dosing culture
   Vero-E6 cells were seeded into 96-well culture plates at 1 × 10⁴ cells/well, and cultured in DMEM containing 10% fetal bovine serum for 16 hours to become 80% pellets, then the cell culture medium was aspirated and discarded from each well, the cells were washed once with sterile PBS, and different drugs (200 µl/well) diluted in the cell maintenance solution were added to each well according to the experimental groups, with 3 duplicate wells set up for each group, and then the wells were placed in a 37°C, 5% CO₂ incubator for 48 h. For the DMSO control group, DMSO diluted with the cell maintenance solution to the corresponding concentration was added, and for the cell control group, 200 µl of the cell maintenance solution was added. For the blank control group, only cell maintenance solution without cells was added.
2.3.2 Detection of toxicity of mefuparib hydrochloride and Olaparib on Vero-E6
   The cell state was observed and recorded under an inverted microscope, and the toxicity of the drug to Vero-E6 cells was detected according to the operation instructions of the CCK-8 kit: 20 µl of CCK-8 solution was added to all experimental wells and control wells, and the cell culture plate was placed in a 37°C, 5% CO₂ incubator for 1 hour, and then its absorbance value at the wavelength of 450 nm was measured using a multifunctional microplate reader. Calculations were conducted according to the formula: cell activity inhibition rate (%) = [1 - (drug group-blank control group)/(cell control group - blank control group)] × 100%.

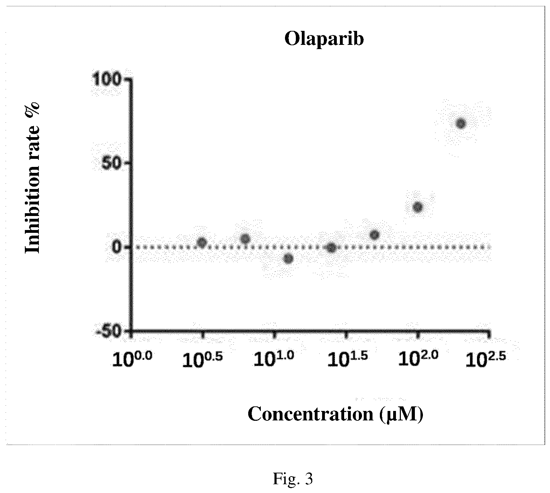

Graphs were plotted with the drug concentrations of mefuparib hydrochloride and Olaparib as the abscissa, and the cell proliferation inhibition rate as the ordinate. The results are shown as solid circles in Fig. 3. It can be seen from the figure, mefuparib hydrochloride has the CC₅₀ (50% cytotoxic concentration, the drug concentration at which 50% of the cells are killed) of about 92 µM in the Vero-E6 cells. Among them, mefuparib hydrochloride at 30 µM has no inhibitory effect on the cells, and basically has no toxicity. It can be seen that mefuparib hydrochloride (CVL218) has good safety. Olaparib has the CC50 (50% cytotoxic concentration, the drug concentration at which 50% of the cells are killed) of about 100-300 µM in the Vero-E6 cells.

### Example 3:

### 3.1 Indirect immunofluorescence assay

Vero E6 cells were treated with 5 µM, 15 µM and 25 µM of CVL218, respectively, following the "whole process" treatment procedure. Infected cells were fixed with 80% acetone in PBS, permeabilized with 0.5% Triton X-100, and then blocked with 5% BSA in PBS buffer containing 0.05% Tween 20 for 30 minutes at room temperature. Further, with SARS-CoV nucleocapsid protein rabbit polyclonal antibody (Cambridgebio, USA) as the primary antibody, the cells were incubated at a dilution of 1:200 for 2 hours, and then Alexa 488 labeled goat anti-rabbit antibody (Beyotime, China) was used as the secondary antibody for incubation at a dilution of 1:500. Nuclei were stained with DAPI (Beyotime, China). Immunofluorescence was observed with a fluorescence microscope.

Immunofluorescence microscopy showed that at 14 h after the SARS-CoV-2 infection, the expression of the viral nucleoprotein (NP) in the CVL218-treated cells had a dose-response relationship with the concentration of the treatment drug, and the expression in the CVL218-treated cells was significantly lower than that in the DSMO treated cells (Fig. 4A). No apparent cytopathic effect was observed in the infected cells treated with 25 µM of CVL218.

### 3.2 Study on duration of action

In order to systematically assess the inhibitory activity of compounds against SARS-CoV-2, we also performed a time-of-addition assay (method) to determine at which stage the compounds inhibited the viral infection. The duration of action was measured using relatively high doses of the test drugs (20 µM for CVL218 and 10 µM for remdesivir). Vero E6 cells at a density of 5 × 104 cells per well were treated with test drugs at different stages of the viral infection, or with DMSO as a control. The MOI was 0.05 for infecting cells with the virus. The "whole process" treatment was designed to evaluate the maximum antiviral effect, and the test drug in the cell culture medium was the same as described in the viral infection assay throughout the experiment. In the "when the virus enters" treatment, test drug was added to cells 1 hour prior to viral infection, then the cells were maintained in the drug-virus mixture for 2 hours during viral infection. Afterwards, the medium containing virus and test drug was replaced with fresh medium until the end of the experiment. In the "after the virus enters" experiment, the virus was first added to the cells and allowed to infect for 2 hours, and then the virus-containing supernatant was replaced with a drug-containing medium until the end of the experiment. At 14 hours after infection, the inhibitory effect of the drug on the virus in the cell supernatant was quantitatively detected by qRT-PCR and calculated with the DMSO group as the reference.

The results show that, compared with the DMSO control group, both CVL218 and remdesivir showed stronger antiviral activity during the whole process of SARS-CoV-2 infection of Vero E6 cells (Fig. 4B). The results of duration of action showed that CVL218 could partially antagonize viral entry and significantly inhibit the replication after viral entry, while remdesivir could only play a role in the post-viral entry stage (Fig. 4B and Fig. 4C). In conclusion, CVL218 may be a more advantageous potential drug for treatment of COVID-19.

### Example 4: CVL218 inhibits CpG-ODN 1826-induced IL-6 production in PBMCs

Interleukin-6 (IL-6) has recently been found to be one of the most important cytokines during viral infection (L. Velazquez-Salinas, A. Verdugo-Rodriguez, L. L. Rodriguez, M. V Borca, The role of interleukin 6 during viral infections, Frontiers in microbiology 10 (2019) 1057). The new human and animal clinical studies suggest that IL-6 oversynthesis is associated with the persistence of many viruses, such as human immunodeficiency virus (HIV) (M. M. McFarland-Mancini, H. M. Funk, A. M. Paluch, M. Zhou,P. V Giridhar, C. A. Mercer, S. C. Kozma, A. F. Drew, Differences in wound healing in mice with deficiency of IL-6 versus IL-6 receptor, The journal of immunology 184 (12) (2010) 7219-7228), foot-and-mouth disease virus (G. A. Palumbo, C. Scisciani, N. Pediconi, L. Lupacchini, D.Alfalate, F. Guerrieri, L. Calvo, D. Salerno, S. Di Cocco, M. Levrero,et al., IL6 inhibits HBV transcription by targeting the epigenetic control of the nuclear cccdna minichromosome, PLoS one 10 (11)) and vesicular stomatitis virus (VSV) (L. Velazquez-Salinas, S. J. Pauszek, C. Stenfeldt, E. S. OHearn, J. M. Pacheco, M. V Borca, A. Verdugo-Rodriguez, J. Arzt, L. L. Rodriguez, Increased virulence of an epidemic strain of vesicular stomatitis virus is associated with interference of the innate response in pigs, Frontiers in microbiology 9 (2018) 1891). Furthermore, *in vivo* studies using the Friend retrovirus (FV) mouse model have shown that IL-6 blockade can reduce viral load and enhance virus-specific CD8+ T cell immunity (W. Wu, K. K. Dietze, K. Gibbert, K. S. Lang, M. Trilling, H. Yan, J. Wu, D. Yang, M. Lu, M. Roggendorf, et al., TLR ligand induced IL-6 counter-regulates the anti-viral CD8+ T cell response during an acute retrovirus infection, Scientific reports 5 (2015) 10501). These findings support a hypothesis that the rapid production of IL-6 may be a possible mechanism leading to deleterious clinical manifestations in viral pathogenesis (J. Zheng, Y. Shi, L. Xiong, W. Zhang, Y Li, P. G. Gibson, J. L. Simpson, C. Zhang, J. Lu, J. Sai, et al., The expression of IL-6, tNF-α, and MCP-1 in respiratory viral infection in acute exacerbations of chronic obstructive pulmonary disease, Journal of immunology research 2017). A recently published study on the clinical characteristics of critically ill patients with SARS-CoV-2 infections has shown that IL-6 is significantly elevated, especially in those patients treated at ICU, causing an overactivated immune response (Y. Zhou, B. Fu, X. Zheng, D. Wang, C. Zhao, Y. Qi, R. Sun, Z. Tian, X. Xu, H. Wei, Aberrant pathogenic GM-CSF+ T cells and inflammatory CD14+ CD16+ monocytes in severe pulmonary syndrome patients of a new coronavirus, bioRxiv; J.-J. Zhang, X. Dong, Y.-Y. Cao, Y.-d. Yuan, Y-b. Yang, Y.-q. Yan, C. A. Akdis, Y.-d. Gao, Clinical characteristics of 140 patients infected by SARS-CoV-2 in Wuhan, China, Allergy; B. Diao, C. Wang, Y. Tan, X. Chen, Y Liu, L. Ning, L. Chen, M. Li, Y Liu, G. Wang, et al., Reduction and functional exhaustion of T cells in patients with coronavirus disease 2019 (COVID-19), medRxiv; X. C. Huang, Y Wang, X. Li, L. Ren, J. Zhao, Y. Hu, L. Zhang, G. Fan, J. Xu, Gu, et al., Clinical features of patients infected with 2019 novel coronavirus in Wuhan, China, The lancet 395 (10223) (2020) 497-506; Y. B. Li, F. Feng, G. Yang, A. Liu, N. Yang, Q. Jiang, H. Zhang, T. Wang, P. Li, Mao, et al., Immunoglobulin G/M and cytokines detections in continuous sera from patients with novel coronaviruses (2019-nCoV) infection, Available at SSRN 3543609). The pathological role of IL-6 in SARS-CoV-2 infection suggests that IL-6 blockade may provide a feasible therapy for treatment of COVID-19.

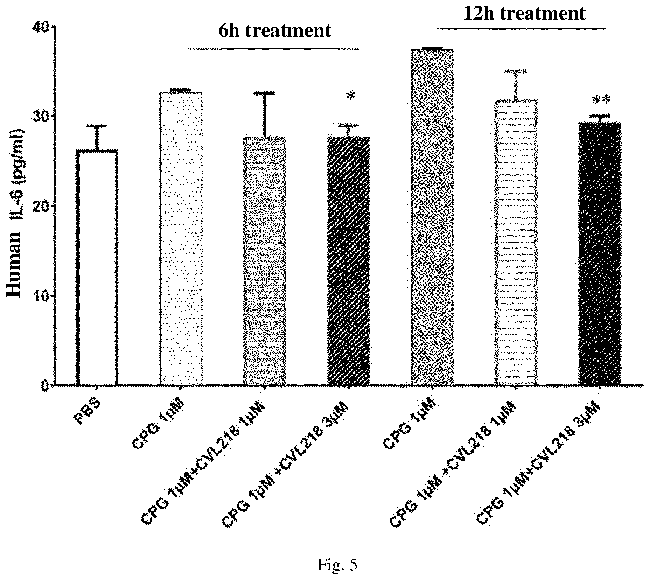

To test whether CVL218 can regulate IL-6 production *in vitro,* we stimulated IL-6 production by peripheral blood mononuclear cells (PMBCs) using CpG-ODN 1826, a potent stimulator of cytokines and chemokines. Incubation of PBMCs with 1 µM CpG-ODN 1826 for 6 hours (method) induced 40% IL-6 production compared to untreated cells (Fig.5). The stimulatory effect of CpG-ODN 1826 was counteracted in the presence of CVL218. Further studies showed that CVL218 inhibited CpG-induced upregulation of IL-6 in a time- and dose-dependent manner (Fig. 5). More specifically, exposure to CVL218 at 1 µM and 3 µM concentrations for 12 hours reduced CpG-induced IL-6 production by 50% and 72.65%, respectively. These results provide *in vitro* evidence for using CVL218 as a potential therapeutic agent for treatment of proinflammatory responses induced by SARS-CoV-2 infections.

Among them, the experimental steps of CpG-PDN1826-induced PBMCs to produce IL-6 are as follows: peripheral blood mononuclear cells (Beijing Yicon) were cultured in 96-well plates in RPMI1640 cell growth medium (Corning, Cat. 10-040-CVR) at 37°C in a 5% CO₂ atmosphere. For stimulation, PBMC cells were incubated with 1 µM CpG-ODN1826 (InvivoGen, Cat.tlrl-1826). To test whether CVL218 could inhibit IL-6 production, CVL218 was added to cell culture media at concentrations of 1 µM and 3 µM for 6 and 12 hours, respectively. Concentrations of IL-6 were determined by ELISA using a commercial kit (Dakewe Biotech, Cat. 1110602).

### Example 5: Metabolism and safety profile of CVL218 in animals

### (I) Experimental steps

### 5.1. Pharmacokinetic studies

Sprague-Dawley rats were purchased from Animal Experiment Center of Shanghai, China. The experimental animals were housed in groups in wire cages, with no more than 6 animals per cage. The experimental conditions were good (temperature 25 ± 2°C; relative humidity 50 ± 20%) and light-dark cycle (12 hours/12 hours). 144 Sprague-Dawley rats were randomized into 4 groups (18 animals/gender/group). CVL218 was administered at doses of 20, 40, 60 and 160 mg/kg. For all groups, 20 rats (10 animals/gender/group) were randomly selected, euthanized on day 28, and sections of various tissues and organs were obtained and frozen. Ten (5/gender/group) animals were euthanized after a 28-day drug-free period, and sections of their tissues and organs were taken and frozen. Six animals (3/gender/group) were euthanized after blood samples were collected. For pharmacokinetics and safety evaluations, the blood concentrations, clinical symptoms, mortality and body weight of animals were examined.

### 5.2. Tissue distribution study

30 Sprague-Dawley rats were randomized into 3 time-point groups (3 animals/gender/group). At 3, 6 and 8 hours after CVL218 administration, animals were sacrificed, and the brain, heart, lung, liver, spleen, stomach and kidney tissues were collected. The tissue samples were washed with ice-cold physiological saline and weighed after excess liquid was removed with paper towels. After tissue sample solutions were weighed, they were stored at -20 ± 2°C until the drug concentration was determined by LC-MS-MS.

### 5.3. Safety study in cynomolgus monkeys.

Healthy male and female monkeys, aged 3-4 years, were purchased from Landao Biotechnology, Guangdong, China. Animals were fed in accordance with the *Guide for Care and Use of Laboratory Animals.* The animals were randomized (5 animals/gender/group), and were fed with CVL218 via nasogastric at dose levels of 0 (control), 5, 20 and 80 mg/kg. Monkeys were observed twice daily for any changes in viability/mortality and behavior, response to treatment or health status.

### 5.4. Statistical analysis

All data represent the mean ± standard deviations (SDs) of n values, where n corresponds to the number of data points used. Numbers were compiled using GraphPad Prism (GraphPad Software, USA). Statistical significance was calculated using SPSS (ver.12), and two values were considered to be significantly different if *p*-value < 0.05.

### (II) Experimental results and conclusion

### 5.5. CVL218 most distributed in rat lung tissues

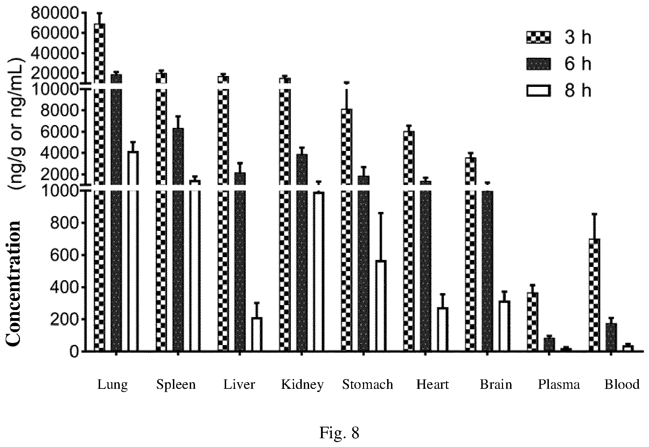

The concentrations of CVL218 in different tissues at different time points after oral administration of different doses in rats are shown in Fig. 8 and Table 5. Among 7 tissues (i.e., lung, spleen, liver, kidney, stomach, heart, brain), we observed the maximum CVL218 concentration in lung, which was 188-fold higher than that in plasma (Table 6). The maximum concentration of CVL218 was observed in lungs, which is consistent with the fact that the SARS-CoV-2 virus has the greatest pathological impact in the lungs and has a high viral load, suggesting that CVL218 has the potential to be used in the indication of lung diseases caused by SARS-CoV-2 infection.

In addition, the results have shown that the pharmacokinetic parameters of CVL218 and arbidol are comparable, with similar plasma concentrations and drug exposures (Table 7). After administration to rats, arbidol is mainly distributed in the stomach and plasma. In contrast, CVL218 has a higher distribution in tissues, especially in the lung rather than in the plasma, which is higher than that of arbidol, indicating that CVL218 has a good pharmacokinetic profile, which may make it a potential antiviral therapeutic drug for SARS-CoV-2 lung infection.

**Table 5 Comparison of tissue distribution in rats after oral administration of CVL218 and arbidol at 20 mg/kg and 54 mg/kg**

| Drug | Dose (mg/kg) | Time (min) | Lung | Spleen | Liver | Kidney | Stomach | Heart | Brain |
|---|---|---|---|---|---|---|---|---|---|
| CVL218 | 20 | 180 | 69318 ± 10476 | 20202 ± 2300 | 17215 ± 1919 | 15201 ± 1984 | 8145 ± 2624 | 6077 ± 496 | 3580 ± 416 |
| | | 360 | 18858 ± 2365 | 6358 ± 1058 | 2187 ± 859 | 3903 ± 594 | 1871 ± 813 | 1390 ± 292 | 998 ± 220 |
| | | 480 | 4183 ± 847 | 1475 ± 324 | 213 ± 88 | 993 ± 327 | 569 ± 293 | 275 ± 80 | 317 ± 55 |
| Arbidol | 54 | 5 | 933 ± 837 | 48 ± 35 | 104 ± 82 | 79 ± 54 | 8210 ± 5410 | 72 ± 47 | 101 ± 67 |
| | | 15 | 2603 ± 1848 | 519 ± 281 | 963 ± 290 | 259 ± 190 | 23180 ± 10170 | 132 ± 69 | 50 ± 10 |
| | | 360 | 833 ± 937 | 143 ± 51 | 262 ± 175 | 58 ± 21 | 52750 ± 3059 | 41 ± 28 | 31 ± 21 |

**Table 6 Comparison of tissue to plasma concentration ratios of CVL218 and Arbidol in rats. The data of Arbidol is sourced from X. Liu, K. Pei, X.-h. Chen, K.-s. Bi, Distribution and excretion of arbidol hydrochloride in rats, Chinese journal of new drugs 22 (7) (2013) 829-833.**

| Tissue | Tissue/serum drug concentration ratio | |
|---|---|---|
| | CVL218 | Arbidol |
| Lung | 188.364 ± 28.467 | 0.553 ± 0.392 |
| Spleen | 54.897 ± 6.250 | 0.110 ± 0.060 |
| Liver | 46.780 ± 5.215 | 0.204 ± 0.062 |
| Kidney | 41.307 ± 5.391 | 0.055 ± 0.040 |
| Stomach | 22.133 ± 7.130 | 4.920 ± 2.159 |
| Heart | 16.514 ± 1.348 | 0.028 ± 0.015 |
| Brain | 9.728 ± 1.130 | 0.011 ± 0.002 |

**Table 7 Comparison of pharmacokinetic parameters in rats after oral administration of CVL218 and arbidol at 20/40 mg/kg and 18/54 mg/kg, respectively. The data of Arbidol is sourced from X. Liu, Q.-g. Zhou, H. Li, B.-c. Cai, X.-h. Chen, K.-s. Bi, Pharmacokinetics of arbidol hydrochloride in rats, Chinese pharmacological bulletin 28 (12) (2012) 1747-1750.**

| Drug | Dose (mg/kg) | Gen der | Tmax (h) | Cmax (ng/mL) | AUC0-t (ngh/mL) | AUC_{0-∞} (ngh/mL) | MRT_{0-∞} (h) | t1/2 (h) |
|---|---|---|---|---|---|---|---|---|
| CVL 218 | 20 | Male | 4.0 | 234 ± 35 | 1070 ± 176 | 1111 ± 192 | 3.91 ± 0.19 | 1.19 ± 0.09 |
| | | Fem ale | 3.0 | 502 ± 80 | 2196 ± 228 | 2222 ± 241 | 3.16 ± 0.41 | 1.1 ± 0.16 |
| | | Total | 3.5 | 368 ± 157 | 1633 ± 643 | 1666 ± 639 | 3.54 ± 0.50 | 1.15 ± 0.13 |
| | 40 | Male | 3.0 | 510 ± 259 | 2802 ± 967 | 2830 ± 983 | 4.51 ± 0.18 | 1.3 ± 0.33 |
| | | Fem ale | 2.0 | 940 ± 117 | 5220 ± 1113 | 5242 ± 1115 | 4.05 ± 0.43 | 1.29 ± 0.21 |
| | | Total | 2.5 | 725 ± 296 | 4011 ± 1620 | 4036 ± 1620 | 4.28 ± 0.39 | 1.3 ± 0.24 |
| Arbi dol | 18 | Male | 0.28 ± 0.11 | 1002 ± 298 | 1956 ± 895 | 2224 ± 1058 | - | 3.6 ± 1.2 |
| | 54 | Male | 0.18 ± 0.06 | 4711 ± 2361 | 6790 ± 2749 | 7558 ± 2877 | - | 3.3 ± 0.7 |

### 5.6. CVL218 has a good safety in animals

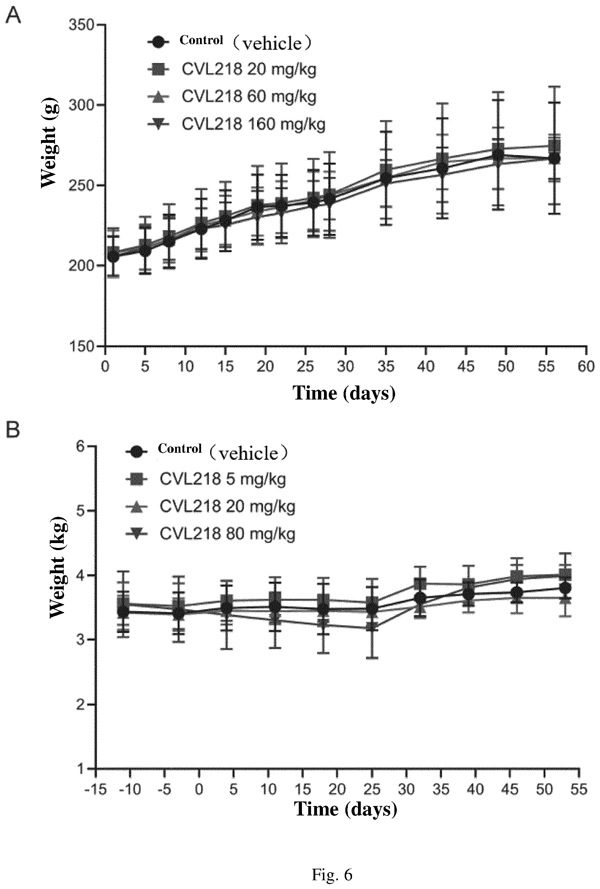

After oral administration of 20/60/160 mg/kg CVL218 to rats for 28 days and then no administration for 28 days (method), we observed no significant difference in body weight between rats and cynomolgus monkeys at different doses and in the control group (Fig. 6).

The *in vivo* data above suggest that CVL218 has favorable pharmacokinetic and safety profiles in rats and monkeys, and its high-level distribution in therapeutic target tissues (i.e., lungs) would be beneficial for treatment of SARS-CoV-2 infections.

### Example 6: Molecular docking indicates interactions between PARP1 inhibitors and the N-terminal domain of coronavirus nucleocapsidin

Molecular docking was performed in the example to investigate the potential mode of interactions between two drugs, olaparib and CVL218, and the N-NTD of SARS-CoV-2.

A molecular interaction model of PARP 1 inhibitors CVL218 and olaparib with the N protein N-terminal domain of SARS-CoV-2 (SARS-CoV-2-N-NTD) was generated using the docking program AutoDock4.2. The structure of SARS-CoV-2-N-NTD for molecular docking was established by homology modeling. The AutoGrid program was employed to generate grid maps with the spacing of 60 × 60 × 60 points of 0.375 Å, which were used to evaluate the binding energy between proteins and ligands.

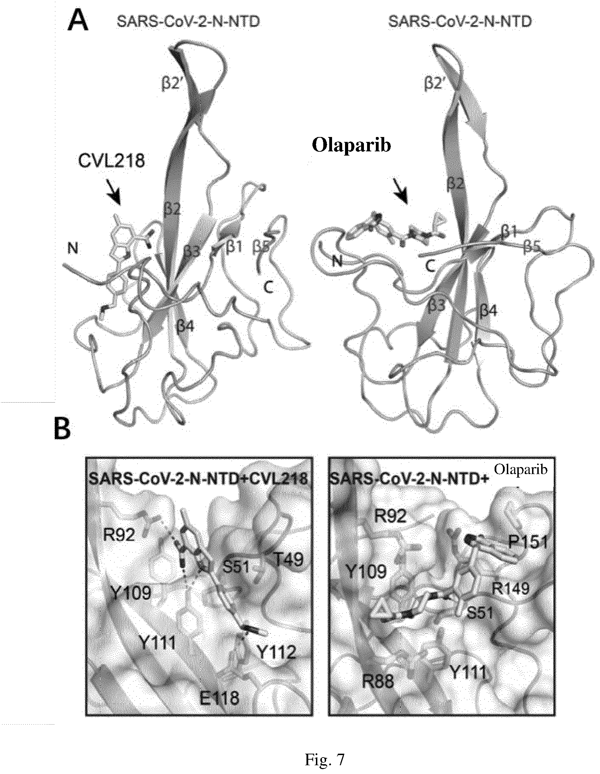

The results showed (Fig. 7) that both CVL218 and olaparib could bind to the N-NTD of SARS-CoV-2. In terms of hydrogen bond formation, CVL218 showed stronger binding ability than olaparib. Meanwhile, key residues on SARS-CoV-2-N-NTD involved in drug binding (i.e., S51, Y109 and Y111) were also highly conserved in other viruses such as SARS-CoV, HCoV-OC43, mouse hepatitis virus (MHV) and infectious bronchitis virus (IBV), indicating that the N-NTDs of different viruses are very likely to exhibit similar binding behaviors to PARP inhibitors, thereby inhibiting viral replication.

In summary, it can be known that PARP1 inhibitors may have therapeutic potential in treatment of diseases caused by viruses such as COVID-19. First, during the life cycle of coronaviruses, PARP1 inhibitors may inhibit viral growth by inhibiting viral replication and preventing the binding of nucleocapsidin to viral RNAs, which can also be supported by our molecular docking results. Second, PARP1 inhibitors play a key role in controlling the inflammatory response by regulating pro-inflammatory factors such as IL-6, thereby providing clinical potential for alleviating cytokine storm and inflammatory response induced by SARS-CoV-2 infection. In the *Diagnosis and Treatment Protocol for Novel Coronavirus Pneumonia (Trial Version* 7) promulgated by the Chinese government, the monoclonal antibody drug tocilizumab against IL-6 is recommended for treatment of COVID-19, which also highlights the important role of inflammatory response in current SARS-CoV-2 treatment. CVL218 can effectively inhibit CpG-induced IL-6 production in PBMCs. This finding suggests that CVL218 may also have IL-6-specific anti-inflammatory effects in those patients with severe SARS-CoV-2 infection.

Although the specific embodiments of the present invention have been described above, those skilled in the art should understand that these are only examples, and various changes or revisions may be made to these embodiments without departing from the principle and essence of the present invention. Therefore, the scope of the present invention shall be defined by the appended claims.

## Claims

1. Application of a poly ADP ribose polymerase inhibitor or a pharmaceutically acceptable salt thereof in the preparation of antiviral agents or in the preparation of medicines for the treatment of diseases caused by viruses, wherein the viruses are β-coronaviruses, preferably viruses causing acute respiratory syndrome such as SARS-related coronavirus, and more preferably SARS-CoV and/or SARS-CoV-2.

2. The application according to claim 1, wherein the poly ADP ribose polymerase inhibitor is an inhibitor against PARP 1 and/or PARP 2.

3. The application according to claim 1, wherein the poly ADP ribose polymerase inhibitor is substance A or a pharmaceutically acceptable salt thereof;
The substance A is selected from one or more of tarazoparib, fluzoparib, simmiparib, IMP4297, BGB-290, ABT-888, the compound shown in formula **I,** the compound shown in formula **II,** the compound shown in formula III and the compound shown in formula **IV;** 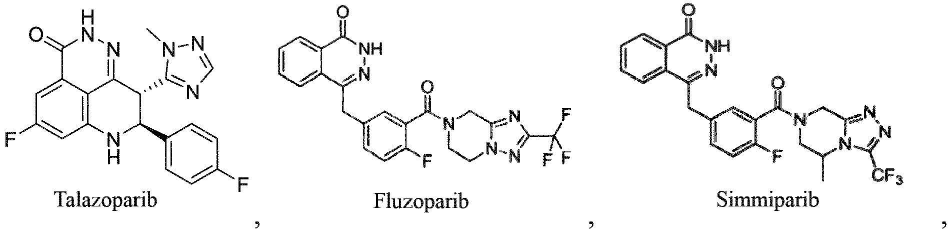 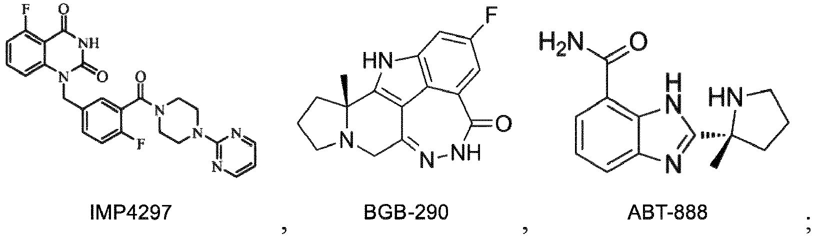 the compound shown in formula I is: 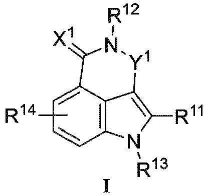
In the formula, R¹¹ is H, halogen, cyano, C₁-C₄ alkyl, C₂-C₄ alkenyl, C₂-C₄ alkynyl, C₃-C₄ cycloalkyl, 3-6-membered heterocycloalkyl "containing 1-2 heteroatoms selected from one or more of O, N and S", C₆-C₁₀ aryl, 5-10-membered heteroaryl "containing 1-2 heteroatoms selected from one or more of O, N and S", C₁-C₄ alkyl substituted by one or more R¹¹⁻¹, C₂-C₄ alkenyl substituted by one or more R¹¹⁻¹, C₂-C₄ alkynyl substituted by one or more R¹¹⁻¹, C₃-C₄ cycloalkyl substituted by one or more R¹¹⁻¹, 3-6-membered heterocycloalkyl "containing 1-2 heteroatoms selected from one or more of O, N and S", C₆-C₁₀ aryl substituted by one or more R¹¹⁻¹ (the "C₆-C₁₀ aryl" is, for example, 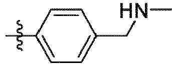 ), 5-10-membered heteroaryl "containing 1-2 heteroatoms selected from one or more of O, N and S", substituted by one or more R¹¹⁻¹, -C(=O)-R¹¹⁻², -C(=O)-O-R¹¹⁻³ or -C(=O)-NR11-4R¹¹⁻⁵;
R¹¹⁻¹ is independently halogen, hydroxyl, carboxyl, nitro, amino, C₁-C₄ alkyl or C₁-C₄ alkyl substituted by one or more NR¹¹⁻¹⁻¹R¹¹⁻¹⁻² (the "C₁-C₄ alkyl" is, for example, methyl);
R¹¹⁻¹⁻¹ and R¹¹⁻¹⁻² are independently hydrogen or C₁-C₄ alkyl (e.g., methyl);
R¹¹⁻², R¹¹⁻³, R¹¹⁻⁴ and R¹¹⁻⁵ are H, C₁-C₄ alkyl, C₂-C₄ alkenyl, C₂-C₄ alkynyl, C₃-C₄ cycloalkyl, 3-6-membered heterocycloalkyl "containing 1-2 heteroatoms selected from one or more of O, N and S", C₆-C₁₀ aryl, 5-10-membered heteroaryl "containing 1-2 heteroatoms selected from one or more of O, N and S", C₁-C₄ alkyl substituted by one or more R¹¹⁻²⁻¹, C₂-C₄ alkenyl substituted by one or more R¹¹⁻²⁻¹, C₂-C₄ alkynyl substituted by one or more R¹¹⁻²⁻¹, C₃-C₄ cycloalkyl substituted by one or more R¹¹⁻²⁻¹, 3-6-membered heterocycloalkyl "containing 1-2 heteroatoms selected from one or more of O, N and S", substituted by one or more R¹¹⁻²⁻¹, C₆-C₁₀ aryl substituted by one or more R¹¹⁻²⁻¹ or 5-10-membered heteroaryl "containing 1-2 heteroatoms selected from one or more of O, N and S", substituted by one or more R¹¹⁻²⁻¹;
R¹¹⁻²⁻¹ is independently halogen, hydroxyl, carboxyl, nitro, amino or C₁-C₄ alkyl;
Y¹ is -(CR^{Y1-1}R^{Y1-2})(CR^{Y1-3}R^{Y1-4})ₙ- or -N=C(R^{Y1-5})-;
n is 0 or 1;
R^{Y1-1}, R^{Y1-2} and R^{Y1-5} are H, C₁-C₄ alkyl, C₂-C₄ alkenyl, C₂-C₄ alkynyl, C₃-C₄ cycloalkyl, 3-6-membered heterocycloalkyl "containing 1-2 heteroatoms selected from one or more of O, N and S", C₆-C₁₀ aryl, 5-10-membered heteroaryl "containing 1-2 heteroatoms selected from one or more of O, N and S", C₁-C₄ alkyl substituted by one or more R^{Y1-1-1}, C₂-C₄ alkenyl substituted by one or more R^{Y1-1-1}, C₂-C₄ alkynyl substituted by one or more R^{Y1-1-1}, C₃-C₄ cycloalkyl substituted by one or more R^{Y1-1-1}, 3-6-membered heterocycloalkyl "containing 1-2 heteroatoms selected from one or more of O, N and S", substituted by one or more R^{Y1-1-1}, C₆-C₁₀ aryl substituted by one or more R^{Y1-1-1} or 5-10-membered heteroaryl "containing 1-2 heteroatoms selected from one or more of O, N and S", substituted by one or more R^{Y1-1-1};
R^{Y1-1-1} is independently halogen, hydroxyl, nitro, amino, C₁-C₄ alkyl or C₁-C₄ alkoxy;
R^{Y1-3} and R^{Y1-4} are H, C₁-C₄ alkyl, C₂-C₄ alkenyl, C₂-C₄ alkynyl, C₃-C₄ cycloalkyl, 3-6-membered heterocycloalkyl "containing 1-2 heteroatoms selected from one or more of O, N and S", 5-10-membered C₆-C₁₀ aryl, heteroaryl "containing 1-2 heteroatoms selected from one or more of O, N and S", C₁-C₄ alkyl substituted by one or more R^{Y1-3-1}, C₂-C₄ alkenyl substituted by one or more R^{Y1-3-1}, C₂-C₄ alkynyl substituted by one or more R^{Y1-3-1}, C₃-C₄ cycloalkyl substituted by one or more R^{Y1-3-1}, 3-6-membered heterocycloalkyl "containing 1-2 heteroatoms selected from one or more of O, N and S", substituted by one or more R^{Y1-3-1}, C₆-C₁₀ aryl substituted by one or more R^{Y1-3-1} or 5-10-membered heteroaryl "containing 1-2 heteroatoms selected from one or more of O, N and S", substituted by one or more R^{Y1-3-1};
R^{Y1-3-1} is independently halogen, hydroxyl, nitro, amino, C₁-C₄ alkyl or C₁-C₄ alkoxy;
R¹² is H or C₁-C₄ alkyl;
X¹ is O or S;
R¹⁴ is H, halogen (for example, fluorine, chlorine or bromine) or C₁-C₄ alkyl;
R¹³ is H or C₁-C₄ alkyl;
The compound shown in formula II is: 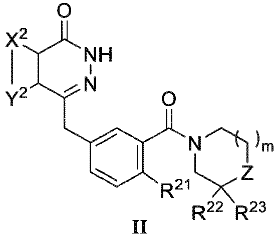
In the formula, X², Y² and the carbon atoms connected thereto together form a C₆-C₁₀ aryl (for example, phenyl), or a C₆-C₁₀ aryl substituted by one or more R^{X2-1};
R^{X2-1} is independently halogen, nitro, hydroxyl, sulfydryl, amino, C₁-C₇ alkyl, C₆-C₂₀ aryl, 3-20-membered heterocyclyl "containing 1-6 heteroatoms selected from one or more O, N and S", -OR^{X2-1-1} or -SR^{X2-1-2};
R^{X2-1-1} and R^{X2-1-2} are independently C₁-C₇ alkyl, C₆-C₂₀ aryl, or 3-20-membered heterocyclyl "containing 1-6 heteroatoms selected from one or more of O, N and S";
R²¹ is H or halogen (for example, fluorine, chlorine or bromine);
Z is -NR^{Z-1}- or -CR^{Z-2}R^{Z-3}-;
When Z is -NR^{Z-1}-, m will be 1 or 2; when Z is -CR^{Z-2}R^{Z-3}-, m will be 1;
R^{Z-1} and R^{Z-2} are independently C₁-C₂₀ alkyl, C₆-C₂₀ aryl, heterocyclyl "containing 1-6 heteroatoms selected from one or more of O, N and S, 3-20-membered", -C(=O)NR^{Z-1-1}R^{Z-1-2}, -C(=O)R^{Z-1-3} (for example, 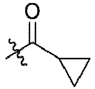 ), -C(=O)OR^{Z-1-4}, -C(=S)NR^{Z-1-5}R^{Z-1-6}, -S(=O)₂R^{Z-1-7}, C₁-C₂₀ alkyl substituted by one or more R^{Z-1-8}, C₆-C₂₀ aryl substituted by one or more R^{Z-1-9}, or 3-20-membered heterocyclyl "containing 1-6 heteroatoms selected from one or more of O, N and S", substituted by one or more R^{Z-1-10};
R^{Z-1-1}, R^{Z-1-2}, R^{Z-1-3}, R^{Z-1-4}, R^{Z-1-5}, R^{Z-1-6} and R^{Z-1-7} are independently hydrogen, C₁-C₇ alkyl, C₆-C₂₀ aryl, or 3-20-membered heterocyclyl "containing 1-6 heteroatoms selected from one or more of O, N and S";
Or, R^{Z-1-1}, R^{Z-1-2} and the carbon atoms connected thereto together form 4-8-membered heterocyclyl "containing 1-2 heteroatoms selected from one or more of O, N and S";
Or, R^{Z-1-5}, R^{Z-1-6} and the carbon atoms connected thereto together form 4-8-membered heterocyclyl "containing 1-2 heteroatoms selected from one or more of O, N and S";
R^{Z-1-8}, R^{Z-1-9} and R^{Z-1-10} are independently C₁-C₇ alkyl, C₆-C₂₀ aryl, 3-20-membered heterocyclyl "containing 1-6 heteroatoms selected from one or more of O, N and S", halogen, hydroxyl, nitro, cyano, carboxyl, sulfydryl, carbamido, -C(=O)NR^{Z-1-8-1}R^{Z-1-8-2}, -C(=O)R^{Z-1-8-3}, -C(=O)OR^{Z-1-8-4}, -C(=S)NR^{Z-1-8-5}R^{Z-1-8-6}, -S(=O)₂R^{Z-1-8-7}, -OR^{Z-1-8-8}, -SR^{Z-1-8-9}, -S(=O)₂NR^{Z-1-8-10}R^{Z-1-8-11}, -OC(=O)R^{Z1-8-1-2}, -S(=O)NR^{Z-1-8-13}R^{Z-1-8-14} or -C(=O)-NH-C(=O)R^{Z-1-8-15};
R^{Z-1-8-1}, R^{Z-1-8-2}, R^{Z-1-8-3}, R^{Z-1-8-4}, R^{Z-1-8-5}, R^{Z-1-8-6}, R^{Z-1-8-7}, R^{Z-1-8-8}, R^{Z-1-8-9}, R^{Z-1-8-10}, R^{Z-1-8-11}, R^{Z-1-8-12}, R^{Z-1-8-13}, R^{Z-1-8-14} and R^{Z-1-8-15} are independently hydrogen, C₁-C₇ alkyl, C₆-C₂₀ aryl, or 3-20-membered heterocyclyl "containing 1-6 heteroatoms selected from one or more of O, N and S";
Or, R^{Z-1-8-1}, R^{Z-1-8-2} and the carbon atoms connected thereto together form 4-8-membered heterocyclyl "containing 1-2 heteroatoms selected from one or more of O, N and S";
Or, R^{Z-1-8-5}, R^{Z-1-8-6} and the carbon atoms connected thereto together form 4-8-membered heterocyclyl "containing 1-2 heteroatoms selected from one or more of O, N and S";
Or, R^{Z-1-8-10}, R^{Z-1-8-11} and the carbon atoms connected thereto together form 4-8-membered heterocyclyl "containing 1-2 heteroatoms selected from one or more of O, N and S";
Or, R^{Z-1-8-13}, R^{Z-1-8-14} and the carbon atoms connected thereto together form 4-8-membered heterocyclyl "containing 1-2 heteroatoms selected from one or more of O, N and S";
R^{Z-3} is H, hydroxyl or amino;
Or, R^{Z-2}, R^{Z-3} and the carbon atoms connected thereto together form C₃-C₇ spirocycloalkyl, or 3-7-membered heterospirocycloalkyl "containing 1-3 heteroatoms selected from one or more of O, N and S";
Both R²² and R²³ are hydrogens, or, when Z is -CR^{Z-2}R^{Z-3}-, R²², R²³, R^{Z-2}, R^{Z-3} and the carbon atoms connected thereto together form a C₆-C₁₀ aryl (for example, phenyl), or a C₆-C₁₀ aryl substituted by one or more R²²⁻¹;
R²²⁻¹ is independently C₁-C₇ alkyl, C₆-C₂₀ aryl, 3-20-membered heterocyclyl "containing 1-6 heteroatoms selected from one or more of O, N and S", hydroxyl, sulfydryl, amino, -OR²²⁻¹⁻¹, -SR²²⁻¹⁻² or -O-(CH₂)ₚ-O-; p is 1, 2 or 3;
R²²⁻¹⁻¹ and R²²⁻¹⁻² are independently C₁-C₇ alkyl, C₆-C₂₀ aryl, or 3-20-membered heterocyclyl "containing 1-6 heteroatoms selected from one or more of O, N and S";
The compound shown in formula **III** is: 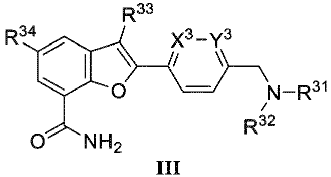
R³¹ and R³² are independently hydrogen, C₁-C₄ alkyl (for example, methyl), C₃-C₄ cycloalkyl or 5- or 6-membered heterocycloalkyl "containing 1-3 heteroatoms selected from one or more of O and N";
Or, R³¹, R³² and the N atoms connected thereto together form 5-6-membered heterocycloalkyl "containing 1-3 heteroatoms selected from one or more of O, N and S", or 5-6-membered heterocycloalkyl "containing 1-3 heteroatoms selected from one or more of O, N and S", substituted by one or more R³¹⁻¹;
R³¹⁻¹ is C₁-C₄ alkyl (for example, methyl) on N;
X³ is CH, CF or N;
Y³ is CH, CF or N;
R³³ is H or Cl;
R³⁴ is H or F;
The compound shown in formula IV is: 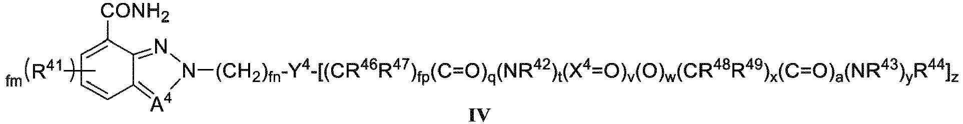
fm is 0, 1, 2 or 3;
R⁴¹ is independently hydroxy, halogen (e.g., fluorine), cyano, C₁₋₆ alkyl, halogenated C₁₋₆ alkyl, C₁₋₆ alkoxy, or halogenated C₁₋₆ alkoxy;
A⁴ is CH or N;
fn is 0, 1, 2, 3, 4, 5 or 6;
Y⁴ is a single bond, C₃₋₅ cycloalkyl, a 4-membered saturated heterocycle containing one N atom, a 5-, 6- or 7-membered saturated or partially saturated heterocycle containing 1, 2 or 3 heteroatoms independently selected from N, O and S, a 5-membered unsaturated heterocycle containing 1, 2, 3 or 4 heteroatoms independently selected from O, N and S but not more than one being O or S, a 6-membered unsaturated heterocycle containing 1, 2 or 3 nitrogen atoms, "a 6-13-membered saturated, partially saturated or unsaturated hydrocarbon ring" (for example, C₆-C₁₀ aryl, or for example, phenyl), or, "a 8-13 membered unsaturated or partially saturated heterocycle containing 1, 2, 3 or 4 heteroatoms independently selected from N, O and S";
z is 1, 2 or 3
fp is 0, 1, 2, 3, 4, 5 or 6;
R⁴⁶ and R⁴⁷ are independently hydrogen or C₁₋₆ alkyl;
q is 0 or 1;
t is 0 or 1;
R⁴² is hydrogen, C₁₋₆ alkyl or C₃₋₁₀ cycloalkyl;
v is 0 or 1;
X⁴ is C or S=O;
w is 0 or 1;
x is 0, 1, 2, 3, 4, 5 or 6;
R⁴⁸ and R⁴⁹ are independently hydrogen, C₁₋₆ alkyl, hydroxy, halo C₁₋₆ alkyl, hydroxyl C₁₋₆ alkyl, amino, C₁₋₆ alkylamino or di(C₁₋₆ alkyl)amino;
a is 0 or 1;
y is 0 or 1;
R⁴³ is hydrogen or C₁₋₆ alkyl;
R⁴⁴ is hydrogen, hydroxy, cyano, halogen (e.g., fluorine, chlorine or bromine), C₁₋₆ alkyl, C₂₋₁₀ alkenyl, halo C₁₋₆ alkyl, hydroxy C₁₋₆ alkyl, C₁₋₆ alkylcarbonyl, C₁₋₆ alkoxy, halo C₁₋₆ alkoxy, C₁₋₆ alkoxycarbonyl, carboxyl, nitro, R⁴⁴⁻¹, or, R⁴⁴⁻³ substituted by one or more -(CH₂)_{b}R⁴⁴⁻²;
R⁴⁴⁻¹ and R⁴⁴⁻³ are independently C₆₋₁₀ aryl, C₆₋₁₀ aryloxy, C₆₋₁₀ arylcarbonyl, C₃₋₁₀ cycloalkyl, 4-membered saturated heterocycle containing one N atom, "5- or 6-membered saturated or partially saturated heterocycle containing 1, 2 or 3 atoms independently selected from N, O and S" (for example, 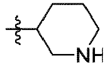 ), "5-membered heteroaromatic ring containing 1, 2, 3 or 4 heteroatoms independently selected from O, N and S but not more than one being O or S", "6-membered heteroaromatic ring containing 1, 2 or 3 nitrogen atoms", or "7- to 15-membered unsaturated, partially saturated or saturated heterocycle containing 1, 2, 3 or 4 heteroatoms independently selected from N, O and S";
b is independently 0, 1, 2, 3, 4, 5 or 6;
R⁴⁴⁻² is independently hydroxy, oxo, cyano, halogen, C₁₋₆ alkyl, C₂₋₁₀ alkenyl, halo C₁₋₆ alkyl, C₁₋₆ alkylcarbonyl, C₁₋₆ alkoxy, halo C₁₋₆ alkoxy, hydroxy C₁₋₆ alkyl, C₁₋₆ alkoxycarbonyl, carboxyl, -NR^{a}R^{b}, -C(=O)NR^{a}R^{b}, S(=O)_{fr}R^{e}, R⁴⁴⁻²⁻¹, or R⁴⁴⁻²⁻³ substituted by one or more R⁴⁴⁻²⁻²;
R^{a} and R^{b} are independently hydrogen, C₁₋₆ alkyl, C₁₋₆ alkylcarbonyl, halo C₁₋₆ alkyl, hydroxyl C₁₋₆ alkyl, S(O)ₛᵣR^{c} or S(O)ₜᵣN(R^{d})₂; sr and tr are independently 0, 1 or 2;
R^{c} is C₁₋₆ alkyl, R^{c-1} or R^{c-3} substituted by one or more R^{c-2};
R^{c-1} and R^{c-3} are independently C₆₋₁₀ aryl, "5-membered heteroaromatic ring containing 1, 2, 3 or 4 heteroatoms independently selected from N, O and S, not more than one being O or S", "6-membered heteroaromatic ring containing 1, 2 or 3 nitrogen atoms", or "7- to 10-membered unsaturated or partially saturated heterocycle containing 1, 2, 3 or 4 heteroatoms independently selected from N, O and S";
R^{c-2} is independently hydroxy, cyano, halogen, C₁₋₆ alkyl, C₂₋₁₀ alkenyl or halo C₁₋₆ alkyl;
R^{d} is independently hydrogen or C₁₋₆ alkyl;
Or, R^{a}, R^{b} and the N atoms connected thereto together form R^{a-1}, or R^{a-3} substituted by one or more R^{a-2};
R^{a-1} and R^{a-3} are independently "4-membered saturated heterocycle containing one N atom", or "5-, 6- or 7-membered saturated or partially saturated heterocycle containing 1, 2 or 3 N atoms and 0 or 1 O atom";
R^{a-2} is independently hydroxy, cyano, halogen, C₁₋₆ alkyl, C₁₋₆ alkoxy, C₂₋₁₀ alkenyl or halo C₁₋₆ alkyl;
fr is 0, 1 or 2;
R^{e} is C₁₋₆ alkyl, R^{c-1} or R^{e-3} substituted by one or more R^{e-2};
R^{e-1} and R^{e-3} are independently C₆₋₁₀ aryl, "5-membered heteroaromatic ring containing 1, 2, 3 or 4 heteroatoms independently selected from N, O and S, no more than one thereof being O or S", "6-membered heteroaromatic ring containing 1, 2 or 3 nitrogen atoms", or "7- to 10-membered unsaturated or partially saturated heterocycle containing 1, 2, 3 or 4 heteroatoms independently selected from N, O and S";
R^{e-2} is independently hydroxy, cyano, halogen, C₁₋₆ alkyl, C₂₋₁₀ alkenyl or halo C₁₋₆ alkyl;
R⁴⁴⁻²⁻¹ and R⁴⁴⁻²⁻³ are independently C₆₋₁₀ aryl, C₆₋₁₀ aryl C₁₋₆ alkyl, "4-membered saturated heterocycle containing one N atom", "5-, 6- or 7-membered saturated or partially saturated heterocycle containing 1, 2 or 3 atoms independently selected from N, O and S", "5-membered heteroaromatic ring containing 1, 2, 3 or 4 heteroatoms independently selected from O, N and S but not more than one being O or S", "6-membered heterocycle containing 1, 2 or 3 nitrogen atoms", or "7- to 10-membered unsaturated or partially saturated heterocycle containing 1, 2, 3 or 4 heteroatoms independently selected from N, O and S";
R⁴⁴⁻²⁻² is independently hydroxy, cyano, halogen, C₁₋₆ alkyl, C₁₋₆ alkoxy, C₂₋₁₀ alkenyl, halo C₁₋₆ alkyl, amino, C₁₋₆ alkylamino and di(C₁₋₆ alkyl)amino.

4. The application according to claim 3, wherein, the compound shown in formula I has the following definitions:
R¹¹ is C₆-C₁₀ aryl substituted by one or more R¹¹⁻¹ (the "C₆-C₁₀ aryl" is, for example, phenyl; the "C₆-C₁₀ aryl substituted by one or more R¹¹⁻¹" is, for example, 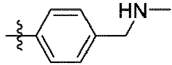 );
R¹¹⁻¹ is independently halogen, hydroxyl, carboxyl, nitro, amino, C₁-C₄ alkyl or C₁-C₄ alkyl substituted by one or more NR¹¹⁻¹⁻¹R¹¹⁻¹⁻² (the "C₁-C₄ alkyl" are for example, methyl);
R¹¹⁻¹⁻¹ and R¹¹⁻¹⁻² are independently hydrogen or C₁-C₄ alkyl (e.g., methyl);
Y¹ is -(CR^{Y1-1}R^{Y1-2})(CR^{Y1-3}R^{Y1-4})ₙ-;
n is 0 or 1;
R^{Y1-1} and R^{Y1-2} are independently H or C₁-C₄ alkyl;
R^{Y1-3} and R^{Y1-4} are independently H or C₁-C₄ alkyl;
R¹² is H or C₁-C₄ alkyl;
X¹ is O or S;
R¹⁴ is H or halogen (for example, fluorine, chlorine or bromine);
R¹³ is H or C₁-C₄ alkyl;
The compound shown in formula **II** has the following definitions:
X², Y² and the carbon atoms connected thereto together form a C₆-C₁₀ aryl (for example, phenyl), or a C₆-C₁₀ aryl substituted by one or more R^{X2-1};
R^{X2-1} is independently halogen, nitro, hydroxyl, sulfydryl, amino, C₁-C₇ alkyl, C₆-C₂₀ aryl, 3-20-membered heterocyclyl "containing 1-6 heteroatoms selected from one or more of O, N and S", -OR^{X2-1-1} or -SR^{X2-1-2};
R^{X2-1-1} and R^{X2-1-2} are independently C₁-C₇ alkyl, C₆-C₂₀ aryl, or 3-20-membered heterocyclyl "containing 1-6 heteroatoms selected from one or more of O, N and S";
R²¹ is H or halogen (for example, fluorine, chlorine or bromine);
Z is -NR^{Z-1}-; m is 1 or 2;
R^{Z-1} is independently -C(=O)NR^{Z-1-1}R^{Z-1-2}, -C(=O)R^{Z-1-3} (for example, 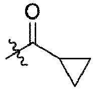 ), -C(=O)OR^{Z-1-4}, -C(=S)NR^{Z-1-5}R^{Z-1-6} or -S(=O)₂R^{Z-1-7};
R^{Z-1-1}, R^{Z-1-2}, R^{Z-1-3}, R^{Z-1-4}, R^{Z-1-5}, R^{Z-1-6} and R^{Z-1-7} are independently hydrogen, C₁-C₇ alkyl, C₆-C₂₀ aryl, or 3-20-membered heterocyclyl "containing 1-6 heteroatoms selected from one or more of O, N and S";
or, R^{Z-1-1}, R^{Z-1-2} and the carbon atoms connected thereto together form 4-8-membered heterocyclyl "containing 1-2 heteroatoms selected from one or more of O, N and S";
or, R^{Z-1-5}, R^{Z-1-6} and the carbon atoms connected thereto together form 4-8-membered heterocyclyl "containing 1-2 heteroatoms selected from one or more of O, N and S";
both R²² and R²³ are hydrogen;
the compound shown in formula **III** has the following definitions:
R³¹ is hydrogen, methyl, ethyl, isopropyl, cyclopropyl, piperidin-4-yl or *(R)*-tetrahydrofuran-3-yl, preferably hydrogen or methyl;
R³² is hydrogen, methyl, ethyl, isopropyl, cyclopropyl, piperidin-4-yl or *(R)*-tetrahydrofuran-3-yl, preferably methyl, isopropyl, cyclopropyl, piperidin-4-yl or *(R)*-tetrahydrofuran-3-yl;
or, R³¹, R³² and the N atom connected thereto together form unsubstituted or substituted morpholinyl, piperazinyl, homopiperazinyl, thiomorpholinyl, piperidinyl or tetrahydropyrrolyl, wherein the substitution means that N is substituted by methyl;
X³ is CH, CF or N;
Y³ is CH, CF or N;
R³³ is H;
R³⁴ is F;
the compound shown in formula **IV** has the following definitions:
fm is 0, 1, 2 or 3;
R⁴¹ is independently hydroxy, halogen, cyano, C₁₋₆ alkyl, halogenated C₁₋₆ alkyl, C₁₋₆ alkoxy, or halogenated C₁₋₆ alkoxy;
A⁴ is CH or N;
fn is 0;
Y⁴ is a 5-, 6- or 7-membered saturated or partially saturated heterocycle containing 1, 2 or 3 heteroatoms independently selected from N, O and S, a 5-membered unsaturated heterocycle containing 1, 2, 3 or 4 heteroatoms independently selected from O, N and S but not more than one being O or S, a 6-membered unsaturated heterocycle containing 1, 2 or 3 nitrogen atoms, "a 6-13-membered saturated, partially saturated or unsaturated hydrocarbon ring" (for example, C₆-C₁₀ aryl, or for example, phenyl), or, "a 8-13 membered unsaturated or partially saturated heterocycle containing 1, 2, 3 or 4 heteroatoms independently selected from N, O and S";
z is 1 or 2;
fp is 0;
q is 0;
t is 0;
v is 0;
w is 0;
x is 0;
a is 0;
y is 0;
R⁴⁴ is halogen or R⁴⁴⁻¹;
R⁴⁴⁻¹ is C₃₋₁₀ cycloalkyl, 4-membered saturated heterocycle containing one N atom, "5- or 6-membered saturated or partially saturated heterocycle containing 1, 2 or 3 atoms independently selected from N, O and S" (for example, 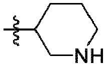 ), or, "7-15-membered unsaturated, partially saturated or saturated heterocycle containing 1, 2, 3 or 4 heteroatoms independently selected from N, O and S";
and/or, the compound shown in formula **IV** has the following definitions: It is a compound as shown in formula IV-1, 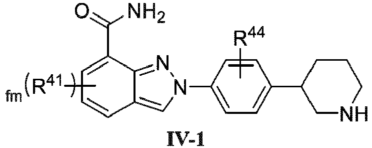
fm is 0 or 1;
R⁴¹ is halogen (e.g., fluorine);
R⁴⁴ is hydrogen or halogen (e.g., fluorine);

5. The application according to claim 4, wherein, the compound shown in formula **I** is any of the following compounds: 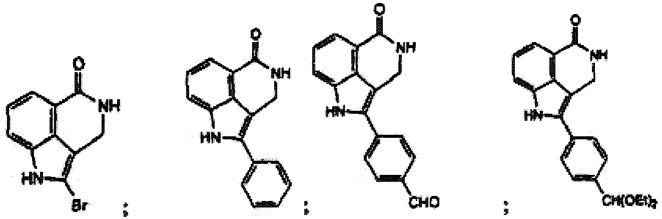 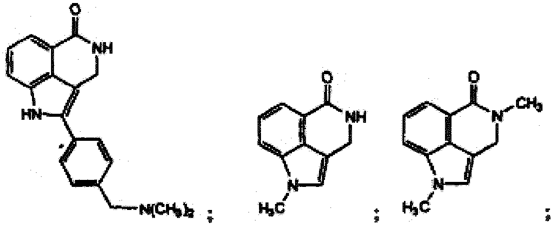 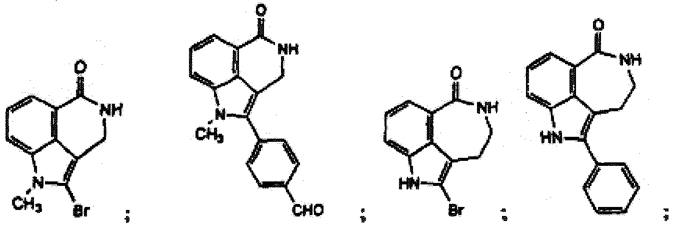 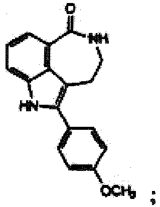 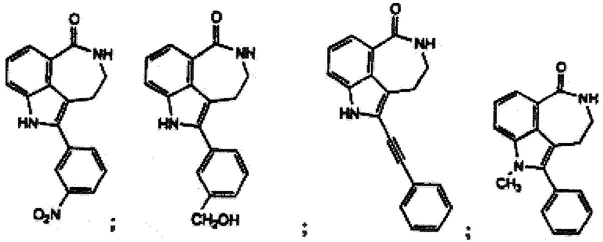 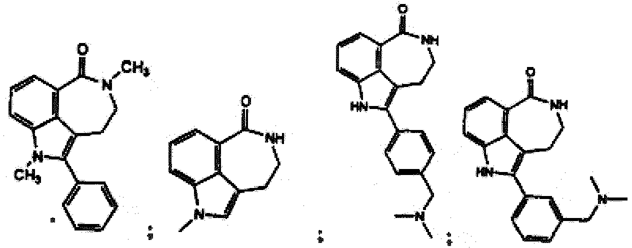 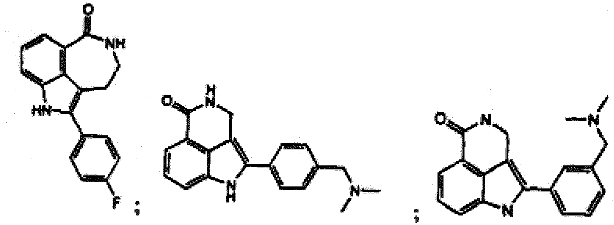 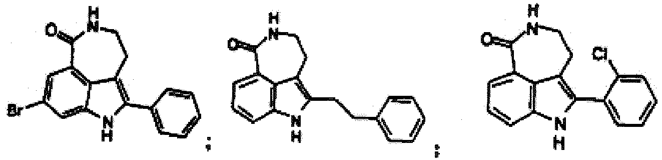 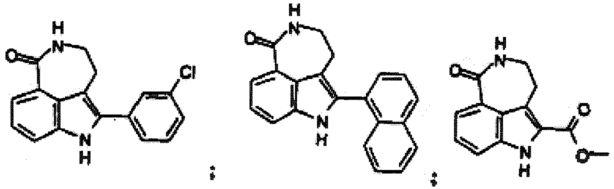 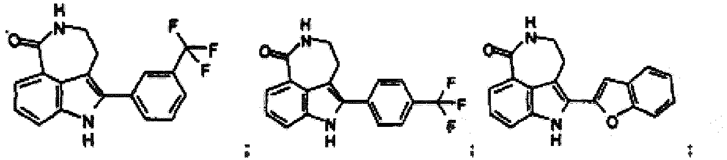 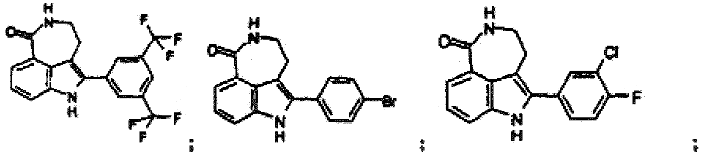 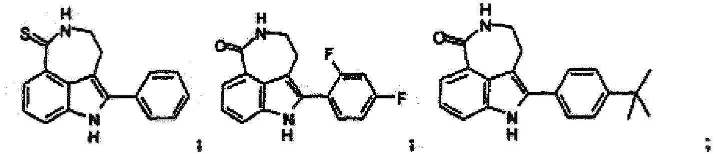 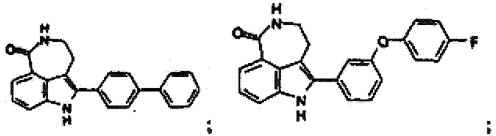 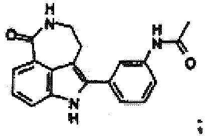 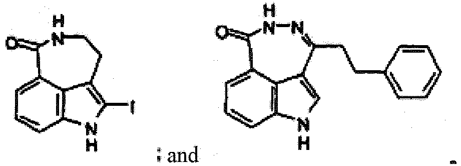 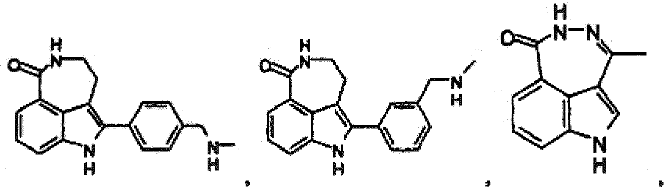 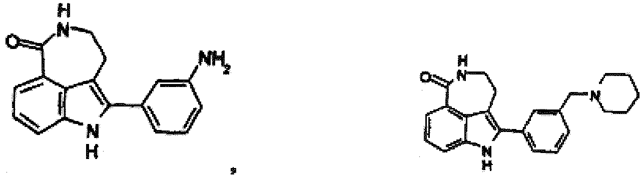 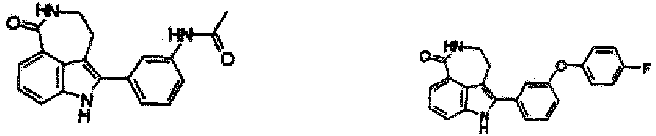 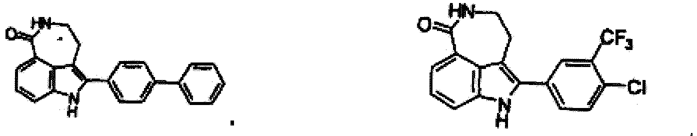 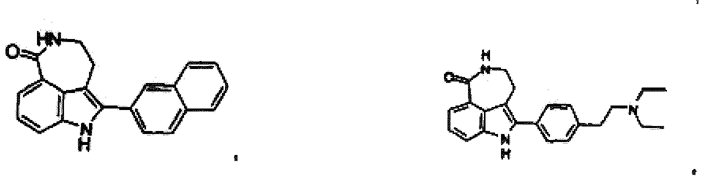 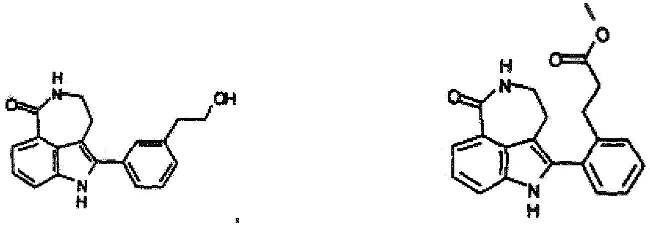 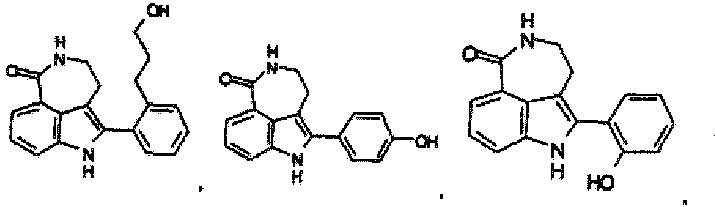 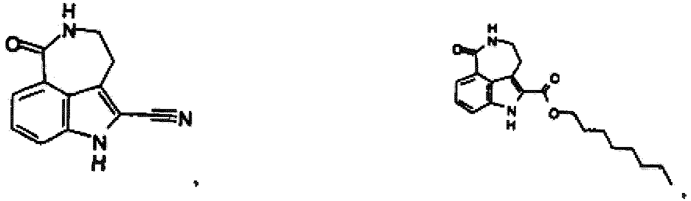 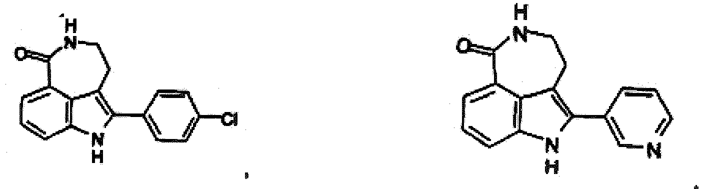 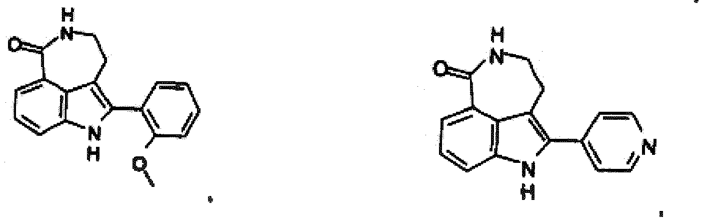 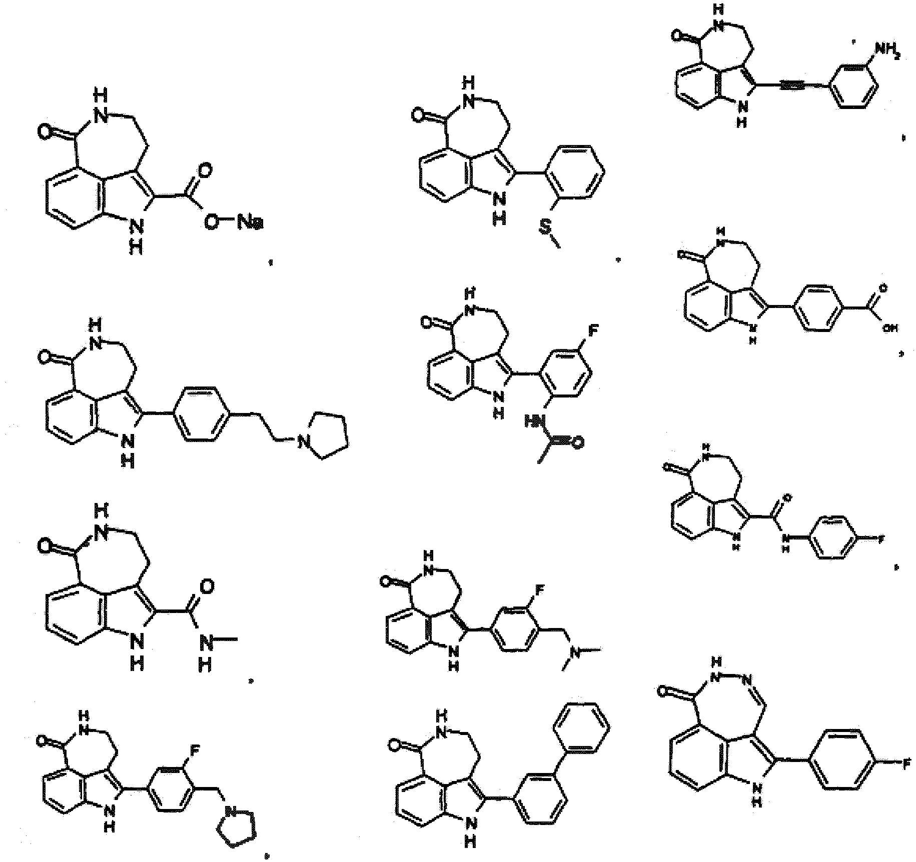 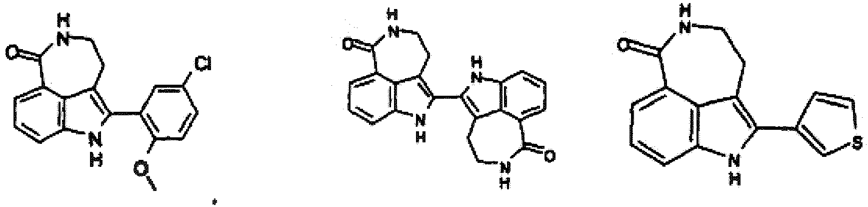 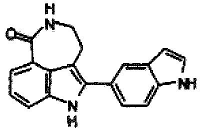 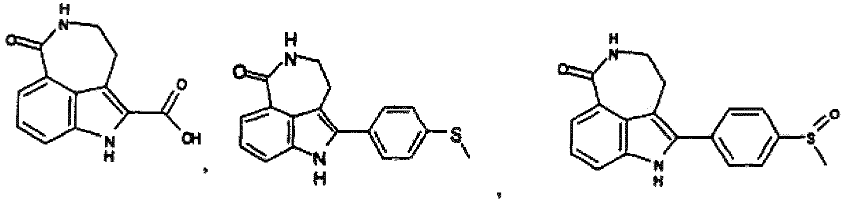 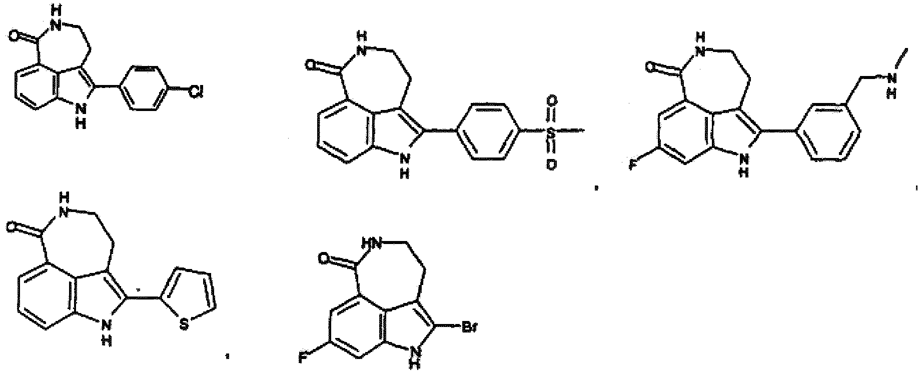 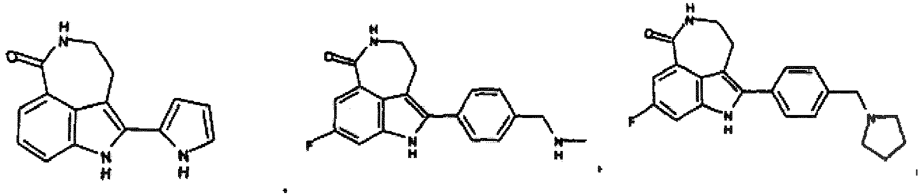 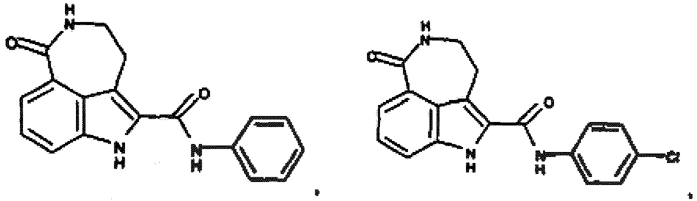 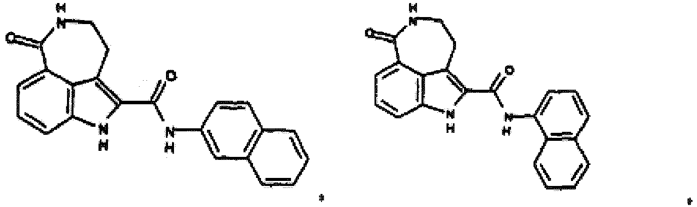 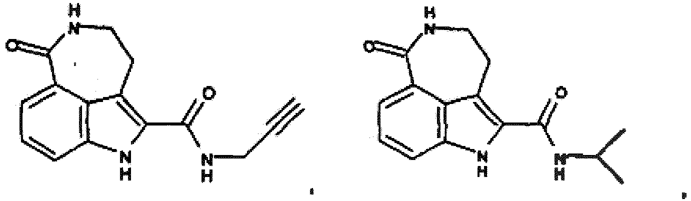 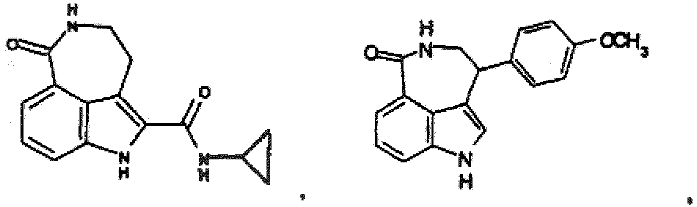 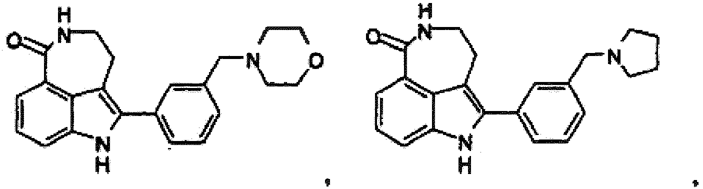 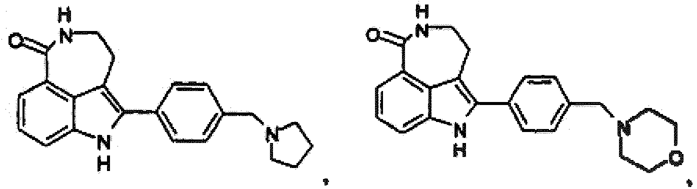 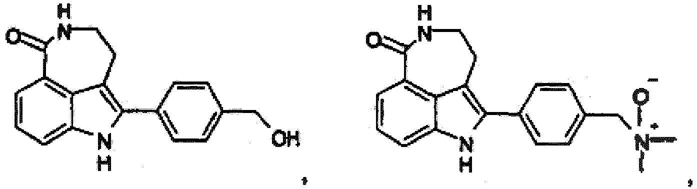 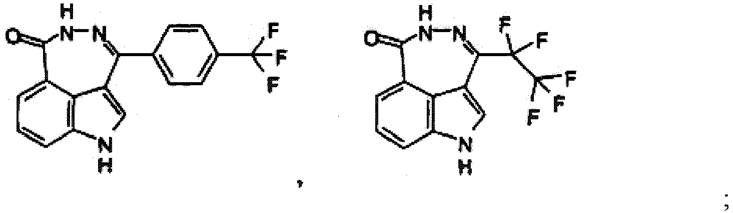
The compound shown in formula **II** is any of the following compounds: 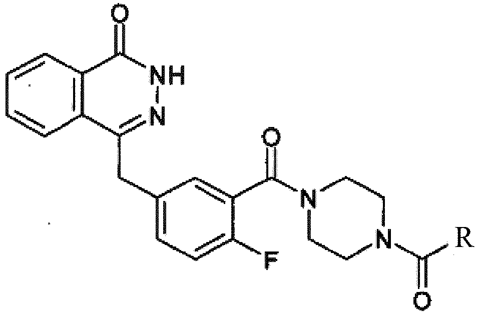 wherein, R is selected from
**a)** 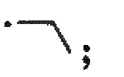
b) 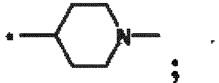
**c)** 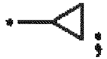
**d)** 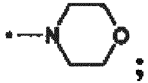
**e)** 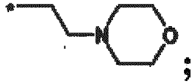
**f)** 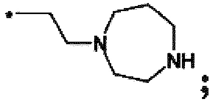
**g)** 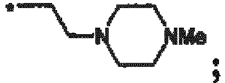 and **h)** 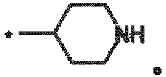 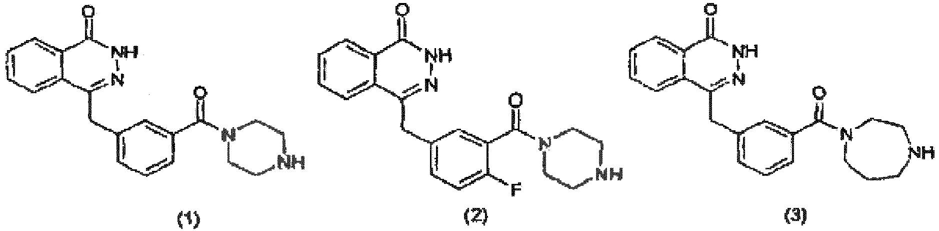 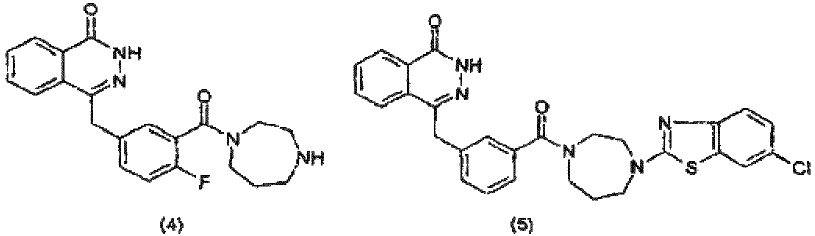 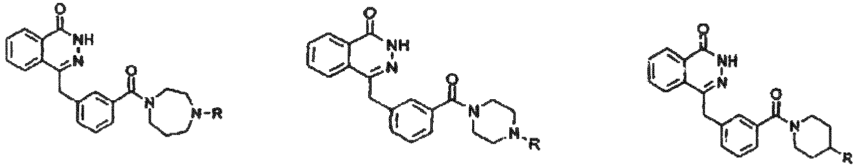 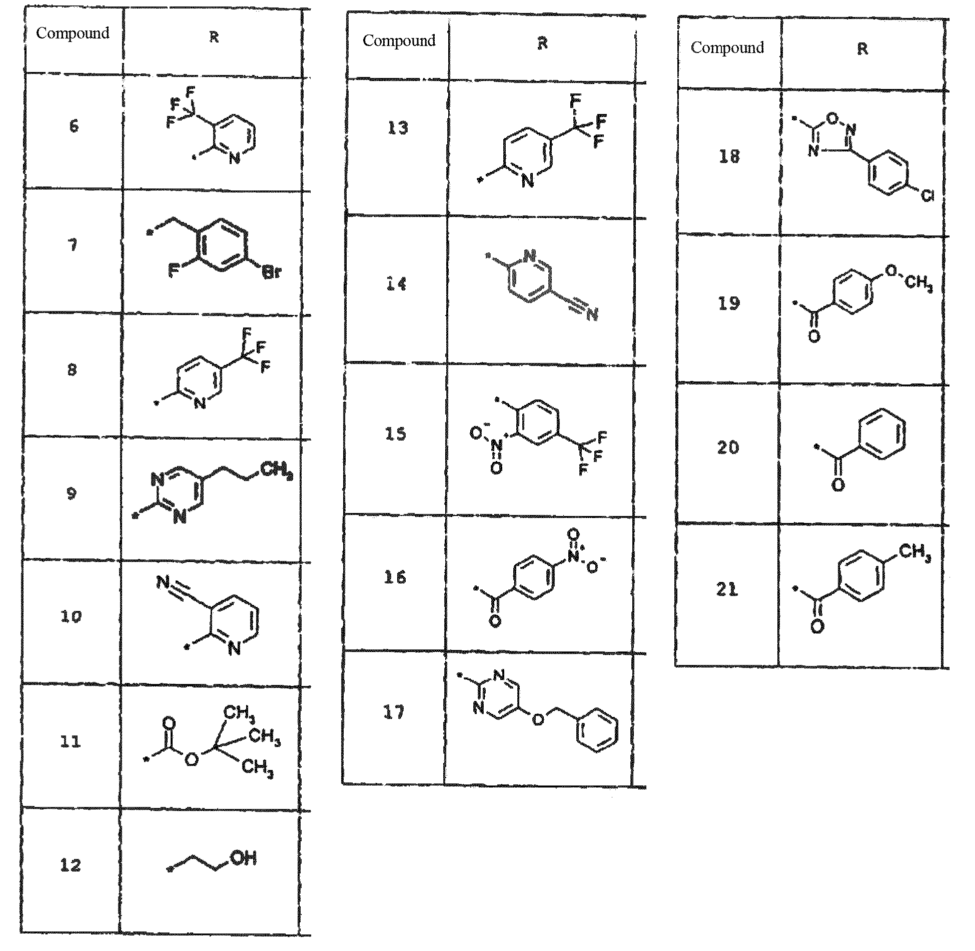 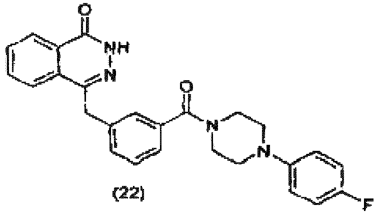 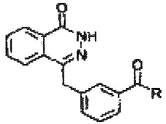 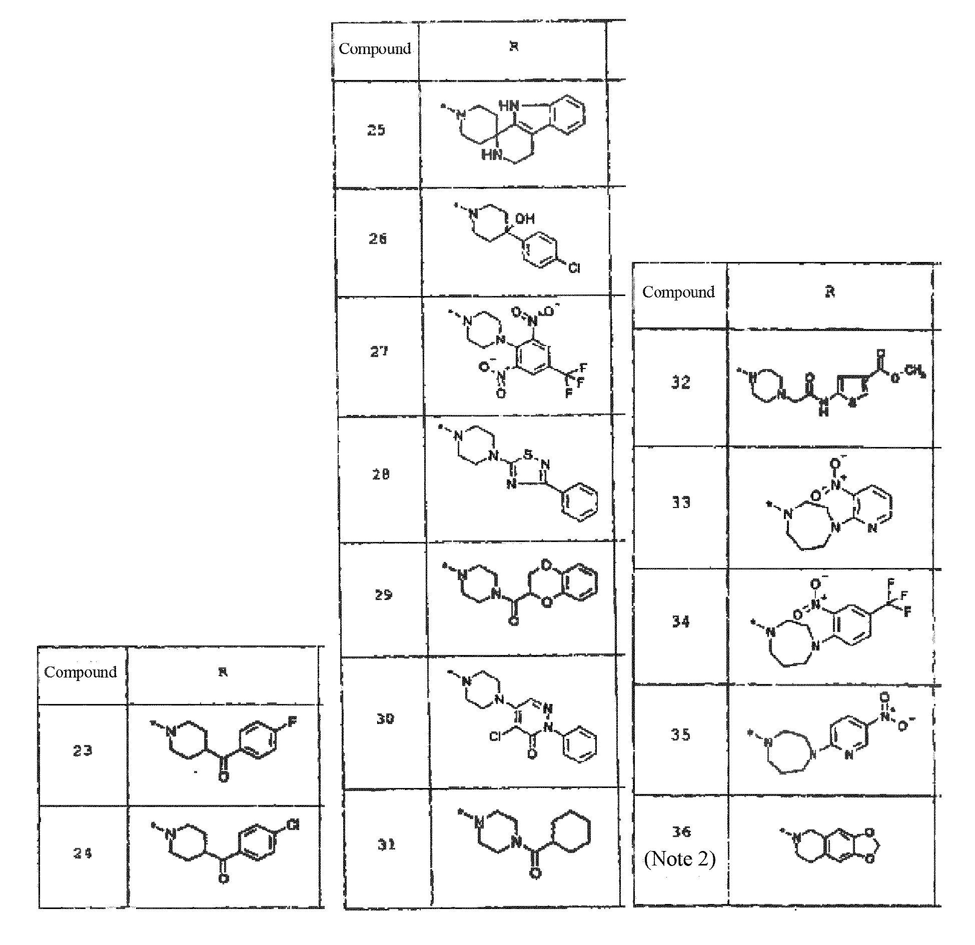 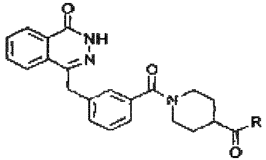 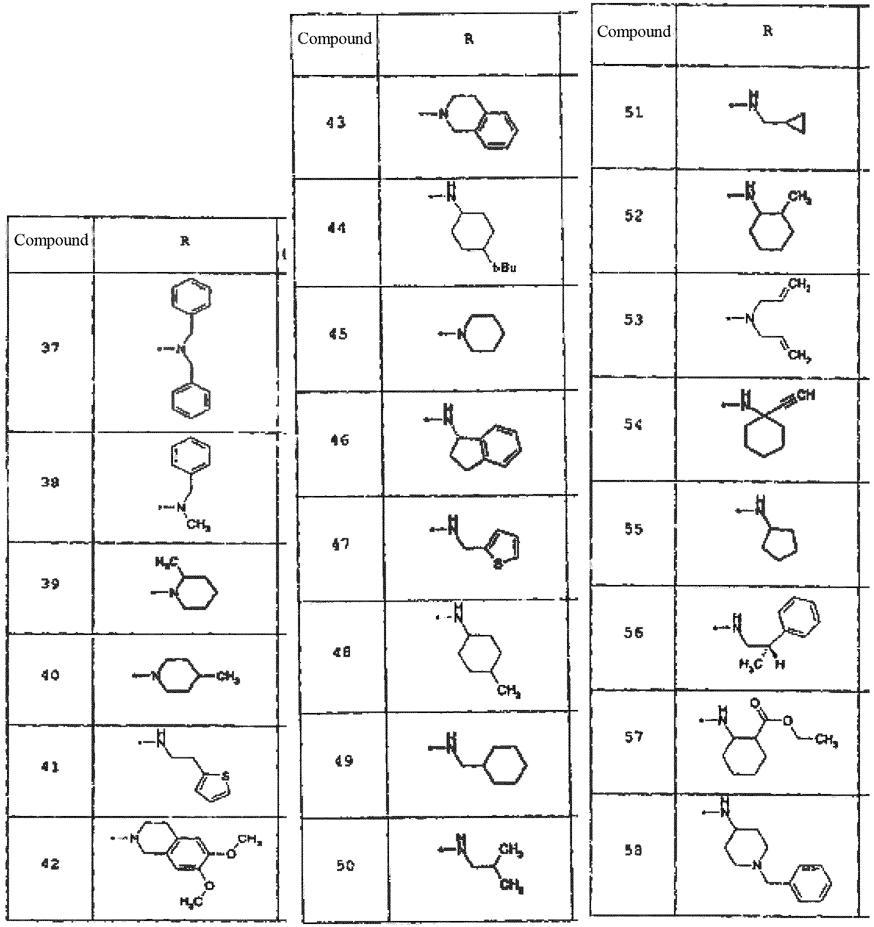 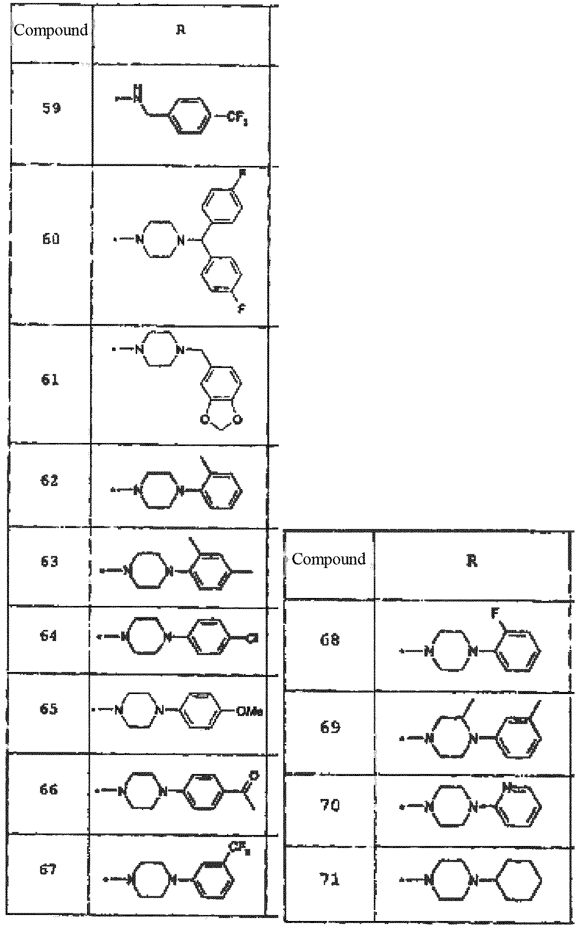 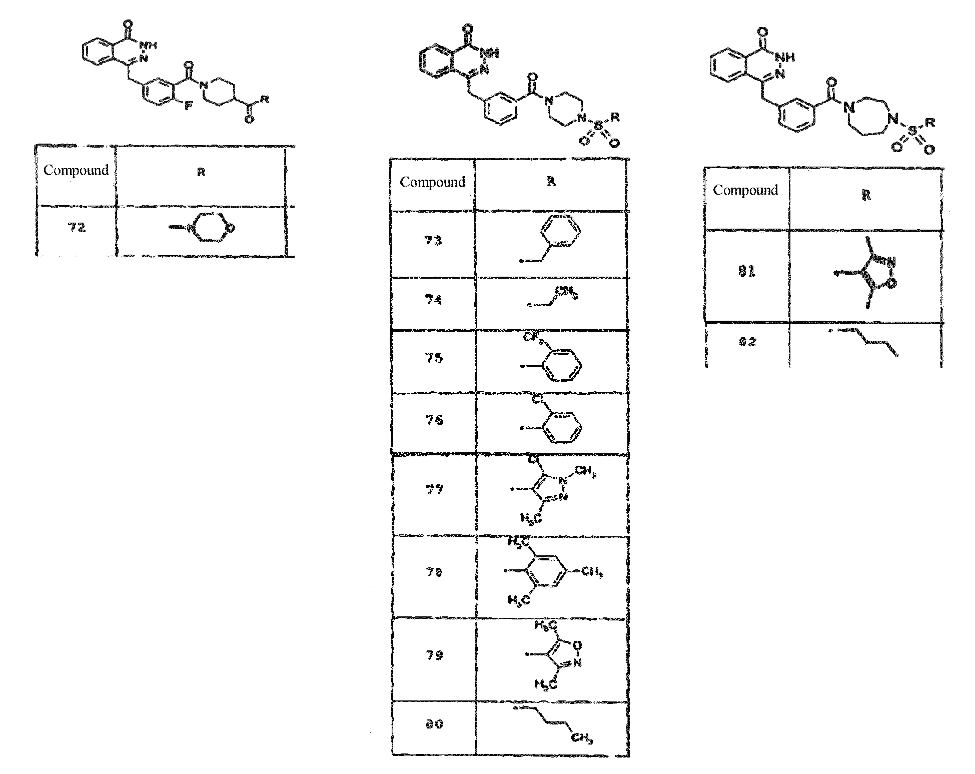 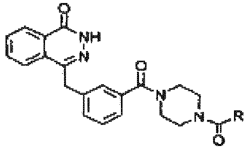 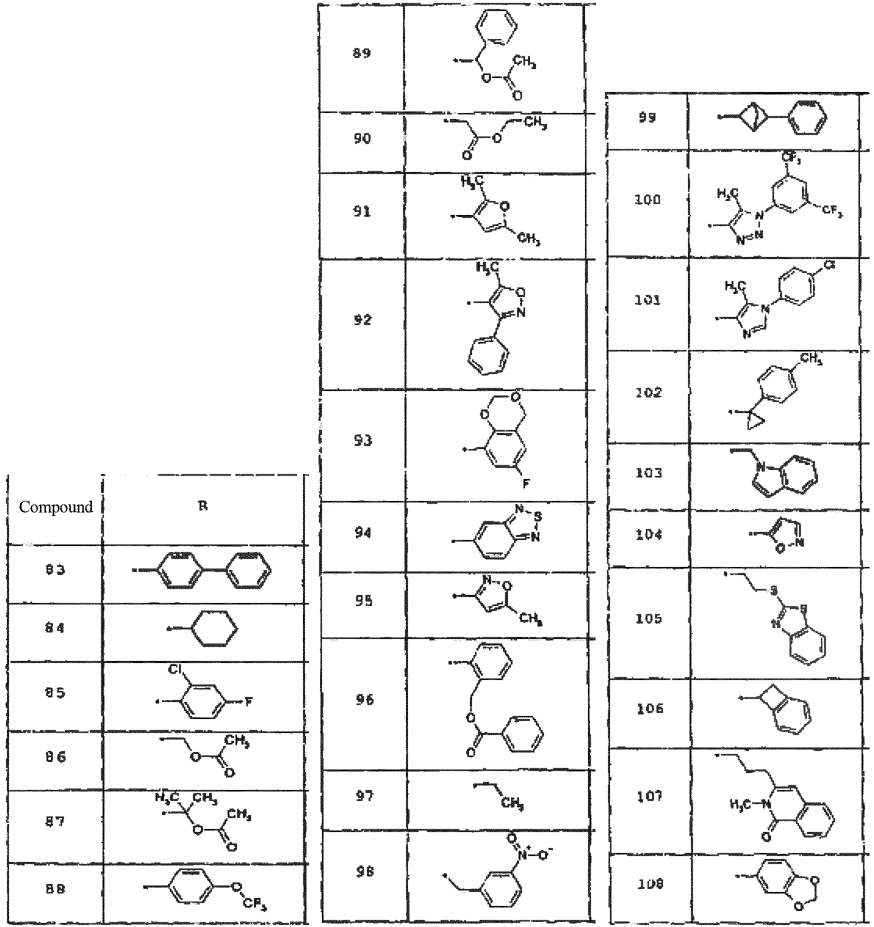 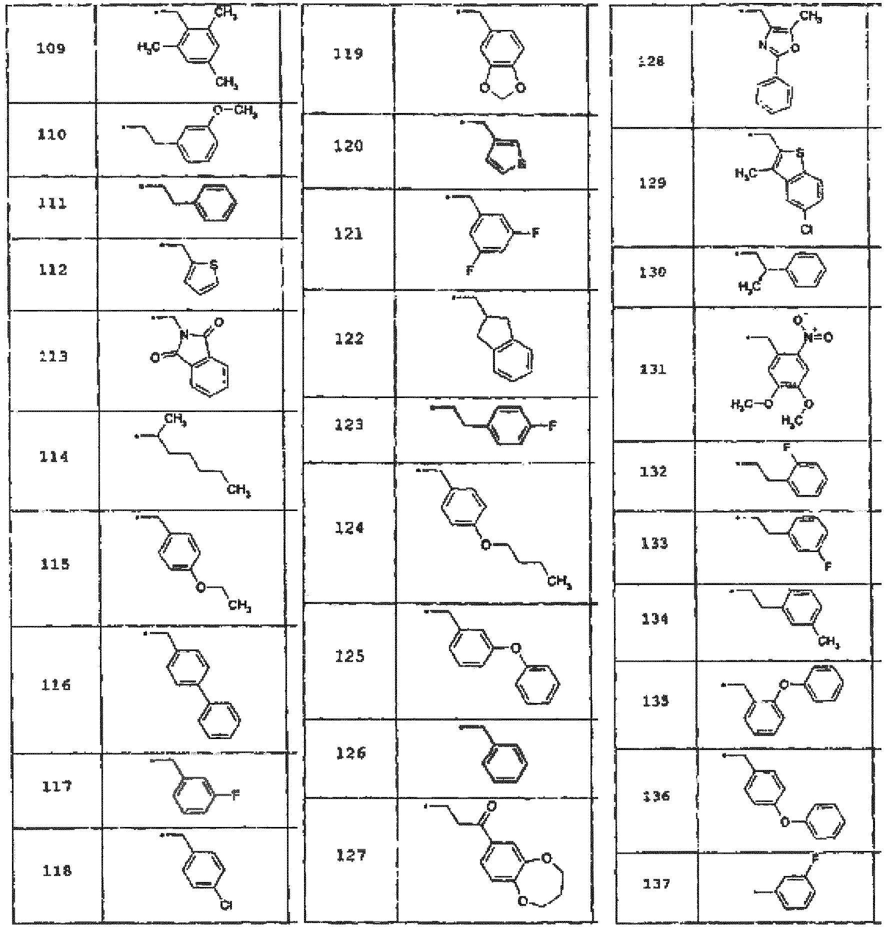 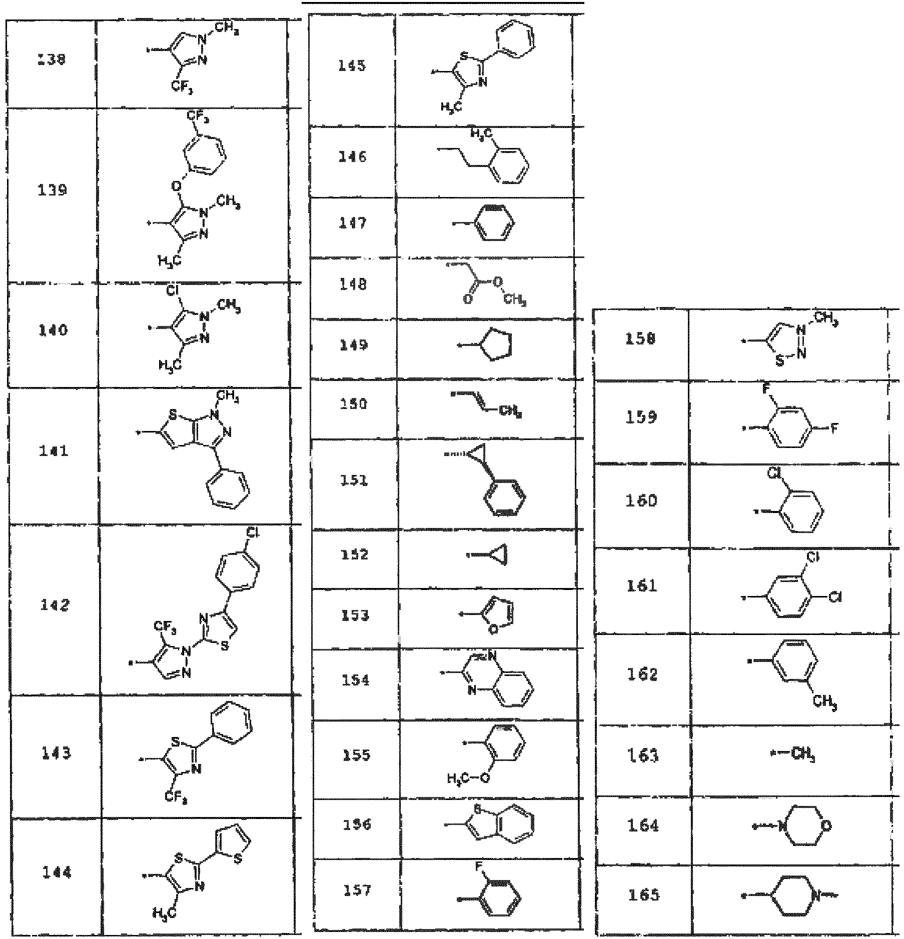 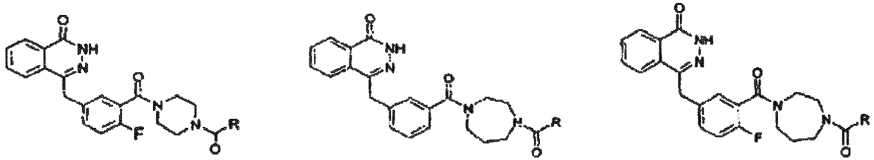 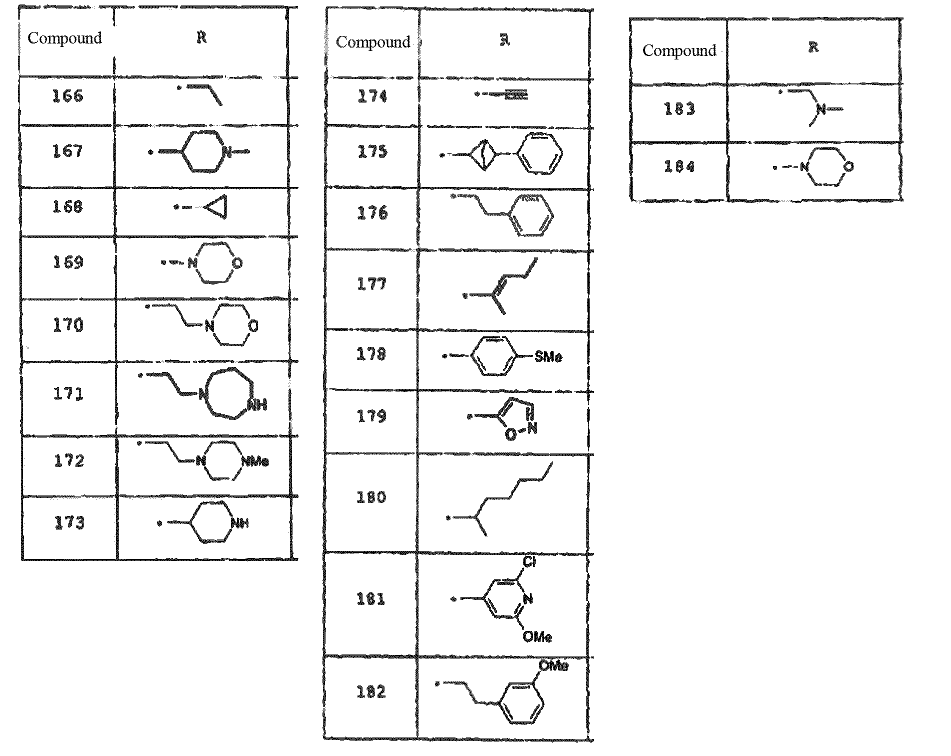 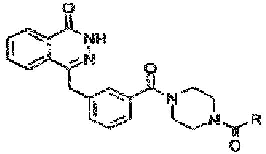 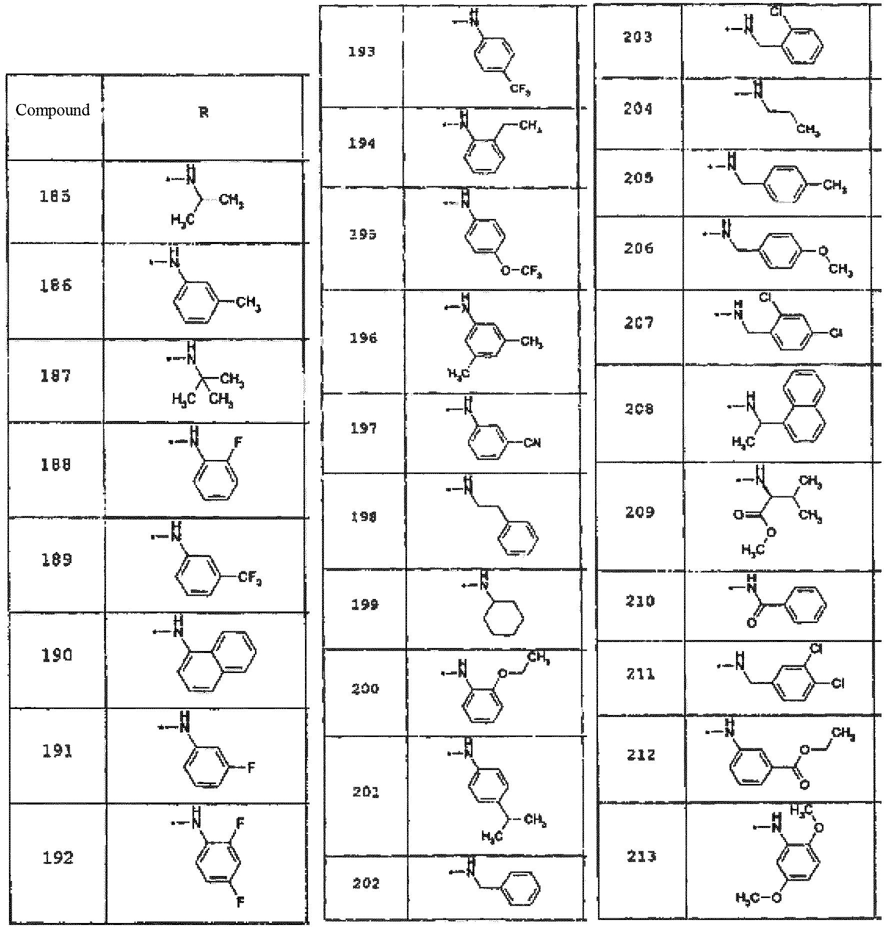 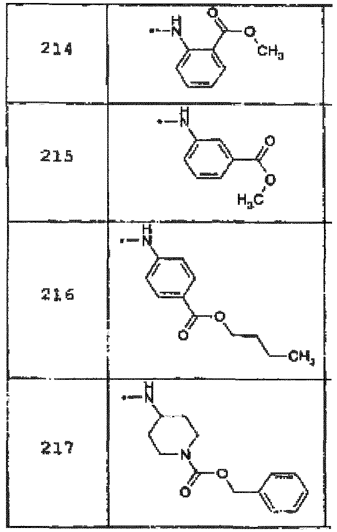 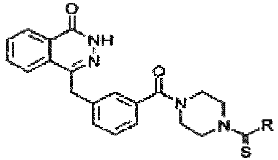 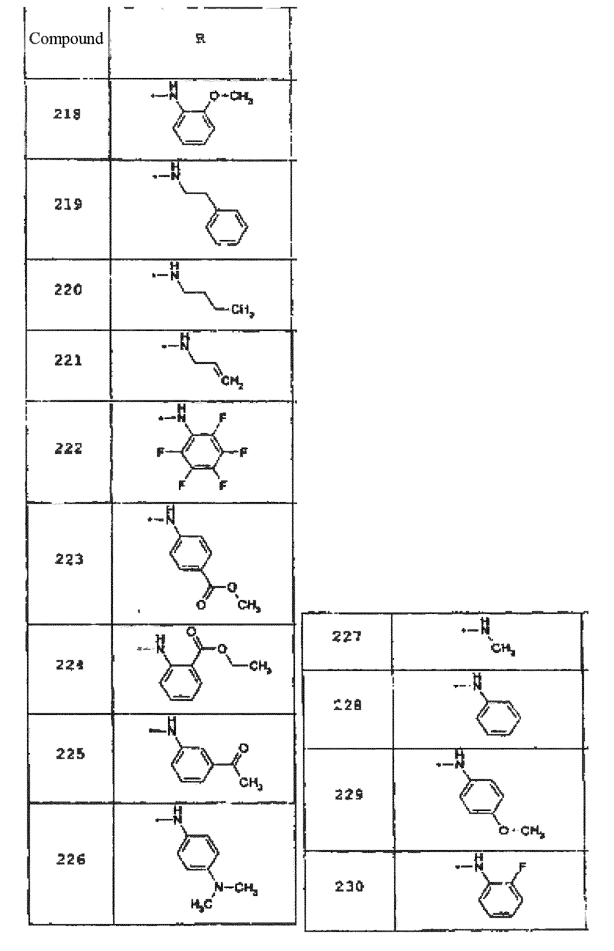 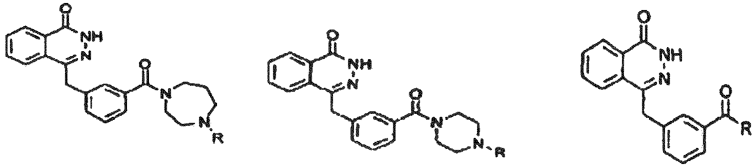 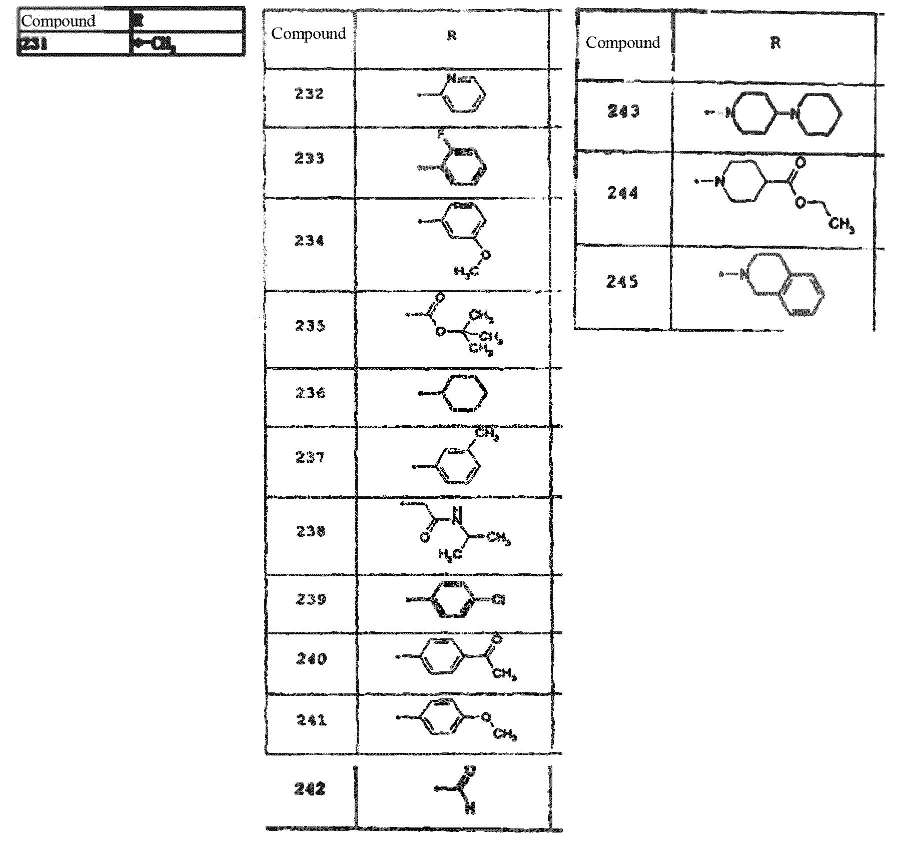 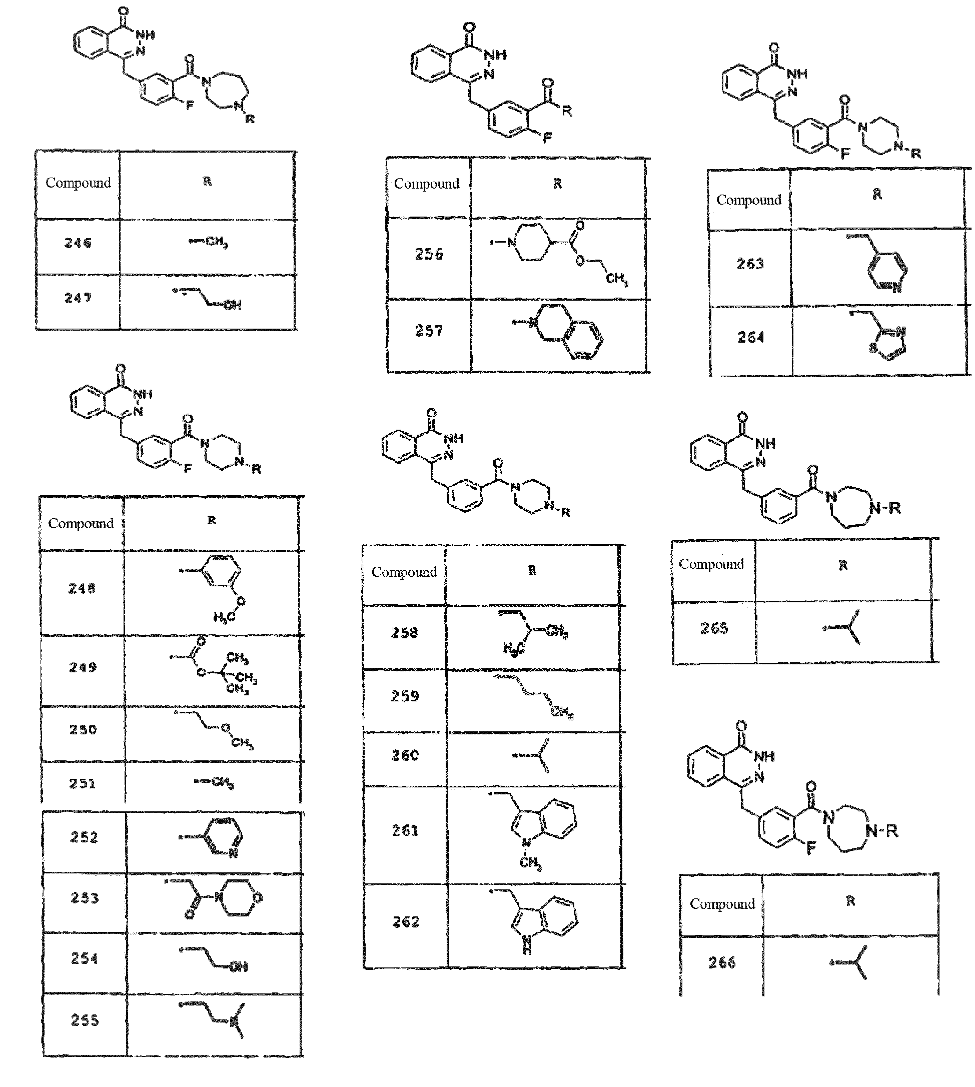
the compound shown in formula **III** is any of the following compounds: 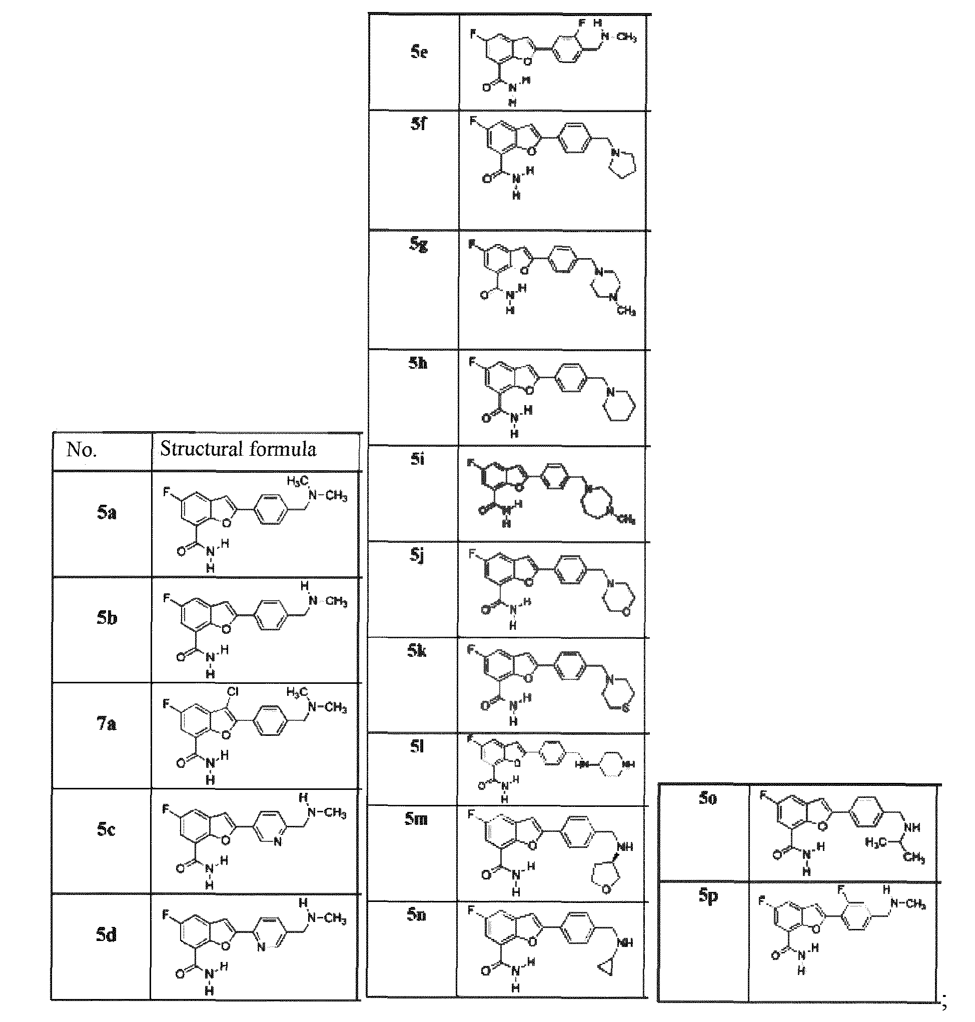
the compound shown in formula **IV** is any of the following compounds:
2-phenyl-2H-indazole-7-carboxamide;
2-(3-chlorophenyl)-2H-indazole-7-carboxamide; and
2-{4-[(dimethylamino)methyl]phenyl}-2H-indazole-7-carboxamide;
2-{4-[(N,N-dimethylglycyl)amino]phenyl}-2H-indazole-7-carboxamide;
2-benzyl-2H-indazole-7-carboxamide; 2-(4-chlorophenyl)-2H-indazole-7-carboxamide;
2-(2-chlorophenyl)-2H-indazole-7-carboxamide;
2-{4-[(4-methylpiperazin-1-yl)methyl]phenyl}-2H-indazole-7-carboxamide;
2-[4-(morpholine-4-ylmethyl)phenyl]-2H-indazole-7-carboxamide;
2-{4-[(methylamino)methyl]phenyl} -2H-indazole-7-carboxamide;
2-[4-(pyrrolidin-1-ylmethyl)phenyl]-2H-indazole-7-carboxamide;
2-[4-(piperidin-1-ylmethyl)phenyl]-2H-indazole-7-carboxamide;
{4-[7-(aminocarbonyl)-2H-indazole-2-yl]phenyl} -N,N-dimethylmethane ammonium chloride;
trifluoroacetate 4-[({4-[7-(aminocarbonyl)-2H-indazole-2-yl]benzoyl}amino)methyl]pyridinium;
2-{4-[1-(methylamino)ethyl]phenyl}-2H-indazole-7-carboxamide;
N-{4-[7-(aminocarbonyl)-2H-indazole-2-yl]benzyl} cyclohexanetrifluoroacetate;
{4-[7-(aminocarbonyl)-4-chloro-2H-indazole-2-yl]phenyl}-N-methylmethane ammonium trifluoroacetate; 2-phenyl-2H-1,2,3-benzotriazole-4-carboxamide;
2-benzyl-2H-1,2,3-benzotriazole-4-carboxamide;
2- {3-[(methylamino)methyl]phenyl} -2H-indazole-7-carboxamide; trifluoroacetate 4-({3-[7-(aminocarbonyl)-2H-indazole-2-yl]benzoyl}amino)piperidinium;
{4-[7-(aminocarbonyl)-2H-Indazole-2-yl]phenyl}-N-methylmethane ammonium chloride;
2-{3-chloro-4-[(dimethylamino)methyl]phenyl}-2H-indazole-7-carboxamide; trifluoroacetate 1-[2-({4-[7-(aminocarbonyl)-2H-indazole-2-yl]phenyl}amino)-2-oxoethyl]-4-methylpiperazine -1-onium;
2-(4-{[(4-pyrrolidine-1-ylpiperidin-1-yl)acetyl]amino}phenyl)-2H-indazole-7-carboxamide;
2-{4-[(pyrrolidin-1-ylacetyl)amino]phenyl}-2H-indazole-7-carboxamide;
2-{4-[(piperidin-1-ylacetyl)amino]phenyl}-2H-indazole-7-carboxamide;
2-{4-[(morpholin-4-ylacetyl)amino]phenyl}-2H-indazole-7-carboxamide; chloride 4-[2-({4-[7-(aminocarbonyl)-2H-indazole-2-yl]phenyl}amino)-2-oxoethyl]morpholine-4-oniu m; 2-{4-[(ethylamino)methyl]phenyl}-2H-indazole-7-carboxamide;
2-{4-[(isopropylamino)methyl]phenyl}-2H-indazole-7-carboxamide;
N-{4-[7-(aminocarbonyl)-2H-indazole-2-yl]benzyl}propane-2-ammonium chloride;
2-(4-{[(2-fluoroethyl)amino]methyl}phenyl)-2H-indazole-7-carboxamide;
2-(4-{[(2,2-difluoroethyl)amino]methyl}phenyl)-2H-indazole-7-carboxamide;
2-{4-[(cyclopropylamino)methyl]phenyl}-2H-Indazole-7-carboxamide;
4-{4-[7-(aminocarbonyl)-2H-indazole-2-yl]benzoyl}-1,4-diazabicycloheptane-1-onium;
2-({4-[7-(aminocarbonyl)-2H-indazole-2-yl]benzoyl}amino)-N,N-dimethylethane ammonium trifluoroacetate; trifluoroacetate 4-[({4-[7-(aminocarbonyl)-2H-indazole-2-yl]benzoyl}amino)methyl]piperidinium;
N-{4-[7-(aminocarbonyl)-2H-indazole-2-yl]benzyl}-N,N',N'-trimethylethane-1,2-diammonium dichloride; trifluoroacetate 4-{4-[7-(aminocarbonyl)-2H-indazole-2-yl]benzoyl}-1-methylpiperazine-1-onium;
trifluoroacetate 3-[({4-[7-(aminocarbonyl)-2H-indazole-2-yl]phenyl}amino)carbonyl]azacyclobutanium;
trifluoroacetate (2S)-2-[({4-[7-(aminocarbonyl)-2H-indazole-2-yl]phenyl}amino)carbonyl]-1-methylpyrrolidin ium; trifluoroacetate 3-[({4-[7-(aminocarbonyl)-2H-indazole-2-yl]phenyl}amino)carbonyl]-1-methylpiperidinium;
trifluoroacetate 4-[({4-[7-(aminocarbonyl)-2H-indazole-2-yl]phenyl}amino)carbonyl]-1-methylpiperidinium;
trifluoroacetate 4-({4-[7-(aminocarbonyl)-2H-indazole-2-yl]benzoyl}amino)-1-benzylpiperidinium;
2-{4-[(pyridin-4-ylamino)carbonyl]phenyl}-2H-indazole-7-carboxamide;
2-{4-[(4-phenylpiperazin-1-yl)carbonyl]phenyl}-2H-indazole-7-carboxamide;
2-(4-{[methyl(quinoxalin-6-ylmethyl)amino]carbonyl}phenyl)-2H-indazole-7-carboxamide;
2-(4-formylphenyl)-2H-indazole-7-carboxamide; trifluoroacetate 1-[2-({4-[7-(aminocarbonyl)-2H-indazole-2-yl]benzoyl}amino)ethyl]pyrrolidinium;
trifluoroacetate 1-[2-({4-[7-(aminocarbonyl)-2H-indazole-2-yl]benzoyl}amino)ethyl]piperidinium;
trifluoroacetate 4-[2-({4-[7-(aminocarbonyl)-2H-indazole-2-yl]benzoyl}amino)ethyl]morpholine-4-onium;
trifluoroacetate 4-({4-[7-(aminocarbonyl)-2H-indazole-2-yl]benzoyl}amino)-1-methylpiperidinium;
2-[4-[(4-methylpiperazin-1-yl)methyl]-3-(trifluoromethyl)phenyl]-2H-indazole-7-carboxamide;
2-[4-[(methylamino)methyl]-3-(trifluoromethyl)phenyl]-2H-indazole-7-carboxamide;
1-{4-[7-(aminocarbonyl)-2H-indazole-2-yl]phenyl}-N-methylethane ammonium chloride;
2-[4-(pyrrolidin-1-ylmethyl)-3-(trifluoromethyl)phenyl]-2H-indazole-7-carboxamide;
2-[4-(piperidin-1-ylmethyl)-3-(trifluoromethyl)phenyl]-2H-indazole-7-carboxamide;
2-[4-[(ethylamino)methyl]-3-(trifluoromethyl)phenyl]-2H-indazole-7-carboxamide;
trifluoroacetate 4-{4-[7-(aminocarbonyl)-4-chloro-2H-indazole-2-yl]benzyl}-1-methylpiperazine-1-onium;
bis(trifluoroacetic acid) 1-[2-({4-[7-(aminocarbonyl)-2H-indazole-2-yl]benzyl}ammonio)ethyl]piperidinium;
bis(trifluoroacetic acid) 4-[2-({4-[7-(aminocarbonyl)-2H-indazole-2-yl]benzyl}ammonio)ethyl]morpholine-4-onium;
bis(trifluoroacetic acid)1-[2-({4-[7-(aminocarbonyl)-2H-indazole-2-yl]benzyl}ammonio)ethyl]pyrrolidinium;
bis(trifluoroacetic acid) 4-({4-[7-(aminocarbonyl)-2H-indazole-2-yl]benzyl}ammonio)-1-methylpiperidinium;
bis(trifluoroacetic acid)4-({4-[7-(aminocarbonyl)-2H-indazole-2-yl]benzyl}ammonio)-1-benzylpiperidinium;
bis(trifluoroacetic acid)1-{4-[7-(aminocarbonyl)-2H-indazole-2-yl]benzyl}-4-benzylpiperidinium;
N- {4-[7-(aminocarbonyl)-2H-indazole-2-yl]benzyl} -2-(dimethylamino)-2-oxoethyl alkyltrifluoroacetate ammonium; bis(trifluoroacetic acid) 2-[({4-[7-(aminocarbonyl)-2H-indazole-2-yl]benzyl}ammonio)methyl]pyridinium;
bis(trifluoroacetic acid) 4-[({4-[7-(aminocarbonyl)-2H-indazole-2-yl]benzyl}ammonio)methyl]pyridinium;
N- {4-[7-(aminocarbonyl)-2H-indazole-2-yl]benzyl} -2-methylpropane-2-ammonium trifluoroacetate; bis(trifluoroacetic acid)N'-{4-[7-(aminocarbonyl)-2H-indazole-2-yl]benzyl}-N,N-dimethylethane-1,2-diammoniu m; {4-[7-(aminocarbonyl)-2H-indazole-2-yl]phenyl}-N-(1,3-oxazol-2-ylmethyl)methane ammonium trifluoroacetate; chloride 7-[7-(aminocarbonyl)-2H-indazole-2-yl]-1,2,3,4-tetrahydroisoquinolinium; chloride 6-[7-(aminocarbonyl)-2H-indazole-2-yl]-1,2,3,4-tetrahydroisoquinolinium; trifluoroacetate 5-[7-(aminocarbonyl)-2H-indazole-2-yl]-1,2,3,4-tetrahydroisoquinolinium; trifluoroacetate 3-[({4-[7-(aminocarbonyl)-5-fluoro-2H-indazole-2-yl]phenyl}amino)carbonyl]azacyclobutaniu m; 2-(4-{[(azetidin-3-ylcarbonyl)(methyl)amino]methyl}phenyl)-2H-indazole-7-carboxamide;
trifluoroacetate 3-[({4-[7-(aminocarbonyl)-2H-indazole-2-yl]benzyl}amino)carbonyl]azacyclobutanium;
2-(4-bromophenyl)-5-fluoro-2H-indazole-7-carboxamide;
5-fluoro-2-(4-pyridine-3-ylphenyl)-2H-indazole-7-carboxamide;
2-(4-pyridin-3-ylphenyl)-2H-indazole-7-carboxamide; trifluoroacetate 4-{4-[7-(aminocarbonyl)-2H-indazole-2-yl]phenyl}-1-methylpiperazine-1-onium;
trifluoroacetate 4- {4-[7-(aminocarbonyl)-2H-indazole-2-yl]phenyl} piperidinium;
2- {4-[7-(aminocarbonyl)-2H-indazole-2-yl]phenyl} -N-methylethane ammonium trifluoroacetate; trifluoroacetate 2-[({4-[7-(aminocarbonyl)-2H-indazole-2-yl]phenyl}amino)carbonyl]azacyclobutanium;
2-{5-[(methylamino)methyl]pyridin-2-yl} -2H-indazole-7-carboxamide;
5-fluoro-2-{3-fluoro-4-[(methylamino)methyl]phenyl}-2H-indazole-7-carboxamide;
5-fluoro-2- {4-[(methylamino)methyl]phenyl} -2H-indazole-7-carboxamide trifluoroacetate;
2-{4-[7-(aminocarbonyl)-2H-indazole-2-yl]phenyl}-N-methylpropane-2-ammonium trifluoroacetate; 2-(6-phenylpyridazine-3-yl)-2H-indazole-7-carboxamide;
{4-[7-(aminocarbonyl)-5-fluoro-2H-indazole-2-yl]phenyl}-N-{[1-(hydroxylmethyl)cyclohexyl ]methyl}methane ammonium trifluoroacetate;
5-chloro-2-(4-formylphenyl)-2H-indazole-7-carboxamide;
2-{3-methoxy-4-[(4-methylpiperazin-1-yl)methyl]phenyl}-2H-indazole-7-carboxamide;
2-{3-methoxy-4-[(methylamino)methyl]phenyl}-2H-indazole-7-carboxamide;
5-chloro-2-{4-[(4-methylpiperazin-1-yl)methyl]phenyl}-2H-indazole-7-carboxamide;
5-chloro-2-{4-[(methylamino)methyl]phenyl}-2H-indazole-7-carboxamide;
{4-[7-(aminocarbonyl)-4-fluoro-2H-indazole-2-yl]phenyl}-N-methylmethane ammonium chloride; {4-[7-(aminocarbonyl)-5-fluoro-2H- indazole-2-yl]phenyl} -N-methylmethane ammonium chloride; chloride 1-{4-[7-(aminocarbonyl)-4-fluoro-2H-indazole-2-yl]benzyl}-4-methylpiperazine-1-onium;
chloride 1-{4-[7-(aminocarbonyl)-5-fluoro-2H-indazole-2-yl]benzyl} -4-methylpiperazine-1-onium; bis(trifluoroacetic acid) 1-{3-[7-(aminocarbonyl)-2H-indazole-2-yl]benzyl} -4-methylpiperazinium; 2-[4-(1 -hydroxy-1 -methylethyl)phenyl]-2H-indazole-7-carboxamide;
2-(4-acetylphenyl)-2H-indazole-7-carboxamide; trifluoroacetate 3-{[{4-[7-(aminocarbonyl)-2H-indazole-2-yl]benzyl}(methyl)amino]carbonyl}-1-methylpiperi dinium; 2-{4-[1-(formylamino)-1-methylylethyl]phenyl}-2H-indazole-7-carboxamide;
2-[3-(1,4-diazabicycloheptane-1-ylcarbonyl)phenyl] -2H-indazole-7-carboxamide;
trifluoroacetate 3-[({4-[7-(aminocarbonyl)-2H-indazole-2-yl]benzyl}amino)carbonyl]-1-methylpiperidinium;
trifluoroacetate (2S)-2-[({4-[7-(aminocarbonyl)-2H-indazole-2-yl]phenyl}amino)carbonyl]pyrrolidinium;
trifluoroacetate 3-[({4- [7-(aminocarbonyl)-2H-indazole-2-yl]phenyl}amino)carbonyl]pyrrolidinium;
trifluoroacetate (2R)-2-[({4-[7-(aminocarbonyl)-2H-indazole-2-yl]phenyl}amino)carbonyl]pyrrolidinium;
trifluoroacetate 3-[({4- [7-(aminocarbonyl)-2H-indazole-2-yl]phenyl}amino)carbonyl]piperidinium;
trifluoroacetate (3R)-3-[({4-[7-(aminocarbonyl)-2H-indazole-2-yl]phenyl}amino)carbonyl]-1-methylpyrrolidin ium; trifluoroacetate
2-[({4-[7-(aminocarbonyl)-2H-indazole-2-yl]phenyl}amino)carbonyl]-1-methylpiperidinium; 4-chloro-2-(4-formylphenyl)-2H-indazole-7-carboxamide; trifluoroacetate 3S)-3-[({4-[7-(aminocarbonyl)-2H-indazole-2-yl]phenyl}amino)carbonyl]-1-methylpyrrolidin ium; (R)-1-{4-[7-(aminocarbonyl)-2H-indazole-2-yl]phenyl}-N-methylethane ammonium chloride; (S)-1-{4-[7-(aminocarbonyl)-2H-indazole-2-yl ]Phenyl}-N-methylethaneammonium chloride; 2-{3-fluoro-4-[(methylamino)methyl]phenyl}-2H-indazole-7-carboxamide; {4-[7-(aminocarbonyl)-2H-indazole-2-yl]-2-fluorophenyl}-N-methane ammonium trifluoroacetate; 2- {4-[1-methyl-1-(methylamino)ethyl]phenyl} -2H-indazole-7-carboxamide;
trifluoroacetate 1-{4-[7-(aminocarbonyl)-2H-indazole-2-yl]-2-hydroxybenzyl}-4-methylpiperazine-1-onium;
chloride (3R)-3-[({4-[7-(aminocarbonyl)-2H-indazole-2-yl]phenyl}amino)carbonyl]-1-methylpiperidini um; chloride (3S)-3-[({4-[7-(aminocarbonyl)-2H-indazole-2-yl]phenyl}amino)carbonyl]-1-methyl chloride piperidinium; bis(trifluoroacetic acid)1-(2-{4-[7-(aminocarbonyl)-2H-indazole-2-yl]phenyl}ethyl)-4-methylpiperazinedinium;
{4-[7-(aminocarbonyl)-4-hydroxy-2H-indazole-2-yl]phenyl}-N-ammonium methylmethane trifluoroacetate; trifluoroacetate 2-[({4-[7-(aminocarbonyl)-2H-indazole-2-yl]phenyl}amino)carbonyl]-4-phenylpyrrolidinium;
(1R,3S)-3-[({4-[7-(aminocarbonyl)-2H-indazole-2-yl]phenyl}amino)carbonyl]cyclopentane ammonium trifluoroacetate; (1R,3R)-3-[({4-[7-(aminocarbonyl)-2H-indazole-2-yl]phenyl}amino)carbonyl]cyclopentane ammonium trifluoroacetate;
(1S,3R)-3-[({4-[7-(aminocarbonyl)-2H-indazole-2-yl]phenyl}amino)carbonyl]cyclopentane ammonium trifluoroacetate; trifluoroacetate 2-[({4-[7-(aminocarbonyl)-2H-indazole-2-yl]phenyl}amino)carbonyl]-2-methylazetidinium;
trifluoroacetate 4-[2-({4-[7-(aminocarbonyl)-2H-indazole-2-yl]phenyl}amino)-2-oxoethyl]-1-methylpiperidini um;
9-[2-({4-[7-(aminocarbonyl)-2H-indazole-2-yl]phenyl}amino)-2-oxoethyl]-3-azaspiro[5.5]und ecane trifluoroacetate; trifluoroacetate 4-[2-({4-[7-(aminocarbonyl)-2H-indazole-2-yl]phenyl}amino)-2-oxoethyl]-4-phenylpiperidini um; trifluoroacetate 2-[({4-[7-(aminocarbonyl)-2H-indazole-2-yl]phenyl}amino)carbonyl]pyridinium;
trifluoroacetate 4-{3-[({4-[7-(aminocarbonyl)-2H-indazole-2-yl]phenyl}amino)carbonyl]pyridin-2-yl}piperazi ne-1-onium; trifluoroacetate
3-[({4-[7-(aminocarbonyl)-2H-indazole-2-yl]phenyl}amino)carbonyl]pyridinium;
trifluoroacetate 4-[({4-[7-(aminocarbonyl)-2H-indazole-2-yl]phenyl}amino)carbonyl]pyridinium;
trifluoroacetate 4-[({4-[7-(aminocarbonyl)-2H-indazole-2-yl]phenyl}amino)carbonyl]quinolinium;
trifluoroacetate 4-[({4-[7-(aminocarbonyl)-2H-indazole-2-yl]phenyl}amino)carbonyl]isoquinolinium;
trifluoroacetate 2-[({4-[7-(aminocarbonyl)-2H-indazole-2-yl]phenyl}amino)carbonyl]-1-methylazepanium;
trifluoroacetate 3-[({4-[7-(aminocarbonyl)-2H-indazole-2-yl]phenyl}amino)carbonyl]-2-methyl-1,2,3,4-tetrahy droisoquinolinium; trifluoroacetate 2-{4-[({4-[7-(aminocarbonyl)-2H-indazole-2-yl]phenyl}amino)carbonyl]piperidin-1-yl}pyrimi dine-1-onium; chloride 1-{4-[7-(aminocarbonyl)-5-fluoro-2H-indazole-2-yl]benzyl}-4-methylpiperazine-1-onium;
5-[({4-[7-(aminocarbonyl)-2H-indazole-2-yl]phenyl}amino)carbonyl]-3-oxoswainsonine-2-ammoniu m trifluoroacetate; trifluoroacetate 2-{3-({4-[7-(aminocarbonyl)-2H-indazole-2-yl]phenyl}amino)carbonyl]piperidin-1-yl}pyridini um; trifluoroacetate 2-[({4-[7-(aminocarbonyl)-2H-indazole-2-yl]phenyl}amino)carbonyl]-4-methylmorpholin-4-o nium;
(1R,4R)-N-{4-[7-(aminocarbonyl)-2H-indazole-2-yl]phenyl}-1'-(methylsulfonyl)-1',2'-dihydro spiro[cyclohexane-1,3'-indole]-4-carboxamide; trifluoroacetate 1-[({4-[7-(aminocarbonyl)-2H-indazole-2-yl]phenyl}amino)carbonyl]octahydro-1H-isoindoliu m; trifluoroacetate 2-[({4-[7-(aminocarbonyl)-2H-indazole-2-yl]phenyl}amino)carbonyl]-4-benzylmorpholine-4-o nium; trifluoroacetate (3S, 4R)-3-[({4-[7-(aminocarbonyl)-2H-indazole-2-yl]phenyl}amino)carbonyl]-4-(methoxycarbony l)pyrrolidinium; bis(trifluoroacetic acid) 4-{(2S)-2-[({4-[7-(aminocarbonyl)-2H-indazole-2-yl]phenyl}amino)carbonyl]pyrrolidinium-1-yl}piperidinium;
(1S,3S)-3-[({4-[7-(aminocarbonyl)-2H-indazole-2-yl]phenyl}amino)carbonyl]cyclopentane ammonium trifluoroacetate; trifluoroacetate 3-[({4-[7-(aminocarbonyl)-2H-indazole-2-yl]phenyl}amino)carbonyl]-1-methylpyrrolidinium;
trifluoroacetate 2-{4-[({4-[7-(aminocarbonyl)-2H-indazole-2-yl]phenyl}amino)carbonyl]piperidin-1-yl}pyrimi dine-1-onium; bis(trifluoroacetic acid)2-(1-{4-[7-(aminocarbonyl)-2H-indazole-2-yl]benzyl}pyrrolidinium-3-yl)pyridinium;
bis(trifluoroacetic acid) 3-(1-{4-[7-(aminocarbonyl)-2H-indazole-2-yl]benzyl}pyrrolidinium-3-yl)pyridinium;
trifluoroacetate (3S,4S)-1-{4-[7-(amino carbonyl)-2H-indazole-2-yl]benzyl}-3,4-difluoropyrrolidinium;
3- {4-[7-(aminocarbonyl)-2H-indazole-2-yl]benzyl} -6-amino-3-azabicyclo[3.1.0]hexanebis(trif luoroacetate); 2-{4-[7-(aminocarbonyl)-2H-indazole-2-yl ]benzyl}-7-methyl-2,7-diazonium heterospiro[4.4]nonane bis(trifluoroacetate); bis(trifluoroacetic acid) 1-{4-[7-(aminocarbonyl)-2H-indazole-2-yl]benzyl}-3-[4-(dimethylammonio)phenyl]pyrrolidin ium; bis(trifluoroacetic acid)5-{4-[7-(aminocarbonyl)-2H-indazole-2-yl]benzyl}-1-methyl-1,2,4,5,6,6a-hexahydropyrr olo[3,4-b]pyrrolidinium; bis(trifluoroacetic acid) 3-{[{4-[7-(aminocarbonyl)-2H-indazole-2-yl]benzyl}(methyl)amino]methyl}-1-methylpiperidi nium;
(1R,4S)-5-{4-[7-(aminocarbonyl)-2H-indazole-2-yl]benzyl}-2-oxa-5-azoniabicyclo[2.2.1]hept ane trifluoroacetate;
N-{4-[7-(aminocarbonyl)-2H-indazole-2-yl]benzyl}-2-hydroxy-2-methylpropane-1-ammoniu m trifluoroacetate;
N- {4-[7-(aminocarbonyl)-2H-indazole-2-yl]benzyl} -3,3-difluorocyclobutane ammonium trifluoroacetate; trifluoroacetate 4-{4-[7-(aminocarbonyl)-2H-indazole-2-yl]benzyl}-6-fluoro-1,4-diazabicycloheptane-1-onium; bis(trifluoroacetic acid) 1-{4-[7-(aminocarbonyl)-2H-indazole-2-yl]benzyl}-4-pyrimidin-1-onium-2-yl-1,4-diazabicycl oheptane-1-onium; bis(trifluoroacetic acid) 3-({4-[7-(aminocarbonyl)-2H-indazole-2-yl]benzyl}ammonio)-1-benzylpyrrolidinium; bis(trifluoroacetic acid) 3-[({4-[7-(aminocarbonyl)-2H-indazole-2-yl]benzyl}ammonio)methyl]-1-methylpyrrolidinium; bis(trifluoroacetic acid) 3-[({4- [7-(aminocarbonyl)-2H-indazole-2-yl]benzyl}ammonio)methyl]-1-benzylpyrrolidinium;
2-{4-[7-(aminocarbonyl)-2H-indazole-2-yl]benzyl}-7-benzyl-2,7-diazaspiro[4.4]nonanebis(trif luoroacetate); 2- {4-[7-(aminocarbonyl)-2H-indazole-2-yl]benzyl} -8-benzyl-2,8-diazonium heterospiro[5.5]undecanbis(trifluoroacetate);
2- {4-[7-(aminocarbonyl)-2H-indazole-2-yl]benzyl} -2,6-diazonium heterospiro[3.3]heptanebis(trifluoroacetate);
7- {4-[7-(aminocarbonyl)-2H-indazole-2-yl]benzyl} -2,7-diazonium heterospiro[3.5]nonanebis(trifluoroacetate);
2- {4-[7-(aminocarbonyl)-2H-indazole-2-yl]benzyl} -2,6-diazonium heterospiro[3.5]nonanebis(trifluoroacetate);
2- {4-[7-(aminocarbonyl)-2H-indazole-2-yl]benzyl} -2,8-diazonium heterospiro[5.5]undecanbis(trifluoroacetate);
2- {4-[7-(aminocarbonyl)-2H-indazole-2-yl]benzyl} -2,8-diazonium heterospiro[4.5]decanebis(trifluoroacetate);
2- {4-[7-(aminocarbonyl)-2H-indazole-2-yl]benzyl} -2,7-diazonium heterospiro[4.5]decanebis(trifluoroacetate);
8- {4-[7-(aminocarbonyl)-2H-indazole-2-yl]benzyl} -2,8-diazonium heterospiro[4.5]decanebis(trifluoroacetate);
3- {4-[7-(aminocarbonyl)-2H-indazole-2-yl]benzyl} -3,9-diazonium heterospiro[5.5]undecanbis(trifluoroacetate); bis(trifluoroacetic acid) 2-{4-[7-(aminocarbonyl)-2H-indazole-2-yl]benzyl}octahydropyrrolo[3,4-c]pyrrolidinium;
bis(trifluoro acetic acid) 5-{4-[7-(aminocarbonyl)-2H-indazole-2-yl]benzyl}octahydropyrrolo[3,4-b]pyrrolidinium; bis(trifluoroacetic acid) 4-({4-[7-(Aminocarbonyl)-2H-indazole-2-yl]benzyl}ammonio)octahydrocyclopentadieno[c]py rrolidinium; N²-{4-[7-(Amino) carbonyl)-2H-indazole-2-yl]benzyl}-N¹,N¹-dimethyl-1-pyridin-2-ylethane-1,2-bis(trifluoroacet ic acid)diammonium; bis(trifluoroacetic acid) 7-(aminocarbonyl)-2-[4-({[2-(2,3-dihydro-1H-indol-1-yl)ethyl]ammonium}methyl)phenyl]-2H -indazole-1-onium; bis(trifluoroacetic acid)(3S,4S)-1-[2-({4-[7-(aminocarbonyl)-2H-indazole-2-yl]benzyl}ammonio)ethyl]-3,4-diflu oropyrrolidinium; trifluoroacetate 5-({4-[7-(aminocarbonyl)-2H-indazole-2-yl]benzyl}amino)-1,3-benzothiazole-3-onium;
1-({4-[7-(aminocarbonyl)-2H-indazole-2-yl]benzyl}ammonio)-8-diazonium heterospiro[4.5]decanebis(trifluoroacetate); bis(trifluoroacetic acid) 4-[({4-[7-(aminocarbonyl)-2H-indazole-2-yl]benzyl}ammonio)methyl]-1-methylpiperidinium;
N- {4-[7-(aminocarbonyl)-2H-indazole-2-yl]benzyl} -2-hydroxyethane ammonium trifluoroacetate; trifluoroacetate 7-[7-(aminocarbonyl)-2H-indazole-2-yl]-1,2,3,4-tetrahydroisoquinolinium; trifluoroacetate 3-[2-({4-[7-(aminocarbonyl)-2H-indazole-2-yl]phenyl}amino)-2-oxoethyl]azacyclobutanium;
bis(trifluoroacetic acid)4-({4-[7-(aminocarbonyl)-2H-indazole-2-yl]benzyl}ammonio)piperidinium;
bis(trifluoroacetic acid)(3R,4R)-4-({4-[7-(aminocarbonyl)-2H-indazole-2-yl]benzyl}ammonio)-3-fluoropiperidin ium; bis(trifluoroacetic acid)(3S,4R)-4-({4-[7-(aminocarbonyl)-2H-indazole-2-yl] benzyl} ammonio)-3 -benzyl-1-methylpiperidinium;
N- {4-[7-(aminocarbonyl)-2H-indazole-2-yl]benzyl} -1 -isobutylpiperidine-4-ammonium trifluoroacetate; bis(trifluoroacetic acid) 2-[4-({4-[7-(aminocarbonyl)-2H-indazole-2-yl]benzyl}ammonium)piperidine-1-yl]-3-methylp yridinium; bis(trifluoroacetic acid) 3-({4-[7-(aminocarbonyl)-2H-indazole-2-yl]benzyl}ammonio) pyridinium; bis(trifluoroacetic acid) 3-({4-[7-(aminocarbonyl)-2H-indazole-2-yl]benzyl}ammonio)-1-benzylpiperidinium;
5- {4-[7-(aminocarbonyl)-2H-indazole-2-yl]benzyl} -5-aza-2-azonium heterobicyclo[2.2.2]octane trifluoroacetate;
(1S,4S)-2- {4-[7-(aminocarbonyl)-2H-indazole-2-yl]benzyl} -5-methyl-2,5-diazonium heterobicyclo[2.2.1]heptane bis(trifluoroacetate); bis(trifluoroacetic acid) 1-{4-[7-(aminocarbonyl)-2H-indazole-2-yl]benzyl}-4-(pyridin-2-ylmethyl base) dinium piperazine; 5- {4-[7-(aminocarbonyl)-2H-indazole-2-yl]benzyl} -2-benzyl-5-aza-2-azonium heterobicyclo[2.2. 2] octane trifluoroacetate;
8-{4-[7-(aminocarbonyl)-2H-indazole-2-yl]benzyl}-3-benzyl-8-aza-3-azonium heterobicyclo[3.2.1]octane trifluoroacetate;
(1S,4S)-5- {4-[7-(aminocarbonyl)-2H-indazole-2-yl]benzyl} -2-benzyl-5-aza-2-azonium heterobicyclo[2.2.1]heptane trifluoroacetate; bis(trifluoroacetic acid)3-({4-[7-(aminocarbonyl)-2H-indazole-2-yl]benzyl}ammonio)pyrrolidinium;
6-({4-[7-(aminocarbonyl)-2H-indazole-2-yl]benzyl} ammonio)-3-diazonium heterobicyclo[3.1.0]hexanebis(trifluoroacetate); trifluoroacetate (3 S,4S)-N- {4-[7-(aminocarbonyl)-2H-indazole-2-yl]benzyl} -4-hydroxytetrahydrothiophene-3-ammonium 1,1-dioxide; bis(trifluoroacetic acid) 4-[({4-[7-(aminocarbonyl)-2H-indazole-2-yl] benzyl}ammonio)methyl]-4-hydroxy-1-methylpiperidinium;
N-{4-[7-(aminocarbonyl)-2H-indazole-2-yl]benzyl}-1-cyclopropyl-2-hydroxyethane ammonium trifluoroacetate;
{4-[7-(aminocarbonyl)-2H-indazole-2-yl]phenyl}-N-{[1-(hydroxymethyl)cyclopentyl]methyl} methane trifluoroacetate; bis(trifluoroacetic acid) 2-{4-[7-(aminocarbonyl)-2H-indazole-2-yl]benzyl}-1,2,3, 4-tetrahydro-2,7-diazanaphthalene;
bis(trifluoroacetic acid) 1-{4-[7-(aminocarbonyl)-2H-indazole-2-yl]benzyl} -3-[(dimethylammonio)methyl]piperidiniu m; bis(trifluoroacetic acid)4-(1-{4-[7-(aminocarbonyl)-2H-indazole-2-yl]benzyl}piperidinium-4-yl)thiomorpholine-4-onium; trifluoroacetate 1-{4-[7-(aminocarbonyl)-2H-indazole-2-yl]benzyl} -4-[(methylsulfonyl)amino]piperidinium;
bis(trifluoroacetic acid)1-{4-[7-(aminocarbonyl)-2H-indazole-2-yl]benzyl}-4-(1H-imidazol-3-onium-1-ylmethyl) piperidinium; 7-{4-[7-(aminocarbonyl)-2H-indazole-2-yl]benzyl}-1-oxa-7-aziumhespiro [4.5] decane trifluoroacetate; trifluoroacetate 1-{4-[7-(aminocarbonyl)-2H-indazole-2-yl]benzyl}-4-(1-hydroxy-1-methylethyl) piperidinium; trifluoroacetate 2-[({4-[7-(aminocarbonyl)-2H-indazole-2-yl]phenyl}amino)carbonyl]-1-benzylpiperidinium;
trifluoroacetate 2-[({4-[7-(aminocarbonyl)-2H-indazole-2-yl]phenyl}amino)carbonyl]-1-ethylpiperidinium;
trifluoroacetate 3-[({4-[7-(aminocarbonyl)-2H-indazole-2-yl]phenyl}amino)carbonyl]-1-ethylpiperidinium;
2-[3-(1,4-diazabicycloheptane-1-ylcarbonyl)-4-fluorophenyl]-2H-indazole-7-carboxamide trifluoroacetate; {4-[7-(aminocarbonyl)-4-chloro-2H-indazole-2-yl]benzyl}methylcarbamate tert-butyl ester; trifluoroacetate 6-[7-(aminocarbonyl)-2H-indazole-2-yl]-1,2,3,4-tetrahydroisoquinolinium; trifluoroacetate 2- {4-[7-(aminocarbonyl)-2H-indazole-2-yl]phenyl} pyrrolidinium;
6-fluoro-2-{4-[(methylamino)methyl]phenyl}-2H-indazole-7-carboxamide;
5-fluoro-2-{2-fluoro-4-[(methylamino)methyl]phenyl}-2H-indazole-7-carboxamide;
2- {3-hydroxy-4-[(methylamino)methyl]phenyl} -2H-indazole-7-carboxamide trifluoroacetate;
2-(4-{[formyl(methyl)amino]methyl}-3-hydroxyphenyl)-2H-indazole-7-carboxamide;
2- {2-chloro-4-[(methylamino)methyl]benzene yl} -5-fluoro-2H-indazole-7-carboxamide;
5-fluoro-2- {3-fluoro-4-[(methylamino)methyl]phenyl} -2H-indazole-7- carboxamide trifluoroacetate;
2-{2,5-difluoro-4-[(methylamino)methyl]phenyl}-5-fluoro-2H-indazole-7-carboxamide trifluoroacetate; 2-(4-bromophenyl)-2H-indazole-7-carboxamide; chloride (3R)-3-[({4-[7-(aminocarbonyl)-5-fluoro-2H-indazole-2-yl]phenyl}amino)carbonyl]-1-methyl piperidinium; trifluoroacetate (3R)-3-[({4-[7-(aminocarbonyl)-5-fluoro-2H-indazole-2-yl]phenyl}amino)carbonyl]-1-methyl piperidinium; 2-(1,2,3,4-tetrahydroisoquinolin-7-yl)-2H-indazole-7-carboxamide;
(R)-2-[4-({3-[(dimethylamin)methyl]piperidm-1-yl}methyl)phenyl]-2H-indazole-7-carboxam ide;
(S)-2-[4-({3-[(dimethylamino)methyl]piperidin-1-yl}methyl)phenyl]-2H-indazole-7-carboxami de;
3-({4-[7-(aminocarbonyl)-5-fluoro-2H-indazole-2-yl]phenyl}amino)-2-(chloromethyl)-3-oxopr opane-1-ammonium trifluoroacetate;
5-fluoro-2-{3-fluoro-4-[(methylamino)methyl]phenyl}-2H-indazole-7-carboxamide hydrochloride;
2- {4-[(dimethylamino)methyl]-3-fluorophenyl} -5-fluoro-2H-indazole-7-carboxamide trifluoroacetate;
2-{4-[(azacyclobutane-3-ylcarbonyl)amino]phenyl}-5-fluoro-2H-indazole-7-carboxamide;
2-[4-(2,7-diazaspiro[4.5]dec-2-ylmethyl)phenyl]-2H-indazole-7-carboxamide;
(1S,4S)-5- {4-[7-(aminocarbonyl)-2H-indazole-2-yl]benzyl} -2-(4-chlorobenzyl)-5-aza-2-azoniu m heterobicyclo[2.2.1]heptane trifluoroacetate;
(1S,4S)-5- {4-[7-(aminocarbonyl)-2H-indazole-2-yl]benzyl} -2-(3-chlorobenzyl)-5-aza-2-azonium heterobicyclo[2.2.1]heptane trifluoroacetate;
trifluoroacetate 1-{4-[7-(aminocarbonyl)-2H-indazole-2-yl]benzyl}-4-[(methylamino)carbonyl]piperazine-1-o nium; N-{4-[7-(aminocarbonyl)-2H-indazole-2-yl]benzyl}-2-hydroxy-2-pyridin-3-ylethane ammonium trifluoroacetate;
N-{4-[7-(aminocarbonyl)-2H-indazole-2-yl]benzyl}-2-cyclohexyl-2-hydroxyethane ammonium trifluoroacetate;
4- {4-[7-(aminocarbonyl)-2H-indazole-2-yl]benzyl} -6-(hydroxymethyl)-trifluoroacetate 1,4-oxaazacycloheptane-4-onium;
{4-[7-(aminocarbonyl)-2H-indazole-2-yl]phenyl}-N-{[1-(hydroxymethyl) cyclobutyl]methyl}methane ammonium trifluoroacetate;
{4-[7-(aminocarbonyl)-2H-indazole-2-yl]phenyl}-N-{[1-(hydroxymethyl)cyclohexyl]methyl} methane ammonium trifluoroacetate; trifluoroacetate 1-{4-[7-(aminocarbonyl)-2H-indazole-2-yl]benzyl}-4-(5-methyl-1H-benzimidazol-2-yl)piperid inium; bis(trifluoroacetic acid)2-(1- {4-[7-(aminocarbonyl)-2H-indazole-2-yl]benzyl} -4-hydroxy piperidinium-4-yl)pyridinium; trifluoroacetate
1-{4-[7-(aminocarbonyl)-2H-indazole-2-yl]benzyl} -3,3-difluoropyrrolidinium;
2-(4-{[(2R)-2-(fluoromethyl)pyrrolidin-1-yl]methyl}phenyl)-2H-indazole-7-carboxamide;
N- {4-[7-(aminocarbonyl)-2H-indazole-2-yl]benzyl} -2-oxopyrrolidine-3 -ammonium trifluoroacetate; 5-fluoro-2-(4-formylphenyl)-2H-indazole-7-carboxamide; trifluoroacetate 3-[({4-[7-(aminocarbonyl)-5-fluoro-2H-indazole-2-yl]phenyl}amino)carbonyl]-1-methyl azacyclobutanium; bis(trifluoroacetic acid)1-{4-[7-(aminocarbonyl)-5-fluoro-2H-indazole-2-yl]benzyl}-3-[(dimethylamino)methyl]p iperidinium; trifluoroacetate 3-[({4-[7-(aminocarbonyl)-5-fluoro-2H-indazole-2-yl]-2-fluorophenyl}amino)carbonyl]azacyc lobutanium; 2- {4-[7-(aminocarbonyl)-5-fluoro-2H-indazole-2-yl]benzyl} -2,7-diazonium
heterospiro[4.5] decanebis(trifluoroacetate);
4,5-difluoro-2-{4-[(methylamino)methyl]phenyl}-2H-indazole-7-carboxamide trifluoroacetate; 5-fluoro-2-(3-fluoro-4-{[(1-methylazetidine-3-yl)carbonyl]amino}phenyl)-2H-indazole-7-carb
oxamide trifluoroacetate; 5-fluoro-2-(3-fluoro-4-formylphenyl)-2H-indazole-7-carboxamide; 5-fluoro-2-(5-fluoro-2-formylphenyl)-2H-indazole-7-carboxamide;
{4-[7-(aminocarbonyl)-5-fluoro-2H-indazole-2-yl]-2-fluorophenyl}-N-{[1-(hydroxymethyl)cy clopentyl]methyl} ammonium trifluoroacetate;
5-fluoro-2-[3-fluoro-4-({[(3R)-1-methylpiperidin-3-yl]carbonyl}amino)phenyl]-2H-indazole-7 -carboxamide trifluoroacetate; bis(trifluoroacetic acid)1-{4-[7-(aminocarbonyl)-5-fluoro-2H-indazole -2-yl]-2-fluorobenzyl} -4-methylpiperazine dinium; bis(trifluoroacetic acid) 4-[({4-[7-(aminocarbonyl)-5-fluoro-2H-indazole-2-yl]benzyl} ammonio)methyl]-1-methylpiper idinium; bis(trifluoroacetic acid)4-[({4-[7-(aminocarbonyl)-5-fluoro-2H-indazole-2-yl]-2-fluorobenzyl)ammonio)methyl]-1-methylpiperidinium;
and pharmaceutically acceptable salts or tautomers thereof.

6. The application according to claim 5, wherein, the compound shown in formula I is 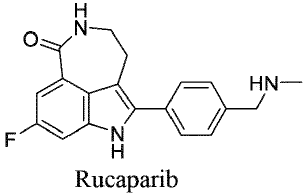 ; the compound shown in formula II is 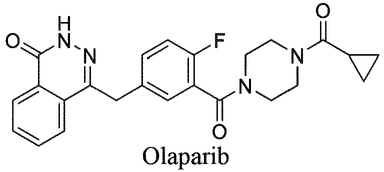 ; the compound shown in formula III is 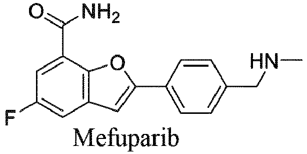 ; the compound shown in formula IV is

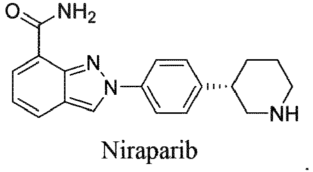

7. The application according to any one of claims 1-6, wherein the pharmaceutically acceptable salt is hydrochloride; preferably

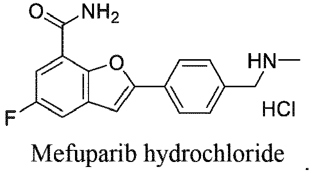

8. The application according to any one of claims 1-7, wherein the poly ADP ribose polymerase inhibitor or a pharmaceutically acceptable salt thereof is present in the form of a pharmaceutical composition comprising the same;
Preferably, the pharmaceutical composition uses the poly ADP ribose polymerase inhibitor or a pharmaceutically acceptable salt thereof as the only active ingredient of the pharmaceutical composition; and/or, the pharmaceutical composition further comprises pharmaceutical acceptable carriers, such as pharmaceutically acceptable excipients.

9. The application according to any one of claims 1-8, wherein the poly ADP ribose polymerase inhibitor or a pharmaceutically acceptable salt thereof is present in the form of a kit composition comprising the same, and the kit also contains drugs to treat coronavirus-related diseases and/or drugs to treat diseases caused by other viruses.

10. The application of a compound represented by formula **III** and/or a compound represented by formula **IV** or a pharmaceutically acceptable salt thereof in the preparation of antiviral agents or in the preparation of medicines for treatment of diseases caused by viruses according to any one of claims 3-6, the viruses being HIV, HPV, EBV, IFV and/or coronaviruses, preferably the subfamily Orthocoronavirinae viruses.

11. The application according to claim 10, wherein the pharmaceutically acceptable salt is hydrochloride; preferably

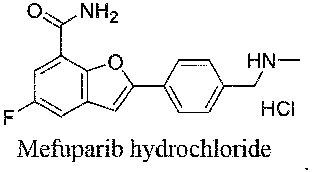

12. The application according to claim 10 or 11, wherein the compound represented by formula **III** and/or a compound represented by formula **IV** or a pharmaceutically acceptable salt thereof is present in the form of a pharmaceutical composition comprising the same;
preferably, the pharmaceutical composition uses the compound represented by formula **III** and/or a compound represented by formula **IV** or a pharmaceutically acceptable salt thereof as the only active ingredient of the pharmaceutical composition; and/or, the pharmaceutical composition further comprises pharmaceutical acceptable carriers, such as pharmaceutically acceptable excipients.

13. The application according to any one of claims 10-12, wherein the compound represented by formula **III** and/or a compound represented by formula **IV** or a pharmaceutically acceptable salt thereof is present in the form of a kit composition comprising the same, and the kit also contains drugs for other anti-coronavirus-induced diseases.

14. The application according to any one of claims 10-13, wherein, the subfamily Orthocoronavirinae virus is α coronavirus, β coronavirus, γ coronavirus and/or δ coronavirus, preferably a coronavirus that causes upper respiratory tract infection, a virus that causes acute respiratory syndrome such as SARS-related coronavirus and/or Middle East respiratory syndrome coronavirus;
preferably, the coronaviruses that cause upper respiratory tract infections are human coronavirus 229E, human coronavirus HKU1, human coronavirus OC43, human coronavirus NL63 and/or mouse hepatitis virus A59; and/or, the SARS-related coronavirus is SARS-CoV or SARS-CoV-2.
